# EUROPEAN PATENT SPECIFICATION

(11) **EP 2 970 315 B1**
(45) Date of publication and mention of the grant of the patent: **15.09.2021**
(21) Application number: 14768414.6
(22) Date of filing: 10.03.2014
(51) Int. Cl.: A61K 31/437, A61P 7/00, C07D 401/04, C07D 231/12, C07D 413/04, C07D 405/04, C07D 403/04

(54) **COMPOUNDS AND USES THEREOF FOR THE MODULATION OF HEMOGLOBIN**
VERBINDUNGEN UND VERWENDUNGEN DAVON ZUR MODULATION VON HÄMOGLOBIN
COMPOSÉS ET LEURS UTILISATIONS POUR LA MODULATION DE L'HÉMOGLOBINE

(30) Priority: 15.03.2013 US 201313815776; 18.11.2013 US 201361905802 P
(43) Date of publication of application: 20.01.2016
(73) Proprietor: Global Blood Therapeutics, Inc., South San Francisco, CA 94080 (US)
(72) Inventor: LI, Zhe, South San Francisco, California 94080 (US); XU, Qing, South San Francisco, California 94080 (US); METCALF, Brian W., South San Francisco, California 94080 (US); GWALTNEY, II, Stephen L., South San Francisco, California 94080 (US); HARRIS, Jason R., South San Francisco, California 94080 (US); YEE, Calvin W., South San Francisco, California 94080 (US)
(74) Representative: Carpmaels & Ransford LLP
(86) International application number: PCT/US2014/022846
(87) International publication number: WO 2014/150289

(56) References cited:
- WO-A1-00/71123
- WO-A1-2013/102142
- WO-A1-2014/150276
- WO-A2-2004/050030
- US-A1- 2003 022 923
- US-A1- 2005 143 420
- US-B2- 7 160 910
- ROLAN P E ET AL: "The pharmacokinetics, tolerability and pharmacodynamics of tucaresol (589C80); 4[2-formyl-3-hydroxyphenoxymethyl] benzoic acid), a potential anti-sickling agent, following oral administration to healthy subjects", BRITISH JOURNAL OF CLINICAL PHARMACOLOGY, BLACKWELL SCIENTIFIC PUBL, GB, vol. 35, no. 4, 1 April 1993 (1993-04-01), pages 419-425, XP002000120, ISSN: 0306-5251
- NNAMANI I N ET AL: "Pyridyl derivatives of benzaldehyde as potential antisickling agents", CHEMISTRY & BIODIVERSITY, HELVETICA CHIMICA ACTA, ZUERICH, CH, vol. 5, no. 9, 1 September 2008 (2008-09-01), pages 1762-1769, XP002732217, ISSN: 1612-1872, DOI: 10.1002/CBDV.200890165 [retrieved on 2008-09-24]
- OSHEIZA ABDULMALIK ET AL: "Sickle cell disease: current therapeutic approaches", EXPERT OPINION ON THERAPEUTIC PATENTS, vol. 15, no. 11, 1 November 2005 (2005-11-01), pages 1497-1506, XP055126525, ISSN: 1354-3776, DOI: 10.1517/13543776.15.11.1497
- None

## Description

### CROSS-REFERENCE TO RELATED APPLICATIONS

### FIELD OF THE INVENTION

This invention provides compounds and pharmaceutical compositions suitable as allosteric modulators of hemoglobin, methods and intermediates for their preparation, and their use in treating disorders mediated by hemoglobin and disorders that would benefit from tissue and/or cellular oxygenation.

### STATE OF THE ART

Sickle cell disease is a disorder of the red blood cells, found particularly among those of African and Mediterranean descent. The basis for sickle cell disease is found in sickle hemoglobin (HbS), which contains a point mutation relative to the prevalent peptide sequence of hemoglobin (Hb).

Hemoglobin (Hb) transports oxygen molecules from the lungs to various tissues and organs throughout the body. Hemoglobin binds and releases oxygen through conformational changes. Sickle hemoglobin (HbS) contains a point mutation where glutamic acid is replaced with valine, allowing HbS to become susceptible to polymerization to give the HbS containing red blood cells their characteristic sickle shape. The sickled cells are also more rigid than normal red blood cells, and their lack of flexibility can lead to blockage of blood vessels. US 7,160,910 discloses compounds that are allosteric modulators of hemoglobin. However, a need exists for additional therapeutics that can treat disorders that are mediated by Hb or by abnormal Hb such as HbS.

### SUMMARY OF THE INVENTION

This invention relates generally to compounds and pharmaceutical compositions suitable as allosteric modulators of hemoglobin. In some aspects, this invention relates to compounds for use in methods for treating disorders mediated by hemoglobin and disorders that would benefit from tissue and/or cellular oxygenation.
The invention provides a compound of Formula (I'): 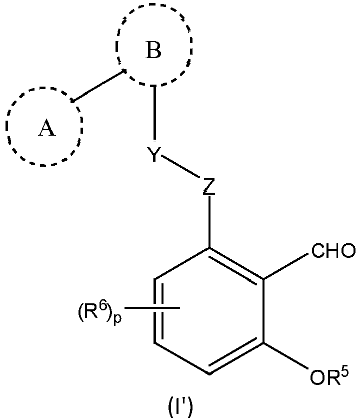 or a tautomer thereof, or a pharmaceutically acceptable salt of each thereof, wherein
ring A is an optionally substituted 5-10 membered heteroaryl containing up to 3 ring N, O, and/or S atoms, and oxidized forms of N and/or S atoms;
wherein ring A is α substituted relative to the Y substituent;
ring B is an optionally substituted C₆-C₁₀ aryl or 4-10 membered heterocycle containing up to 5 ring heteroatoms, wherein the heteroatom is selected from the group consisting of O, N, S, and oxidized forms of N and S;
Y-Z is -CH₂O-, wherein the right hand side of the substituent is joined with the substituted phenyl ring;
R⁵ is hydrogen or C₁-C₆ alkyl, wherein the C₁-C₆ alkyl is optionally substituted with 1-5 halo;
R⁶ is halo, C₁-C₆ alkyl, C₁-C₆ alkoxy, C₁-C₆ alkylthio, C₁-C₆ alkyl-S(O)-, or C₁-C₆ alkylS(O)₂-, wherein the C₁-C₆ alkyl is optionally substituted with 1-5 halo; or
R⁶ is 4-10 membered cycloalkyl or heterocycle substituted with an R'R'N- moiety wherein each R' is independently C₁-C₆ alkyl or hydrogen;
p is 0, 1, 2, or 3; and the term heterocycle refers to a non-aromatic, mono-, bi-, or tricyclic ring containing 2-12 ring carbon atoms and 1-8 ring heteroatoms, provided that the ring contains at least 3 ring atoms.

In certain aspects of the disclosure, a compound of Formula (A) is provided: 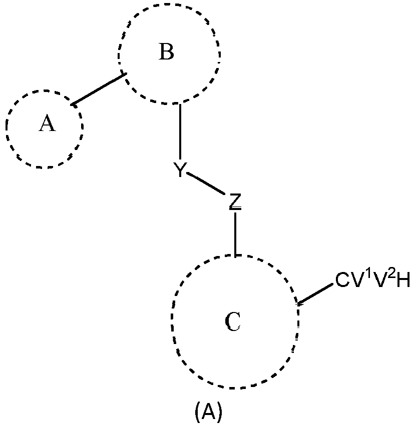 or a tautomer thereof, or a pharmaceutically acceptable salt of each thereof, wherein
ring A is an optionally substituted 5-10 membered heteroaryl containing up to 3 ring N, O, and/or S atoms, and oxidized forms of N and/or S atoms;
wherein ring A is α or β substituted relative to the Y substituent;
ring B is an optionally substituted C₆-C₁₀ aryl or 4-10 membered heterocycle containing up to 5 ring heteroatoms, wherein the heteroatom is selected from the group consisting of O, N, S, and oxidized forms of N and S;
each Y and Z is independently CR¹⁰R¹¹, O, S, SO, SO₂, or NR¹²; each R¹⁰ and R¹¹ independently is hydrogen or C₁-C₃ alkyl, optionally substituted with halo, OH, or alkoxy, or CR¹⁰R¹¹ is C=O; R¹² is hydrogen or C₁-C₆ alkyl; provided that if one of Y and Z is O, S, SO, SO₂, then the other is not CO, and provided that Y and Z are both not heteroatoms or oxidized forms thereof;
ring C is C₆-C₁₀ aryl, optionally subtituted;
V¹ and V² independently are C₁-C₆ alkoxy; or V¹ and V² together with the carbon atom they are attached to form a ring of formula: 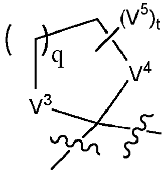
wherein each V³ and V⁴ are independently O, S, or NH, provided that when one of V³ and V⁴ is S, the other is NH, and provided that V³ and V⁴ are both not NH; q is 1 or 2; each V⁵ is independently C₁-C₆ alkyl or CO₂R⁶⁰, where each R⁶⁰ independently is C₁-C₆ alkyl or hydrogen; t is 0, 1, 2, or 4; or CV¹V² is C=V, wherein V is O, NOR⁸⁰, or NNR⁸¹R⁸²;
R⁸⁰ is optionally substituted C₁-C₆ alkyl;
R⁸¹ and R⁸² independently are selected from the group consisting of hydrogen, optionally substituted C₁-C₆ alkyl, COR⁸³, or CO₂R⁸⁴;
R⁸³ is hydrogen or optionally substituted C₁-C₆ alkyl; and
R⁸⁴ is optionally substituted C₁-C₆ alkyl.

In one aspect of the disclosure, the compound provided is of formula (I): 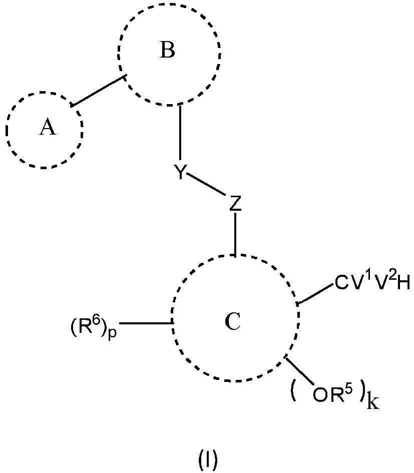 wherein
R⁵ is hydrogen or C₁-C₆ alkyl optionally substituted with 1-5 halo;
R⁶ is a substituent that is halo, C₁-C₆ alkyl, C₁-C₆ alkoxy, C₁-C₆ alkylthio, C₁-C₆ alkylS(O)-, or C₁-C₆ alkyl-S(O)₂-, wherein the C₁-C₆ alkyl is optionally substituted with 1-5 halo; or
R⁶ is 4-10 membered cycloalkyl or heterocycle substituted with an R'R'N- moiety wherein each R' is independently C₁-C₆ alkyl or hydrogen;
k is 0 or 1; and
p is 0, 1, 2, or 3.
and the remaining variables are defined as above.

In further aspects of the invention, a composition is provided comprising any of the compounds of the invention as claimed and at least a pharmaceutically acceptable excipient.

In still further aspects of the invention, a compound or composition of the invention as claimed is provided for use in a method for increasing oxygen affinity of hemoglobin S in a subject, the method comprising administering to a subject in need thereof any of the compounds or compositions described herein.

In further aspects of the invention, a compound or composition of the invention as claimed is provided for use in a method for treating oxygen deficiency associated with sickle cell anemia, the method comprising administering to a subject in need thereof any of the compounds or compositions of the invention as claimed.

### DETAILED DESCRIPTION OF THE INVENTION

### Definitions

It must be noted that as used herein and in the appended claims, the singular forms "a", "an", and "the" include plural referents unless the context clearly dictates otherwise. Thus, for example, reference to "a solvent" includes a plurality of such solvents.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition or process consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations. Each numerical parameter should at least be construed in light of the number of reported significant digits and by applying ordinary rounding techniques. The term "about" when used before a numerical designation, e.g., temperature, time, amount, and concentration, including range, indicates approximations which may vary by (+ )or (-) 10 %, 5 % or 1 %.

As used herein, Cₘ-Cₙ, such as C₁-C₁₂, C₁-C₈, or C₁-C₆ when used before a group refers to that group containing m to n carbon atoms.

The term "alkoxy" refers to -O-alkyl. The term alkylthio refers to -S-alkyl.

The term "alkyl" refers to monovalent saturated aliphatic hydrocarbyl groups having from 1 to 30 carbon atoms (i.e., C₁-C₃₀ alkyl) or 1 to 22 carbon atoms (i.e., C₁-C₂₂ alkyl), 1 to 8 carbon atoms (i.e., C₁-C₈ alkyl), or 1 to 4 carbon atoms. This term includes, by way of example, linear and branched hydrocarbyl groups such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), *t*-butyl ((CH₃)₃C-), n-pentyl (CH₃CH₂CH₂CH₂CH₂-), and neopentyl ((CH₃)₃CCH₂-).

The term "aryl" refers to a monovalent, aromatic mono- or bicyclic ring having 6-10 ring carbon atoms. Examples of aryl include phenyl and naphthyl. The condensed ring may or may not be aromatic provided that the point of attachment is at an aromatic carbon atom. For example, and without limitation, the following is an aryl group:

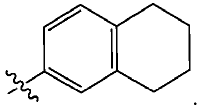

The term "-CO₂H ester" refers to an ester formed between the -CO₂H group and an alcohol, preferably an aliphatic alcohol. A preferred example included -CO₂R^{E}, wherein R^{E} is alkyl or aryl group optionally substituted with an amino group.

The term "chiral moiety" refers to a moiety that is chiral. Such a moiety can possess one or more asymmetric centers. Preferably, the chiral moiety is enantiomerically enriched, and more preferably a single enantiomer. Non limiting examples of chiral moieties include chiral carboxylic acids, chiral amines, chiral amino acids, such as the naturally occurring amino acids, chiral alcohols including chiral steroids, and the likes.

The term "cycloalkyl" refers to a monovalent, preferably saturated, hydrocarbyl mono-, bi-, or tricyclic ring having 3-12 ring carbon atoms. While cycloalkyl, refers preferably to saturated hydrocarbyl rings, as used herein, it also includes rings containing 1-2 carbon-carbon double bonds. Nonlimiting examples of cycloalkyl include cyclopropyl, cyclobutyl, cyclopentyl, cyclohexyl, cycloheptyl, adamentyl, and the like. The condensed rings may or may not be non-aromatic hydrocarbyl rings provided that the point of attachment is at a cycloalkyl carbon atom. For example, and without limitation, the following is a cycloalkyl group:

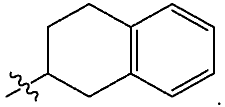

The term "halo" refers to F, Cl, Br, and/or I.

The term "heteroaryl" refers to a monovalent, aromatic mono-, bi-, or tricyclic ring having 2-16 ring carbon atoms and 1-8 ring heteroatoms selected preferably from N, O, S, and P and oxidized forms of N, S, and P, provided that the ring contains at least 5 ring atoms. Nonlimiting examples of heteroaryl include furan, imidazole, oxadiazole, oxazole, pyridine, quinoline, and the like. The condensed rings may or may not be a heteroatom containing aromatic ring provided that the point of attachment is a heteroaryl atom. For example, and without limitation, the following is a heteroaryl group:

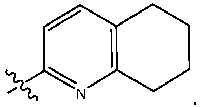

The term "heterocyclyl" or heterocycle refers to a non-aromatic, mono-, bi-, or tricyclic ring containing 2-12 ring carbon atoms and 1-8 ring heteroatoms selected preferably from N, O, S, and P and oxidized forms of N, S, and P, provided that the ring contains at least 3 ring atoms. While heterocyclyl preferably refers to saturated ring systems, it also includes ring systems containing 1-3 double bonds, provided that the ring is non-aromatic. Nonlimiting examples of heterocyclyl include, azalactones, oxazoline, piperidinyl, piperazinyl, pyrrolidinyl, tetrahydrofuranyl, and tetrahydropyranyl. The condensed rings may or may not contain a non-aromatic heteroatom containing ring provided that the point of attachment is a heterocyclyl group. For example, and without limitation, the following is a heterocyclyl group:

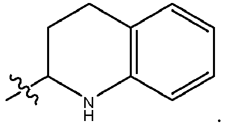

The term "hydrolyzing" refers to breaking an R^{H}-O-CO-, R^{H}-O-CS-, or an R^{H}-O-SO₂- moiety to an R^{H}-OH, preferably by adding water across the broken bond. A hydrolyzing is performed using various methods well known to the skilled artisan, non limiting examples of which include acidic and basic hydrolysis.

The term "oxo" refers to a C=O group, and to a substitution of 2 geminal hydrogen atoms with a C=O group.

The term "optionally substituted" refers to a substituted or unsubstituted group. The group may be substituted with one or more substituents, such as e.g., 1, 2, 3, 4 or 5 substituents. Preferably, the substituents are selected from the group consisting of oxo, halo, -CN, NO₂, -N₂+, -CO₂R¹⁰⁰, -OR¹⁰⁰, -SR¹⁰⁰, -SOR¹⁰⁰, SO₂R¹⁰⁰, -NR¹⁰¹R¹⁰², -CONR¹⁰¹R¹⁰²,-SO₂NR¹⁰¹R¹⁰², C₁-C₆ alkyl, C₁-C₆ alkoxy, -CR¹⁰⁰=C(R¹⁰⁰)₂, -CCR¹⁰⁰, C₃-C₁₀ cycloalkyl, C₃-C₁₀ heterocyclyl, C₆-C₁₂ aryl and C₂-C₁₂ heteroaryl, wherein each R¹⁰⁰ independently is hydrogen or C₁-C₈ alkyl; C₃-C₁₂ cycloalkyl; C₃-C₁₀ heterocyclyl; C₆-C₁₂ aryl; or C₂-C₁₂ heteroaryl; wherein each alkyl, cycloalkyl, heterocyclyl, aryl, or heteroaryl is optionally substituted with 1-3 halo, 1-3 C₁-C₆ alkyl, 1-3 C₁-C₆ haloalkyl or 1-3 C₁-C₆ alkoxy groups. Preferably, the substituents are selected from the group consisting of chloro, fluoro, -OCH₃, methyl, ethyl, *iso*-propyl, cyclopropyl, vinyl, ethynyl, -CO₂H, -CO₂CH₃, -OCF₃, -CF₃ and -OCHF₂.

R¹⁰¹ and R¹⁰² independently is hydrogen; C₁-C₈ alkyl, optionally substituted with-CO₂H or an ester thereof, C₁-C₆ alkoxy, oxo, -CR¹⁰³=C(R¹⁰³)₂, -CCR, C₃-C₁₀ cycloalkyl, C₃-C₁₀ heterocyclyl, C₆-C₁₂ aryl, or C₂-C₁₂ heteroaryl, wherein each R¹⁰³ independently is hydrogen or C₁-C₈ alkyl; C₃-C₁₂ cycloalkyl; C₃-C₁₀ heterocyclyl; C₆-C₁₂ aryl; or C₂-C₁₂ heteroaryl; wherein each cycloalkyl, heterocyclyl, aryl, or heteroaryl is optionally substituted with 1-3 alkyl groups or 1-3 halo groups, or R¹⁰¹ and R¹⁰² together with the nitrogen atom they are attached to form a 5-7 membered heterocycle.

The term "pharmaceutically acceptable" refers to safe and non-toxic for *in vivo,* preferably, human administration.

The term "pharmaceutically acceptable salt" refers to a salt that is pharmaceutically acceptable.

The term "salt" refers to an ionic compound formed between an acid and a base. When the compound provided herein contains an acidic functionality, such salts include, without limitation, alkali metal, alkaline earth metal, and ammonium salts. As used herein, ammonium salts include, salts containing protonated nitrogen bases and alkylated nitrogen bases. Exemplary, and non-limiting cations useful in pharmaceutically acceptable salts include Na, K, Rb, Cs, NH₄, Ca, Ba, imidazolium, and ammonium cations based on naturally occurring amino acids. When the compounds utilized herein contain basic functionality, such salts include, without limitation, salts of organic acids, such as carboxylic acids and sulfonic acids, and mineral acids, such as hydrogen halides, sulfuric acid, phosphoric acid, and the likes. Exemplary and non-limiting anions useful in pharmaceutically acceptable salts include oxalate, maleate, acetate, propionate, succinate, tartrate, chloride, sulfate, bisalfate, mono-, di-, and tribasic phosphate, mesylate, tosylate, and the likes.

The terms "treat", "treating" or "treatment", as used herein, include alleviating, abating or ameliorating a disease or condition or one or more symptoms thereof, preventing additional symptoms, ameliorating or preventing the underlying metabolic causes of symptoms, inhibiting the disease or condition, e.g., arresting or suppressing the development of the disease or condition, relieving the disease or condition, causing regression of the disease or condition, relieving a condition caused by the disease or condition, or suppressing the symptoms of the disease or condition, and are intended to include prophylaxis. The terms also include relieving the disease or conditions, e.g., causing the regression of clinical symptoms. The terms further include achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the individual, notwithstanding that the individual is still be afflicted with the underlying disorder. For prophylactic benefit, the compositions are administered to an individual at risk of developing a particular disease, or to an individual reporting one or more of the physiological symptoms of a disease, even though a diagnosis of this disease has not been made.

The terms "preventing" or "prevention" refer to a reduction in risk of acquiring a disease or disorder (i.e., causing at least one of the clinical symptoms of the disease not to develop in a subject that may be exposed to or predisposed to the disease but does not yet experience or display symptoms of the disease). The terms further include causing the clinical symptoms not to develop, for example in a subject at risk of suffering from such a disease or disorder, thereby substantially averting onset of the disease or disorder.

The term "effective amount" refers to an amount that is effective for the treatment of a condition or disorder by an intranasal administration of a compound or composition described herein. In some embodiments, an effective amount of any of the compositions or dosage forms described herein is the amount used to treat a disorder mediated by hemoglobin or a disorder that would benefit from tissue and/or cellular oxygenation of any of the compositions or dosage forms described herein to a subject in need thereof.

The term "carrier" as used herein, refers to relatively nontoxic chemical compounds or agents that facilitate the incorporation of a compound into cells, e.g., red blood cells, or tissues.

### Compounds

In certain aspects of the disclosure, a compound of Formula (I) is provided: 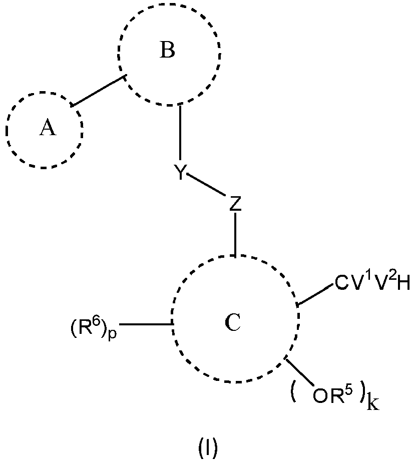 or a tautomer thereof, or a pharmaceutically acceptable salt of each thereof, wherein
ring A is an optionally substituted 5-10 membered heteroaryl containing up to 3 ring N, O, and/or S atoms, and oxidized forms of N and/or S atoms;
wherein ring A is α or β substituted relative to the Y substituent;
ring B is an optionally substituted C₆-C₁₀ aryl or 4-10 membered heterocycle containing up to 5 ring heteroatoms, wherein the heteroatom is selected from the group consisting of O, N, S, and oxidized forms of N and S;
each Y and Z is independently CR¹⁰R¹¹, O, S, SO, SO₂, or NR¹²; each R¹⁰ and R¹¹ independently is hydrogen or C₁-C₃ alkyl, optionally substituted with halo, OH, or alkoxy, or CR¹⁰R¹¹ is C=O; R¹² is hydrogen or C₁-C₆ alkyl; provided that if one of Y and Z is O, S, SO, SO₂, then the other is not CO, and provided that Y and Z are both not heteroatoms or oxidized forms thereof;
ring C is C₆-C₁₀ aryl;
V¹ and V² independently are C₁-C₆ alkoxy; or V¹ and V² together with the carbon atom they are attached to form a ring of formula: 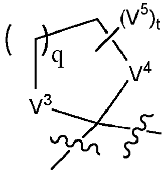
wherein each V³ and V⁴ are independently O, S, or NH, provided that when one of V³ and V⁴ is S, the other is NH, and provided that V³ and V⁴ are both not NH; q is 1 or 2; each V⁵ is independently C₁-C₆ alkyl or CO₂R⁶⁰, where each R⁶⁰ independently is C₁-C₆ alkyl or hydrogen; t is 0, 1, 2, or 4; or CV¹V² is C=V, wherein V is O, NOR⁸⁰, or NNR⁸¹R⁸²;
R⁵ is hydrogen or C₁-C₆ alkyl optionally substituted with 1-5 halo;
R⁶ is a substituent that is halo, C₁-C₆ alkyl, C₁-C₆ alkoxy, C₁-C₆ alkylthio, C₁-C₆ alkylS(O)-, or C₁-C₆ alkyl-S(O)₂-, wherein the C₁-C₆ alkyl is optionally substituted with 1-5 halo; or
R⁶ is 4-10 membered cycloalkyl or heterocycle substituted with an R'R'N- moiety wherein each R' is independently C₁-C₆ alkyl or hydrogen;
R⁸⁰ is optionally substituted C₁-C₆ alkyl;
R⁸¹ and R⁸² independently are selected from the group consisting of hydrogen, optionally substituted C₁-C₆ alkyl, COR⁸³, or CO₂R⁸⁴;
R⁸³ is hydrogen or optionally substituted C₁-C₆ alkyl;
R⁸⁴ is optionally substituted C₁-C₆ alkyl;
k is 0 or 1; and
p is 0, 1, 2, or 3.

In certain aspects of the disclosure, t is 0. In certain aspects of the disclosure, t is 1. In certain aspects of the disclosure, t is 2. In certain aspects of the disclosure, t is 3.

As used herein, R⁶⁰ can be hydrogen, provided that the CO₂R⁶⁰ is not joined to a nitrogen atom.

In certain embodiments, Y and Z are both not a heteroatom or a heteroatom containing moiety. In some preferred embodiments, one of Y and Z is a methylene or substituted methylene and the other is a heteroatom or a heteroatom containing moiety. More preferably, Y is an alkylene, and Z is a heteroatom or a heteroatom containing moiety, which, yet more preferably is oxygen.

In certain aspects of the disclosure, V¹ and V² together with the carbon atom they are attached to form a ring of formula:

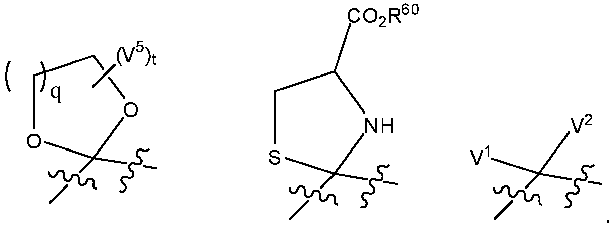

In certain aspects of the disclosure, V¹ and V² independently are C₁-C₆ alkoxy; or V¹ and V² together with the carbon atom they are attached to form a ring of formula: 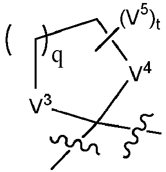 wherein each V³ and V⁴ are independently O, S, or NH, provided that when one or V³ and V⁴ is S the other is NH, and provided that V³ and V⁴ are both not NH; q is 1 or 2; each V⁵ is independently C₁-C₆ alkyl or CO₂R⁶⁰, where each R⁶⁰ independently is C₁-C₆ alkyl or hydrogen; t is 0, 1, 2, or 4; or CV¹V² is C=V, wherein V is O, and wherein the remaining variables are defined herein.

In certain embodiments, ring B contains a double bond. In some other embodiments, ring B contains no double bonds.

In the invention, the compound is of Formula (I'): 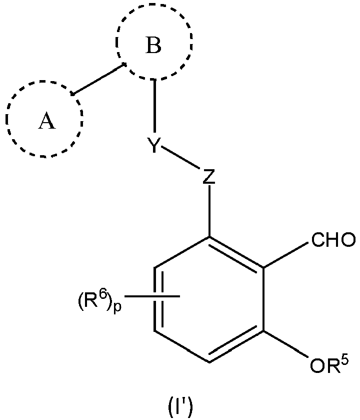 or a tautomer thereof, or a pharmaceutically acceptable salt of each thereof, wherein
ring A is an optionally substituted 5-10 membered heteroaryl containing up to 3 ring N, O, and/or S atoms, and oxidized forms of N and/or S atoms;
wherein ring A is α substituted relative to the Y substituent;
ring B is an optionally substituted C₆-C₁₀ aryl or 4-10 membered heterocycle containing up to 5 ring heteroatoms, wherein the heteroatom is selected from the group consisting of O, N, S, and oxidized forms of N and S;
Y-Z is -CH₂O-, wherein the right hand side of the substituent is joined with the substituted phenyl ring;
R⁵ is hydrogen or C₁-C₆ alkyl, wherein the C₁-C₆ alkyl is optionally substituted with 1-5 halo;
R⁶ is a substituent that is halo, C₁-C₆ alkyl, C₁-C₆ alkoxy, C₁-C₆ alkylthio, C₁-C₆ alkylS(O)-, or C₁-C₆ alkyl-S(O)₂-, wherein the C₁-C₆ alkyl is optionally substituted with 1-5 halo; or
R⁶ is 4-10 membered cycloalkyl or heterocycle substituted with an R'R'N- moiety wherein each R' is independently C₁-C₆ alkyl or hydrogen;
p is 0, 1, 2, or 3; and the term heterocycle refers to a non-aromatic, mono-, bi-, or tricyclic ring containing 2-12 ring carbon atoms and 1-8 ring heteroatoms, provided that the ring contains at least 3 ring atoms.

In certain embodiments, the compound is of Formula IA, IB or IC: 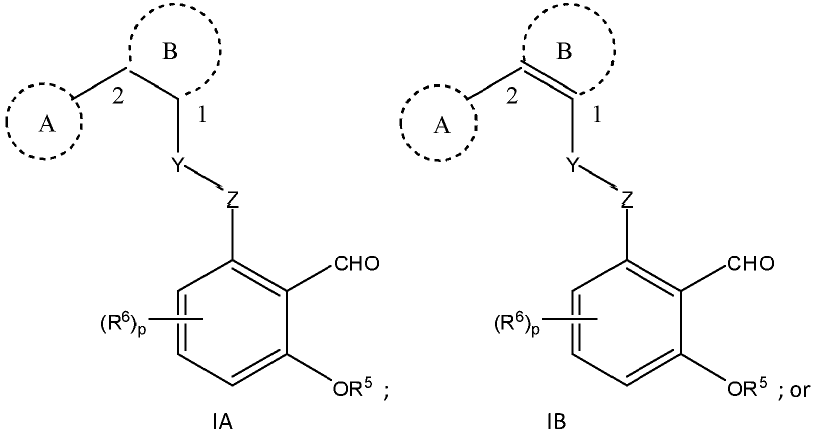 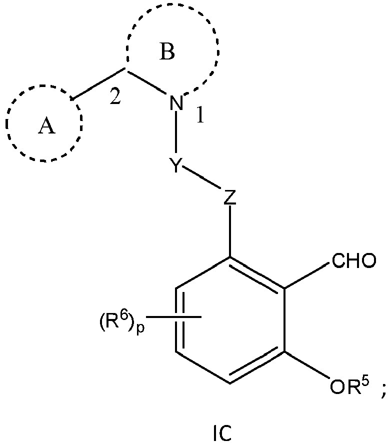 wherein 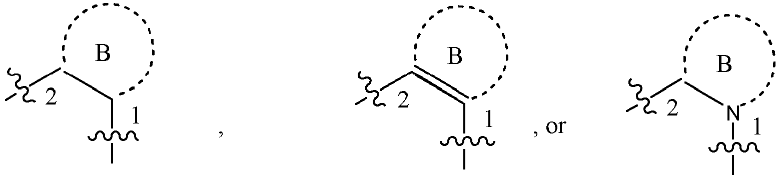 is an optionally substituted 4-10 membered heterocycle as defined herein, and the remaining variables are defined herein.

In certain embodiments, ring A is substituted with 1-3 substituents independently selected from: halo, OH, C₁-C₆ alkyl, and C₁-C₆ alkoxy, wherein the C₁-C₆ alkyl is optionally substituted with 1-5 halo.

In certain embodiments, ring B is substituted with 1-3 substituents independently selected from: halo, OH, C₁-C₆ alkyl, COR¹⁵, and COOR¹⁵; and
R¹⁵ is C₁-C₆ alkyl, C₆-C₁₀aryl, 5-10 membered heteroaryl, or a 4-10 membered heterocycle containing up to 5 ring heteroatoms, wherein the heteroatom is selected from the group consisting of O, N, S, and oxidized forms of N and S, wherein the alkyl, aryl, heteroaryl or heterocycle is optionally substituted.

In certain embodiments, the compound is selected from the group consisting of 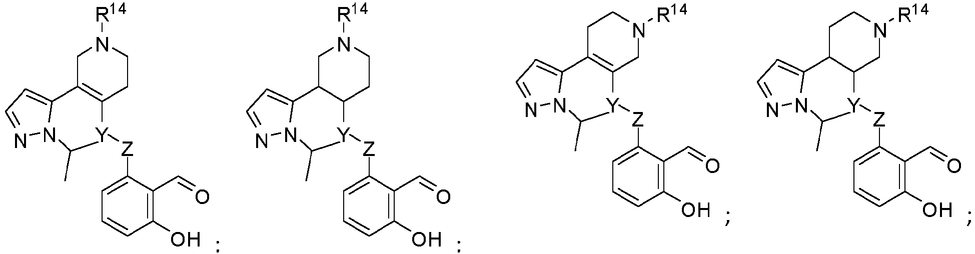 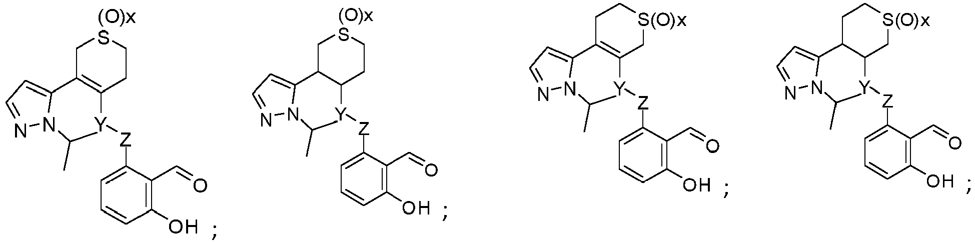 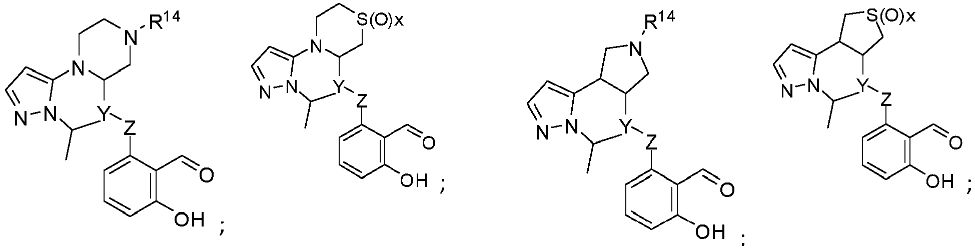 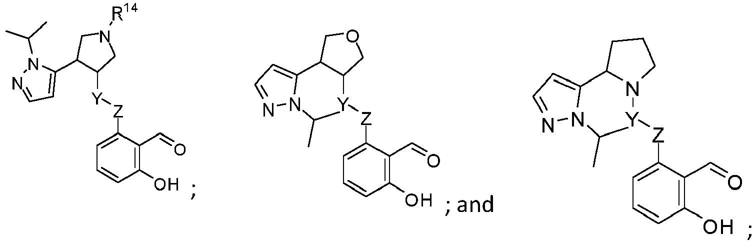 or an N oxide thereof, wherein
Y-Z is -CH₂O-, wherein the right hand side of the substituent is joined with the substituted phenyl ring;
x is 0, 1, or 2;
R¹⁴ is C₁-C₆ alkyl or C₃-C₈ cycloalkyl, COR¹⁵, or COOR¹⁵;
and R¹⁵ is optionally substituted C₁-C₆ alkyl, optionally substituted C₆-C₁₀aryl, optionally substituted 5-10 membered heteroaryl, or optionally substituted 4-10 membered heterocycle containing up to 5 ring heteroatoms, wherein the heteroatom is selected from the group consisting of O, N, S, and oxidized forms of N and S.

In certain embodiments, the compound is selected from the group consisting of 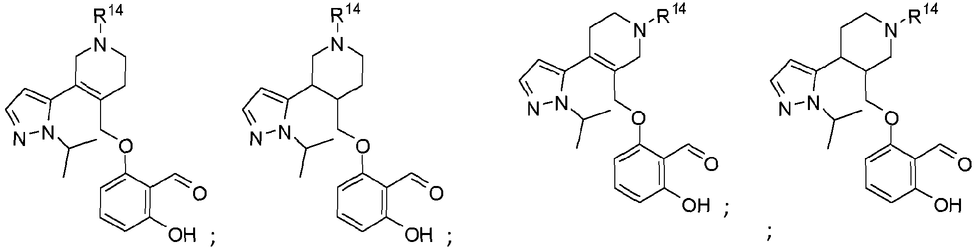 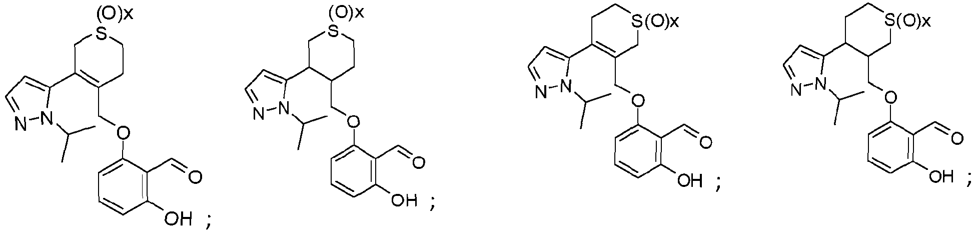 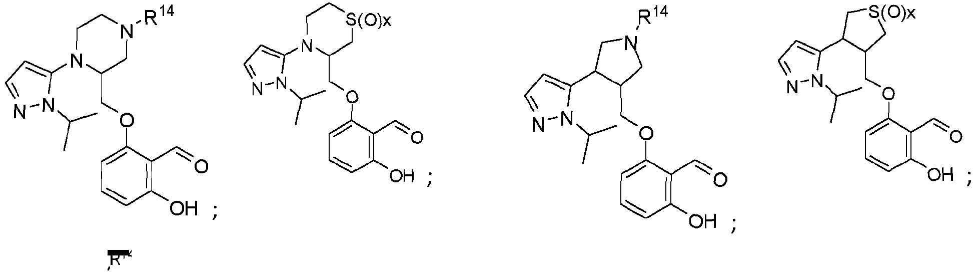 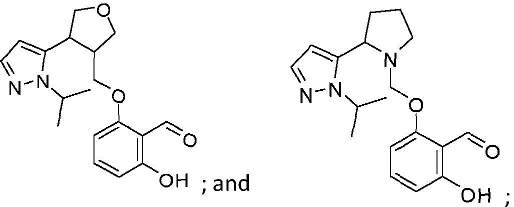 or an N oxide thereof, wherein
x is 0, 1, or 2;
R¹⁴ is C₁-C₆ alkyl and C₃-C₈ cycloalkyl, COR¹⁵, CNR^{15'}R¹⁵ or COOR¹⁵;
and R¹⁵ is optionally substituted C₁-C₆ alkyl, optionally substituted C₆-C₁₀aryl, optionally substituted 5-10 membered heteroaryl, or optionally substituted 4-10 membered heterocycle containing up to 5 ring heteroatoms, wherein the heteroatom is selected from the group consisting of O, N, S, and oxidized forms of N and S.

In certain aspects of the invention, a compound is provided, wherein the compound is selected from the group consisting of: 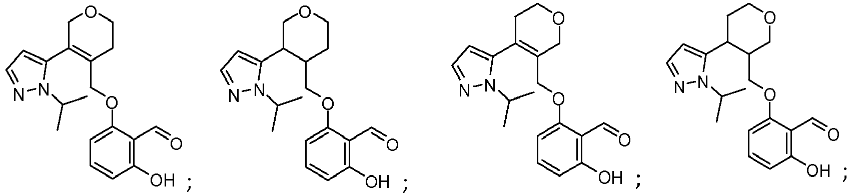 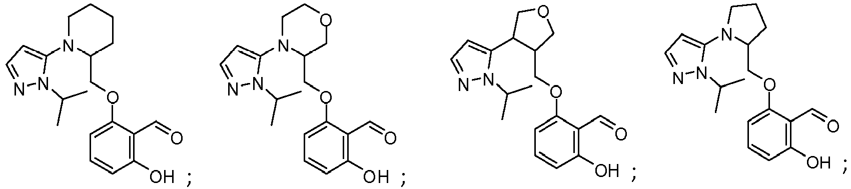 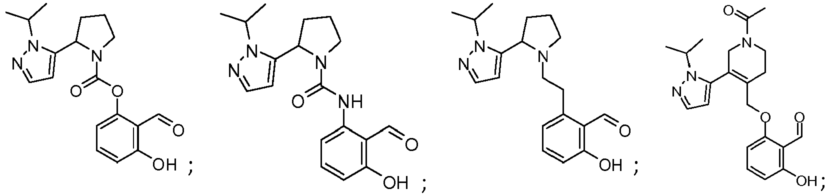 and 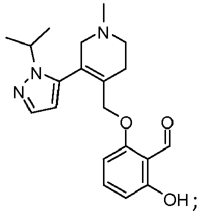 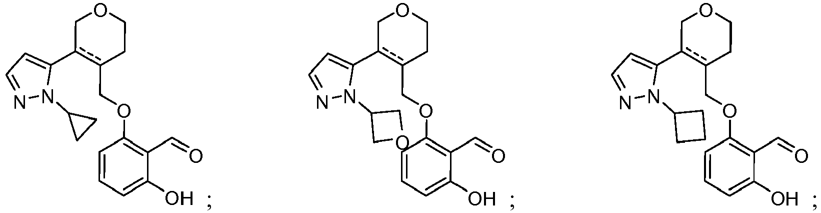 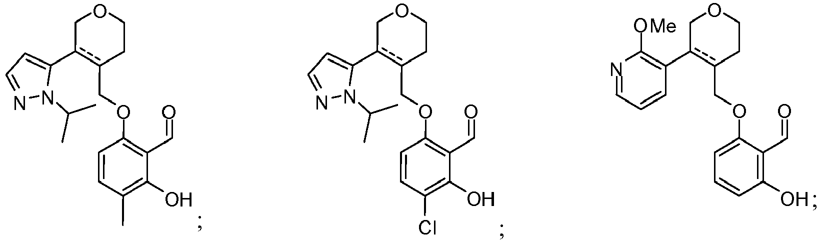 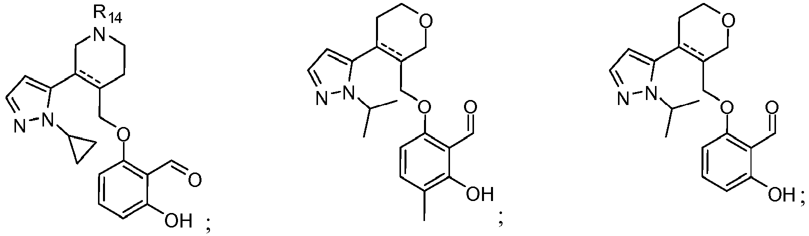 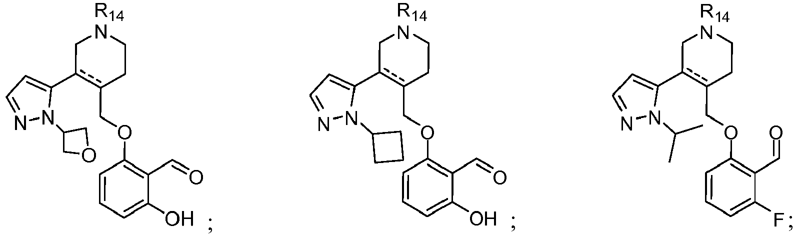 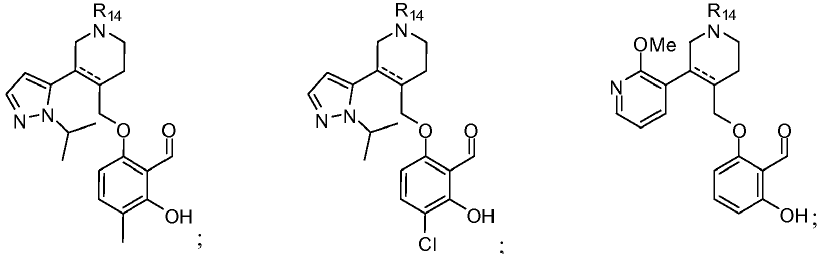 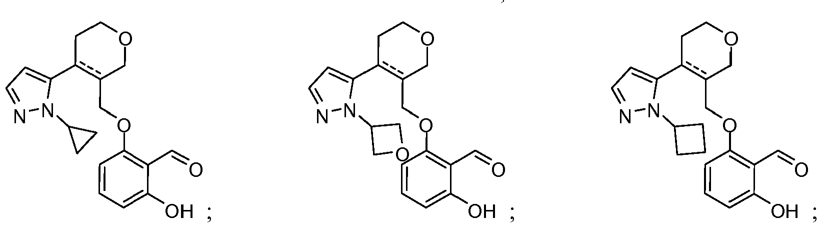 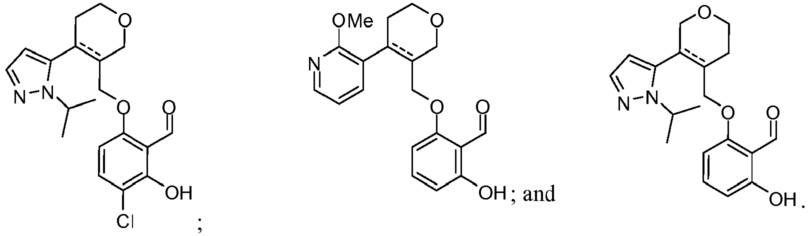 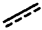 is a double or a single bond.
or an N oxide thereof, or a pharmaceutically acceptable salt of each thereof.

In certain aspects of the invention, a compound is provided, wherein the compound is selected from the group consisting of: 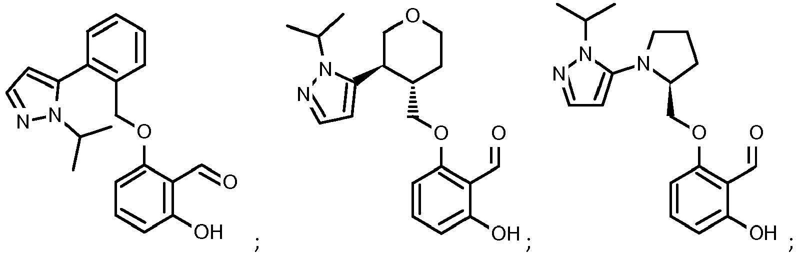 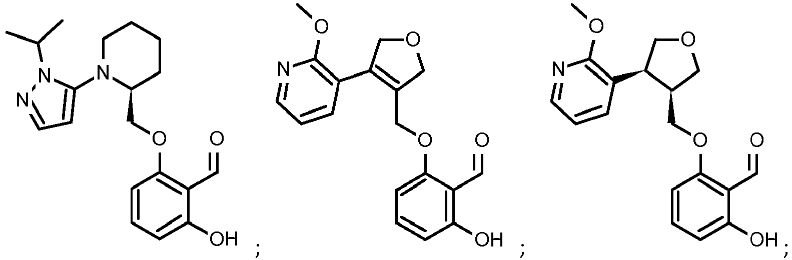 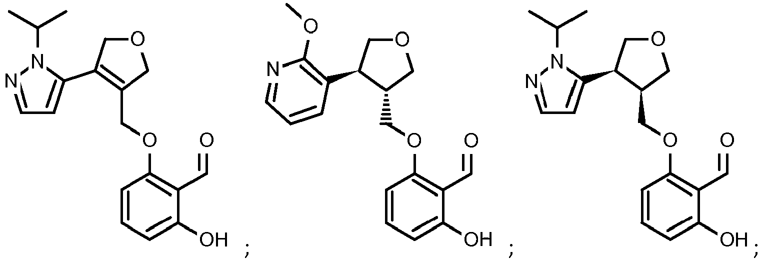 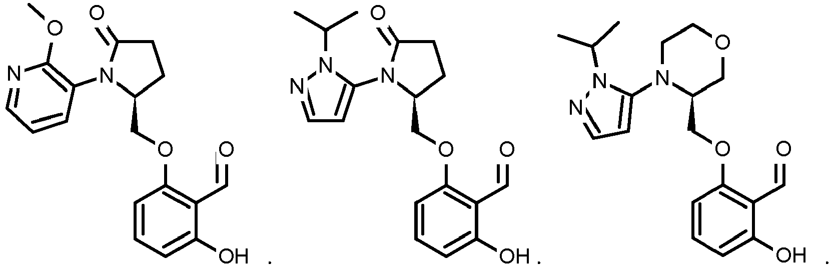 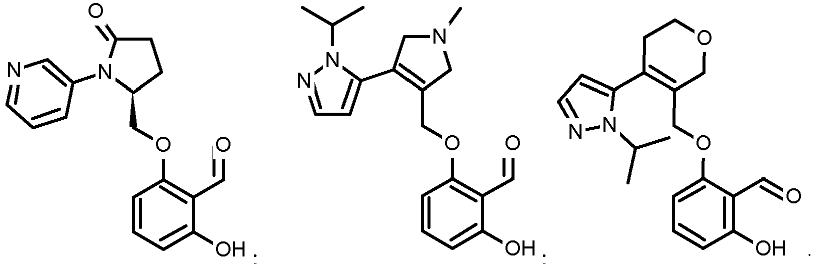 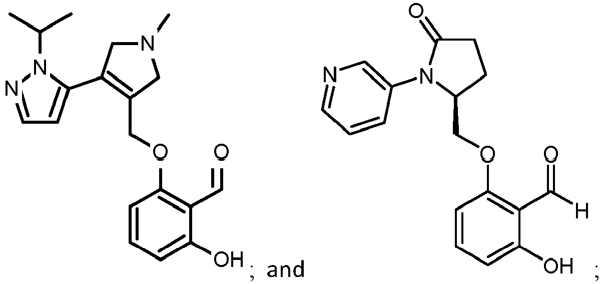 or a pharmaceuticlaly acceptable salt of each thereof.

Other compounds provided herein are included in the Examples section.

### Pharmaceutical Compositions

In further aspects of the invention, a composition is provided comprising any of the compounds of the invention as claimed and at least a pharmaceutically acceptable excipient.

In another aspect, this invention provides a composition comprising any of the compounds of the invention as claimed and a pharmaceutically acceptable excipient.

Such compositions can be formulated for different routes of administration. Although compositions suitable for oral delivery will probably be used most frequently, other routes that may be used include transdermal, intravenous, intraarterial, pulmonary, rectal, nasal, vaginal, lingual, intramuscular, intraperitoneal, intracutaneous, intracranial, and subcutaneous routes. Suitable dosage forms for administering any of the compounds described herein include tablets, capsules, pills, powders, aerosols, suppositories, parenterals, and oral liquids, including suspensions, solutions and emulsions. Sustained release dosage forms may also be used, for example, in a transdermal patch form. All dosage forms may be prepared using methods that are standard in the art (see e.g., Remington's Pharmaceutical Sciences, 16th ed., A. Oslo editor, Easton Pa. 1980).

Pharmaceutically acceptable excipients are non-toxic, aid administration, and do not adversely affect the therapeutic benefit of the compound of this invention. Such excipients may be any solid, liquid, semi-solid or, in the case of an aerosol composition, gaseous excipient that is generally available to one of skill in the art. Pharmaceutical compositions in accordance with the invention are prepared by conventional means using methods known in the art.

The compositions disclosed herein may be used in conjunction with any of the vehicles and excipients commonly employed in pharmaceutical preparations, e.g., talc, gum arabic, lactose, starch, magnesium stearate, cocoa butter, aqueous or non-aqueous solvents, oils, paraffin derivatives, glycols, etc. Coloring and flavoring agents may also be added to preparations, particularly to those for oral administration. Solutions can be prepared using water or physiologically compatible organic solvents such as ethanol, 1,2-propylene glycol, polyglycols, dimethylsulfoxide, fatty alcohols, triglycerides, partial esters of glycerin and the like.

Solid pharmaceutical excipients include starch, cellulose, hydroxypropyl cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. In certain embodiments, the compositions provided herein comprises one or more of α-tocopherol, gum arabic, and/or hydroxypropyl cellulose.

In one embodiment, this invention provides sustained release formulations such as drug depots or patches comprising an effective amount of a compound of the invention as claimed. In another embodiment, the patch further comprises gum Arabic or hydroxypropyl cellulose separately or in combination, in the presence of alpha-tocopherol. Preferably, the hydroxypropyl cellulose has an average MW of from 10,000 to 100,000. In a more preferred embodiment, the hydroxypropyl cellulose has an average MW of from 5,000 to 50,000.

Compounds and pharmaceutical compositions of this invention maybe used alone or in combination with other compounds. When administered with another agent, the co-administration can be in any manner in which the pharmacological effects of both are manifest in the patient at the same time. Thus, co-administration does not require that a single pharmaceutical composition, the same dosage form, or even the same route of administration be used for administration of both the compound of this invention and the other agent or that the two agents be administered at precisely the same time. However, co-administration will be accomplished most conveniently by the same dosage form and the same route of administration, at substantially the same time. Obviously, such administration most advantageously proceeds by delivering both active ingredients simultaneously in a novel pharmaceutical composition in accordance with the present invention.

### Compounds for use in Methods of Treatment

In aspects of the disclosure, a method is provided for increasing tissue and/or cellular oxygenation, the method comprising administering to a subject in need thereof any of the compounds or compositions of the invention as claimed.

In aspects of the invention, a compound or composition according to the invention is provided for use in a method for increasing oxygen affinity of hemoglobin S in a subject, the method comprising administering to a subject in need thereof any of the compounds or compositions of the invention as claimed.

In aspects of the invention, a compound or composition according to the invention is provided for use in a method for treating a condition associated with oxygen deficiency, the method comprising administering to a subject in need thereof any of the compounds or compositions of the invention as claimed.

In further aspects of the invention, a compound or composition according to the invention is provided for use in a method for treating oxygen deficiency associated with sickle cell anemia, the method comprising administering to a subject in need thereof any of the compounds or compositions of the invention as claimed.

In further aspects of the invention, a compound or composition according to the invention is provided for use in a method for treating sickle cell disease, the method comprising administering to a subject in need thereof any of the compounds or compositions of the invention as claimed. In still further aspects of the invention, a compound or composition according to the invention is provided for use in a method for treating cancer, a pulmonary disorder, stroke, high altitude sickness, an ulcer, a pressure sore, Alzheimer's disease, acute respiratory disease syndrome, and a wound, the method comprising administering to a subject in need thereof any of the compounds or compositions of the invention as claimed.

### Synthetic Methods

Certain methods for making the compounds described herein are also provided. The reactions are preferably carried out in a suitable inert solvent that will be apparent to the skilled artisan upon reading this disclosure, for a sufficient period of time to ensure substantial completion of the reaction as observed by thin layer chromatography, ¹H-NMR, etc. If needed to speed up the reaction, the reaction mixture can be heated, as is well known to the skilled artisan. The final and the intermediate compounds are purified, if necessary, by various art known methods such as crystallization, precipitation, column chromatography, and the likes, as will be apparent to the skilled artisan upon reading this disclosure.

An illustrative and non-limiting method for synthesizing a compound of formula (I), is schematically shown below.
In the following Schemes, 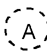 , 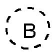 and 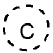 refer to rings A, B and C as described herein.
A⁵ and B⁵ are independently NR⁷⁰, O, S, S(O)x, NBoC, CH₂, CHR⁷⁰, C(R⁷⁰)₂ provided that when only one of A⁵ or B⁵ is present, then A⁵ or B⁵ is not CH₂, CHR⁷⁰, C(R⁷⁰)₂, and when both A⁵ and B⁵ are present in a ring, both are not CH₂, CHR⁷⁰, C(R⁷⁰)₂;
wherein R⁷⁰ is C₁-C₆ alkyl or defined as R¹⁴ as defined herein;
X, and X⁵ represent a leaving group and are independently selected from Cl, F, Br, and I.
R⁷¹ is C₁-C₆ alkyl;
R⁷² is C₁-C₆ alkyl;
n is 0, 1, or 2; and
Where variables already used in the structures hereinabove are used in the shcemes, the context makes it unambiguous as to what the variable refers to.

### General Synthetic Schemes

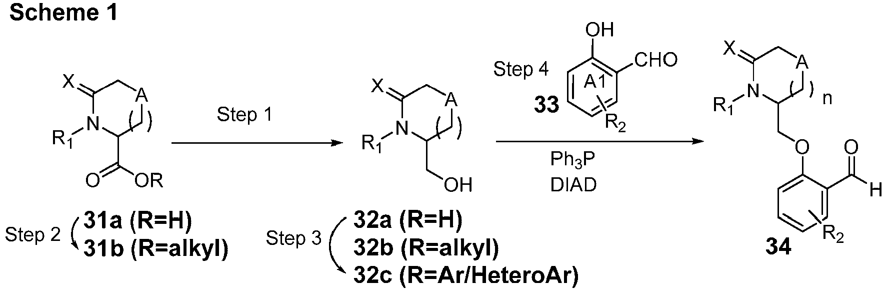

Compounds of structure **34** can be synthesized via general synthetic scheme 1. Reduction of carboxylic acid derivative **31** gives hydrxoymethyl analog **32,** which can be N-derivativtized at via copper-mediated N-arylation reaction (CuI, Ar-I, base such as N,N-dimethylethylenediamine and potassium phosphate, heat) to give key hydroxymethyl intermediate **32.** Coupling of **32** with phenol aldehyde **33** produces the desired aldehyde analog **34** via typical Mistunobu conditions using either triphenylphosphine or polymer supported triphenylphosphine.

**General method step 1- reduction of carboxylic acid derivative 1 to methyl alcohol 2:** To a suspension of carboxylic acid **1**(1-10mmol) in MeOH or EtOH (2-10 mL) at 0 °C was added SOCl₂ (1.5eq). After stirred at room temperature for 1-12h, it was concentrated to remove all solvents, dried under high vacuum to give corresponding methyl or ethyl ester. The ester was dissolved in MeOH or EtOH (5-30 mL), to this solution, was added NaBH₄ (1-4eq) at 0 °C, the mixture was warmed up to room temperature and stirred for additional 1-24 h. The mixture was quenched with Sat. NH₄Cl, filtered off the insolubles and the filtrate was concentrated to give crude product, which was purified by flash silica gel chromatography to give the corresponding hydroxymethylene compound **32.**

**General method step 2** - **N-alkylation (1a to 1b):** The carboxylate **31a** (R₁=H) can be first alkylated and then reduced to give N-alkyl hydroxymethylene analog **31b** (R₁=alkyl). In a typical procedure, the carboxylate **31a** (1-10mmol) is first dissolved in DMF (2-20 mL); to this was then added a base such as NaH or Cs₂CO₃ (1-1.2eq), followed by the addition of alkyl halide (eg, BnBr) (0.9-1.5eq). The reaction allowed to proceed at room temperature of heat at 40 to 115 °C for 0.5 to 24 h. In workup A, water was added to the reaction mixture, the precipitated product was collected, washed with water, and then subjected to preparative HPLC or flash silica gel chromatography purification. In workup B (for products that did not precipitate), diluted HCI or aqueous NH₄Cl was added at 0 °C to adjusted the pH to ∼7, the reaction mixture was partitioned between ethyl acetate or dichloromethane and aqueous sodium chloride and the organic layer separated, dried, and solvent removed under vacuum to afford crude product which was purified by automated silica gel column chromatography, reaction appropriate solvents mixture (e.g., ethyl acetate/hexanes).

**General method step 3** - **Copper-mediated N-arylation from 32a to 32c:** For cyclic amines (X=H, H), to a solution of hydroxymethylene compound **32a** (1-10 mmol) and aryl/hetero iodide (1-1.5eq) in iPrOH (0.5-10 mL) was added ethylene diol (1.3eq) and CuI (6.7mol%), followed by K₃PO₄ (1.3eq), then it was degassed and heated at 88 °C for 6-24 h. Alternatively, for lactams (X=O), to a solution of hydroxymethylene compound **32a** (1-10mmol) and aryl/hetero iodide (1-1.5eq) in Dioxane (2-20 mL) was added CuI (0.17eq), N,N-dimethylethylenediamine (0.17eq), K₃PO₄ (1.7eq), then it was degassed and heated at 100 °C for 6-48 h.

Workup for both procedures: the reaction mixture was cooled to room temperature the mixture was diluted with EtOAc and water, organic layer was separated and the aqueous layer was extracted with EtOAc, organic layer was combined, washed with brine, dried and concentrated to give crude product, which was purified by flash silica gel chromatography to give N-aryl/heteroaryl compound **32c.**

**General method C-Mitsunobu conditions** A hydroxyl (hetero)arylaldehyde derivatives (**34**) (0.1-2 mmol) mixture with substituted methylene alcohol (**33**) (0.8 to 1.2eq) and (polymer-supported) PPh₃ (1-1.5eq) in anhydrous THF (1-10mL) was stirred under nitrogen until complete dissolution. The solution was cooled to 0 °C on ice bath and DIAD or DEAD (1.1 eq) in THF or toluene was added dropwise over a 1-20 min period. The ice cooling bath was allowed to expire over 90 min and the mixture was stirred at RT for 2-48 hours. The mixture was filtered through a pad of silica. The silica was washed with ethyl acetate 2-20mL. The combined filtrates were evaporated and the residue was dried on highvac. The residue was purified by preparative HPLC or flash silica gel chromatography.

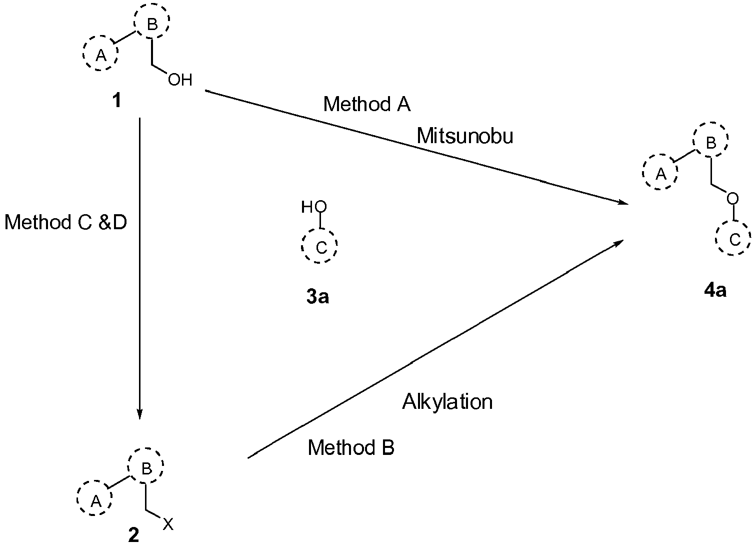

**General method A for preparing aryloxy ether analogs (4a) from substituted methylene alcohol (1) and hydroxyl aryl aldehyde derivatives (3a).** A hydroxyl (hetero)arylaldehyde derivatives (3a) (0.1-2 mmol) mixture with substituted methylene alcohol (1) (0.8 to 1.2eq) and PPh₃ (1-1.5eq) in anhydrous THF (1-10mL) was stirred under nitrogen until complete dissolution. The solution was cooled to 0 °C on ice bath and DIAD or DEAD (1.1 eq) in THF or toluene was added dropwise over a 1-20 min period. The ice cooling bath was allowed to expire over 90 min and the mixture was stirred at RT for 2-48 hours. The mixture was stirred for 10 min, then filtered through a pad of silica. The silica was washed with ethyl acetate 2-20mL. The combined filtrates were evaporated and the residue was dried on highvac. The residue was purified by preparative HPLC or flash silica gel chromatography.

**General method B for preparing aryloxyether analogs (4a) from substituted methylene halide (2) and hydroxyl aryl aldehyde derivatives (3a).** A mixture of hydroxyl (hetero)arylaldehyde derivatives (**3a**) (0.1-2 mmol, 1-4 eq.), substituted methylene chloride or bromide (**2**) (1eq), and K₂CO₃ (2-5 eq.) (catalytic amount of NaI or Bu₄NI may also be added) in DMF or acetonitrile (1 to 10 mL) was stirred at RT or heating up to 120 °C for 0.5-8 h under nitrogen atmosphere. In workup A, water was added to the reaction mixture, the precipitated product was collected, washed with water, and then subjected to preparative HPLC or flash silica gel chromatography purification. In workup B (for products that did not precipitate), diluted HCI or aqueous NH₄Cl was added at 0 °C to adjusted the pH to ∼7, the reaction mixture was partitioned between ethyl acetate or dichloromethane and aqueous sodium chloride and the organic layer separated, dried, and solvent removed under vacuum to afford crude product which was purified by automated silica gel column chromatography using appropriate solvents mixture (e.g., ethyl acetate/hexanes).

**General method C for preparing substituted methylene chloride (2a).** To a solution of substituted methylene alcohol (**1**) (0.1 to 2 mmol) in DCM (1-10 mL) was added SOCl₂ dropwise (2eq to 5eq) at 0 °C or RT. The reaction mixture was stirred at RT for 10min to 6 h, or until reaction is judged complete (LC/MS). The reaction mixture is concentrated to dryness over a rotavap. The crude chloride residue was suspended in toluene, sonicated and concentrated to dryness. The process was repeated three times and dried under vacuum to give the substituted methylene chloride (**2**), usually as an off-white solid, which was used for next step without further purification. Alternatively, a solution of aqueous 1N Na₂CO₃ is then added to produce a solution of pH∼ 8. the mixture was extracted with DCM (3 x10-50mL), dried over sodium sulfate, and concentrated to the crude substituted methylene chloride (**2a**), which is then purified by column chromatography on silica gel (0-100% ethyl acetate-hexanes).

**General method D for preparing substituted methylene bromide (2b).** To a solution of substituted methylene alcohol (**1**) (0.1 to 2 mmol) in DCM (1-10 mL) was added Ph₃P Br₂ dropwise (2eq to 5eq) at 0 °C or RT. The reaction mixture was stirred at RT for 10 min to 2 h, or until reaction is judged complete (LC/MS). The reaction mixture is concentrated to dryness over a rotavap. The residue purified by column chromatography on silica gel (0-100% ethyl acetate-hexanes) to afford the pure bromide **2b.**

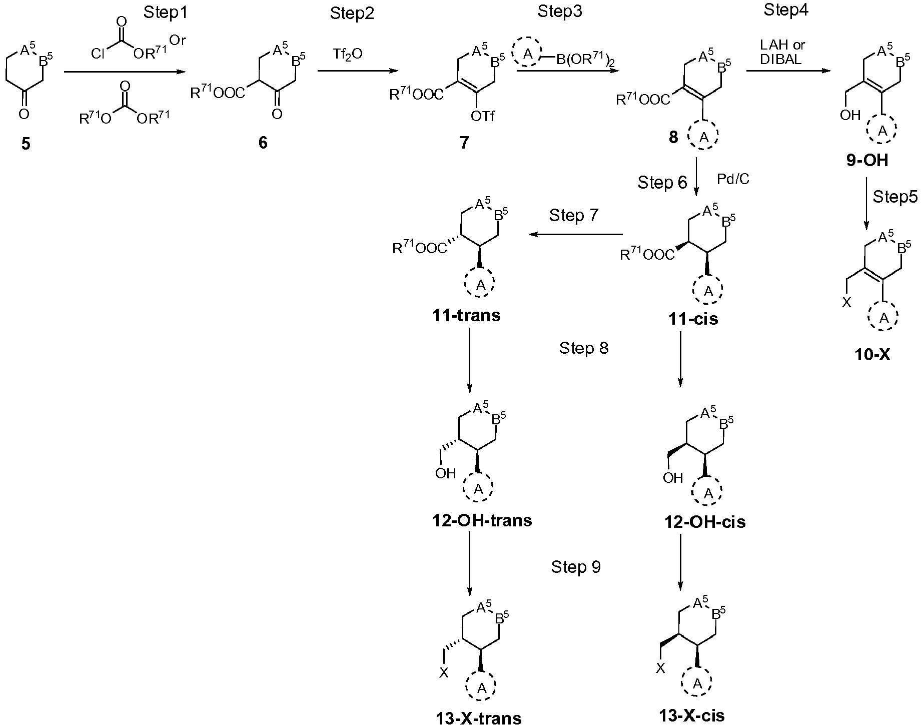

**General method E for preparing heterocyclic methylene derivatives 9, 10, 12 and 13.** Condensation of heterocyclic ketone analog **5** with chlorformate or dialkyl carbonate gives (hetero)cyclic beta-ketone ester **6** (Step 1). The ketone ester **6** is converted to the triflate intermediate **7** by treating with a triflating agent (e.g, triflic anhydride) in the presence of an organic base such as Hunig's base (Step 2). Suzuki coupling of the triflate **7** with a boronic acid or ester affords heterocyclohexene carboxylate **8** (Step 3). Subsequent reduction of the ester group by LAH or DIBAL gives the corresponding alcohol **9-OH** (Step 4). Further reaction of the alcohol **9-OH** with thionyl chloride, Ph₃PBr₂ (or CBr₄-Ph₃P or PBr₃), or alkyl/aryl sufonyl chloride produces the corresponding **10-X** chloride, bromide or sulfonate (Step 5).

Alternatively, the double bond of heterocyclohexene carboxylate **8** is reduced to give the *cis*-heterocyclohexane **11-cis** carboxylate under palladium catalyzed hydrogenation conditions (Step 6). Reduction of the ester group of **11-cis** by LAH or DIBAL yields cis-alcohol **12-OH-cis** (Step 8). Conversion of the alcohol **12-OH-cis** to its chloride, bromide or sulfonate (such as mesylate, tosylate) **13-X-cis** can be achieved by reacting with thionyl chloride, or Ph₃PBr₂, or sufonyl chloride (such as mesyl chloride or tosyl chloride) (Step 9). The *cis-*cyclohexane carboxylate **11-cis** can also be isomerized to the thermodynamically more stable trans-isomer **11-trans** by the treatment with an alcoholic alkoxide (e.g., ethoxide) solution. Analogously, transformation of **11-trans** ester to **12-trans** alcohol and **13-X-trans** halide is accomplished by applying conditions of Step 8 and Step9 similar to these for the corresponding cis-isomers.

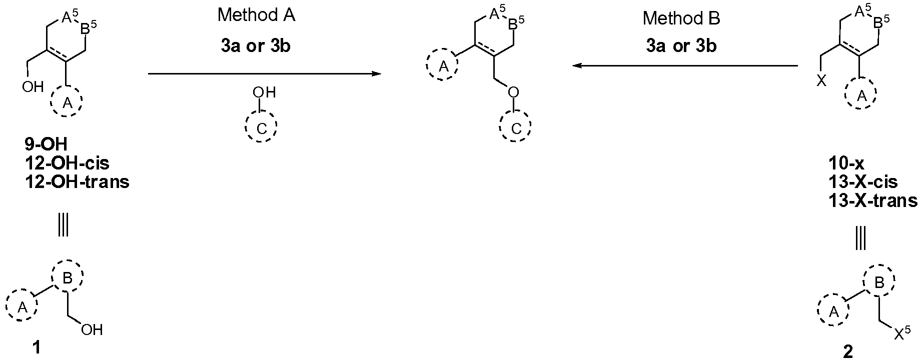

Coupling of the (hetero)cyclic methylene derivatives **9, 10, 12** and **13** with hydroxyl (hetero)arylaldehyde derivatives (**3a/3b**) by general method A or B affords the corresponding aryloxy/heteroarylether analogs (**4c** and **4d**).

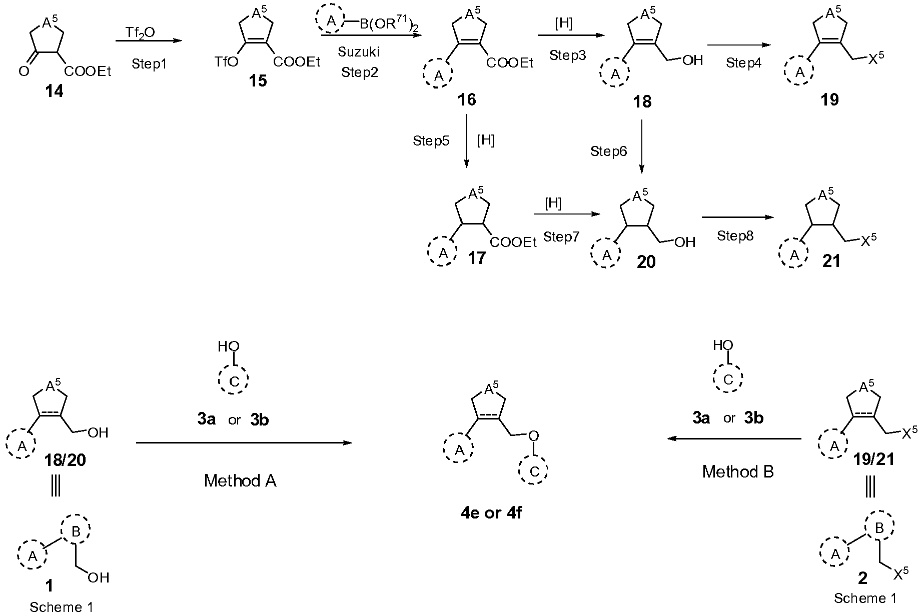

**General method F Scheme 2 for preparing heterocyclic methylene derivatives 18, 19, 20 and 21.** The ketone ester **14** is converted to the triflate intermediate **15** by treating with a triflating agent (e.g, triflic anhydride) in the presence of an organic base such as Hunig's base (Step 1). Suzuki coupling of the triflate **15** with a boronic acid or ester affords heterocyclo carboxylate **16** (Step 2). Subsequent reduction of the ester group by LAH or DIBAL gives the corresponding alcohol **18** (Step 3). Further reaction of the alcohol **18** with thionyl chloride, Ph₃PBr₂ (or CBr₄-Ph₃P or PBr₃), or alkyl/aryl sufonyl chloride produces the corresponding **19** chloride, bromide or sulfonate (Step 4).

Alternatively, the double bond of **16** is reduced to give the saturated heterolic analog **17** under palladium catalyzed hydrogenation conditions (Step 5). Reduction of the ester group of **17** by LAH or DIBAL yields alcohol **20** (Step 7). Conversion of the alcohol **20** to its chloride, bromide or sulfonate (such as mesylate, tosylate) **21** can be achieved by reacting with thionyl chloride, or Ph₃PBr₂, or sufonyl chloride (such as mesyl chloride or tosyl chloride) (Step 8).

Coupling of the (hetero)cyclic methylene derivatives **18, 19, 20** and **21** with hydroxyl (hetero)arylaldehyde derivatives (**3a/3b**) by general method A or B affords the corresponding aryloxy/heteroaryloxyether analogs (**4e** and **4f**).

Chiral pyrrolidine methylene derivatives **25** and **26** can be prepared according to reaction sequence depicted herein. The pyrrolidine ester **24** is produced via a 1,3-dipolar cycloaddition of alkene **22** with azomethine-ylide generated in situ from formaldehyde and amino acid **23** alkene (Step1). Subsequent reduction of the ester to alcohol **24** and further conversion **25** are accomplished by analogous methods described herein. If a chiral auxiliary group such as chiral oxazolidinone derivative **22a** is used, optically active pyrrolidine derivatives **25** and **26** can also be obtained. Coupling of **25** and **26** with hydroxyl (hetero)arylaldehyde derivatives (**3a/3b**) by general method A or B affords the corresponding aryloxy/heteroaryloxyether analogs (**4**).

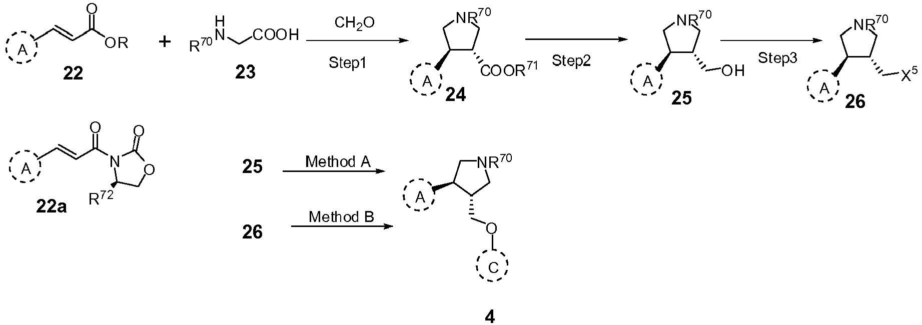

Separate from the general synthesis of tetrahydrothiophenes (i.e., **20** and **21,** A⁵=S) described herein, also described is a different synthetic approach to this class of analogs.

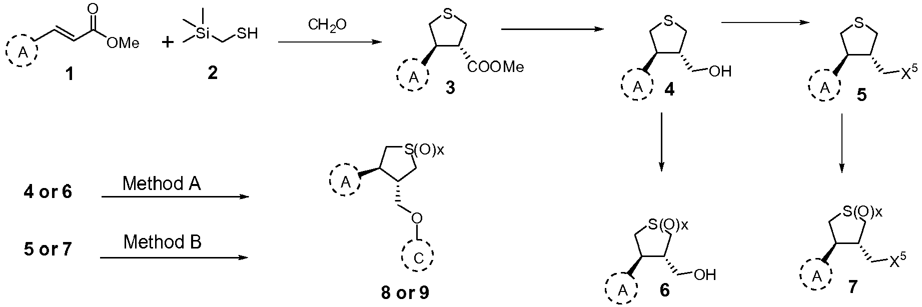

Other heterocyclic analogs (compound 5) with C-N linkage are synthesized by applying Buchwald/Hartwig amination conditions. Many of the cyclic amines (1) are available commercially (e.g., 1a, 1b, 1c, 1d, and 1e).

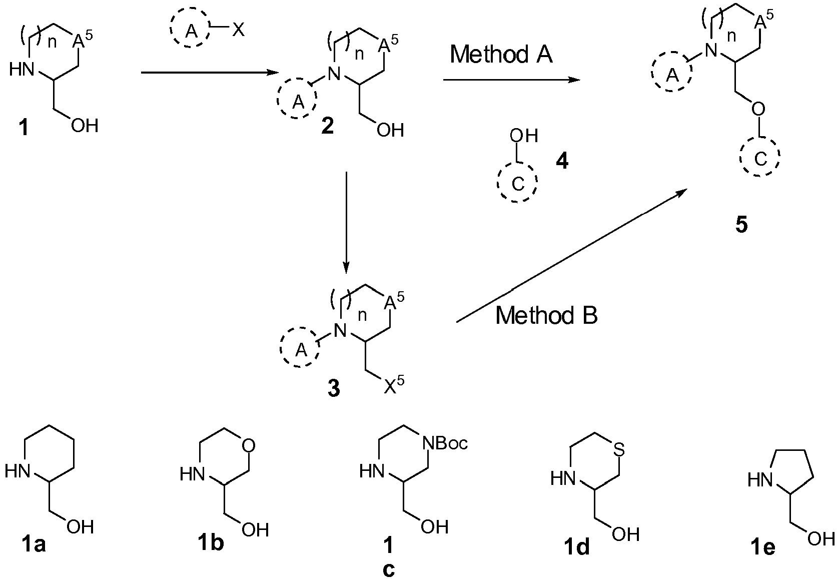

Protected amides of formula -CONHR⁹⁵ and -CONHOR⁹⁵ can be converted e.g., hydrolyzed to the corresponding amides according to methods known to the skilled artisan.

### Prodrug Synthesis

Syntheses of the ester prodrugs start with the free carboxylic acid bearing the tertiary amine. The free acid is activated for ester formation in an aprotic solvent and then reacted with a free alcohol group in the presence of an inert base, such as triethyl amine, to provide the ester prodrug. Activating conditions for the carboxylic acid include forming the acid chloride using oxalyl chloride or thionyl chloride in an aprotic solvent, optionally with a catalytic amount of dimethyl formamide, followed by evaporation. Examples of aprotic solvents, include, but are not limited to methylene chloride, tetrahydrofuran, and the like. Alternatively, activations can be performed in situ by using reagents such as BOP (benzotriazol-l-yloxytris(dimethylamino) phosphonium hexafluorolphosphate, and the like (see Nagy et al., 1993, Proc. Natl. Acad. Sci. USA 90:6373-6376) followed by reaction with the free alcohol. Isolation of the ester products can be affected by extraction with an organic solvent, such as ethyl acetate or methylene chloride, against a mildly acidic aqueous solution; followed by base treatment of the acidic aqueous phase so as to render it basic; followed by extraction with an organic solvent, for example ethyl acetate or methylene chroride; evaporation of the organic solvent layer; and recrystalization from a solvent, such as ethanol. Optionally, the solvent can be acidified with an acid, such as HCI or acetic acid to provide a pharmaceutically acceptable salt thereof. Alternatively the crude reaction can be passed over an ion exchange column bearing sulfonic acid groups in the protonated form, washed with deionized water, and eluted with aqueous ammonia; followed by evaporation.

Suitable free acids bearing the tertiary amine are commercially available, such as 2-(N-morpholino)-propionic acid, N,N- dimethyl-beta-alanine, and the like. Non- commercial acids can be synthesized in straightforward manner via standard literature procedures.

Carbonate and carbamate prodrugs can be prepared in an analogous way. For example, amino alcohols and diamines can be activated using activating agents such as phosgene or carbonyl diimidazole, to provide an activated carbonates, which in turn can react with the alcohol and/or the phenolic hydroxy group on the compounds utilized herein to provide carbonate and carbamate prodrugs.

Various protecting groups and synthetic methods related to them that can be used or adapted to make compounds of the invention can be adapted from the references Testa et al., Hydrolysis in Drug and Prodrug Metabolism, June 2003, Wiley- VCH, Zurich, 419-534 and Beaumont et al., Curr. Drug Metab. 2003, 4:461-85.

Described herein is a method of synthesizing an acyloxymethyl version of a prodrug by adapting a method from the reference Sobolev et al., 2002, J. Org. Chem. 67:401-410. 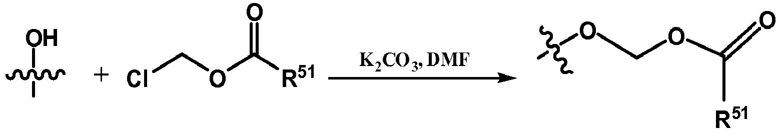 R⁵¹ is C₁-C₆ alkyl.

Described herein is a method for synthesizing a phosphonooxymethyl version of a prodrug by adapting a method from Mantyla et al., 2004, J. Med. Chem. 47:188-195.

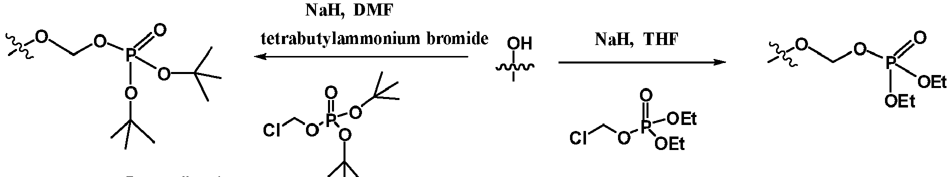

Described herein is a method of synthesizing an alkyloxymethyl version of a prodrug 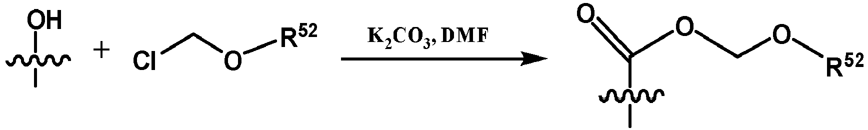 R⁵² is C₁-C₆ alkyl, C₃-C₈ cycloalkyl, C₃-C₉ heterocyclyl, C₆-C₁₀ aryl, or C₃-C₉ heteroaryl.

### Examples

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention. Changes therein and other uses which are encompassed within the invention as defined by the scope of the claims will occur to those skilled in the art.

In the examples below as well as throughout the application, the following abbreviations have the following meanings. If not defined, the terms have their generally accepted meanings.

| | |
|---|---|
| °C | = degrees Celsius |
| RT | = Room temperature |
| min | = minute(s) |
| h | = hour(s) |
| µL | = Microliter |
| mL | = Milliliter |
| mmol | = Millimole |
| eq | = Equivalent |
| mg | = Milligram |
| ppm | = Parts per million |
| atm | = Atmospheric pressure |
| MS | = Mass spectrometry |
| LC-MS | = Liquid chromatography-mass spectrometry |
| HPLC | = High performance liquid chromatography |
| NMR Sat. | = Nuclear magnetic resonance Saturated |
| MeOH | = Methanol |
| EtOH | = Ethanol |
| EtOAc | = Ethyl acetate |
| Et₃N | = Triethylamine |
| ACN | = Acetonitrile |
| Ac₂O | = Acetic anhydride |
| Na(OAc)₃BH | = Sodium triacetoxy borohydride |
| PBr₃ | = phosphorus tribromide |
| Ph₃P | = Triphenylphosphine |
| Ph₃PBr₂ CBr₄ | = Triphenylphosphine dibromide Tetrabromomethane |
| DMF | = N, N-Dimethylformamide |
| DCM | = Dichloromethane |
| LAH/ LiAlH₄ | = Lithium aluminum hydride |
| THF | = Tetrahydrofuran |
| DIBAL | = Diisobutylaluminium hydride |
| DIAD | = Diisopropyl azodicarboxylate |
| DEAD | = Diethyl azodicarboxylate |
| DIPEA | = N,N-Diisopropylethylamine |
| Tf₂O | = Trifluoromethanesulfonic (triflic) anhydride |
| Pd(dppf)Cl₂ | = [1,1'-Bis(diphenylphosphino)ferrocene] dichloropalladium(II), complex |

### Preparation of 2-hydroxy-6-((5-(1-isopropyl-1H-pyrazol-5-yl)-3,6-dihydro-2H-pyran-4-yl)methoxy)benzaldehyde

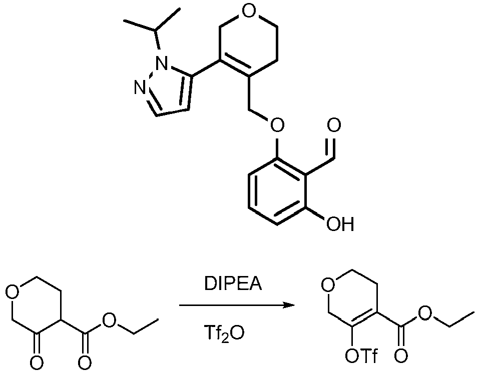

**Step 1:** To a solution of ethyl 3-oxotetrahydro-2H-pyran-4-carboxylate (1.0 g, 5.81 mmol)in DCM (30 mL) was added DIPEA (1.22 mL, 6.97 mmol) and Tf₂O (1.08 mL, 6.39 mmol) at -78 °C, then it was warmed up to room temperature and stirred at room temeperature for 2 h, the solution was diluted with DCM, washed with Sat. NaHCO₃, brine, dried and concentrated to give ethyl 5-(((trifluoromethyl)sulfonyl)oxy)-3,6-dihydro-2H-pyran-4-carboxylate as crude product (2 g). 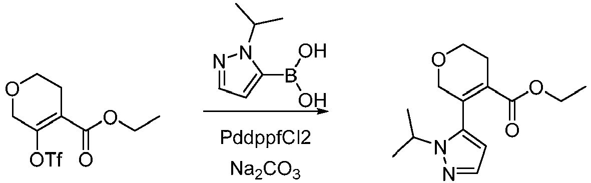
**Step 2:** To a solution of ethyl 5-(((trifluoromethyl)sulfonyl)oxy)-3,6-dihydro-2H-pyran-4-carboxylate (crude from step 1) and 1-isopropyl-5-(4,4,5,5-tetramethyl-1,3,2-dioxaborolan-2-yl)-1H-pyrazole (1.37 g, 5.82 mmol) in dioxane (20 ml) was added Pd(dppf)Cl₂ (430 mg, 0.58 mmol) and Na₂CO₃ (1.85 g, 17.46 mmol) in water (6 mL), the mixture was degased with N2 for 5 min, and was heated at 100 °C for 15 h, after cooling to room temperature the mixture was diluted with EtOAc and washed with Sat. NaHCO₃ and brine, organic layer was combined, dried and concentrated to give crude product, which was purified by column chromatography (Hexanes/EtOAc=3:1) to give ethyl 5-(1-isopropyl-1H-pyrazol-5-yl)-3,6-dihydro-2H-pyran-4-carboxylate (850 mg). 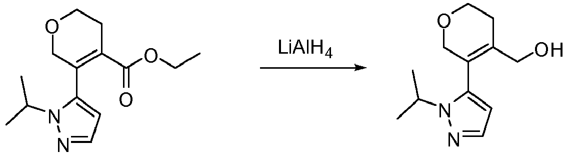
**Step 3:** To a solution of ethyl 5-(1-isopropyl-1H-pyrazol-5-yl)-3,6-dihydro-2H-pyran-4-carboxylate (600 mg, 2.27 mmol) in THF (10 mL) was added LiAlH₄ (1M in THF, 2.72 mL, 2.72 mmol) at -20 °C, the reaction was stirred at -20 °C for 30 min, and was quenched with Sat. NH₄Cl, the aqueous layer was extracted with EtOAc, the combined organics were washed with brine, dried and concentrated to give crude oil, which was purified by column (Hexanes/EtOAc= 100:0 to 20:80) to give (5-(1-isopropyl-1H-pyrazol-5-yl)-3,6-dihydro-2H-pyran-4-yl)methanol (500 mg). 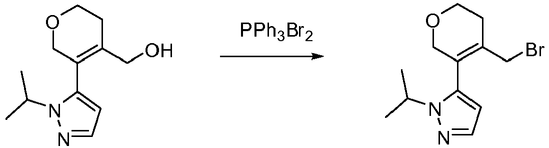
**Step 4:** To a solution of (5-(1-isopropyl-1H-pyrazol-5-yl)-3,6-dihydro-2H-pyran-4-yl)methanol (300 mg, 1.35 mmol) in DCM (5 mL) was added dibromotriphenylphosphorane (630 mg, 1.35 mmol) at room temperature, after stirring for 30 min, it was diluted with DCM, organic layer was washed with Sat. NaHCO₃, brine, dried and concentrated to give crude product, which was purified by column(Hexanes/EtOAc= 4:1) to give 5-(4-(bromomethyl)-5,6-dihydro-2H-pyran-3-yl)-1-isopropyl-1H-pyrazole (360 mg). 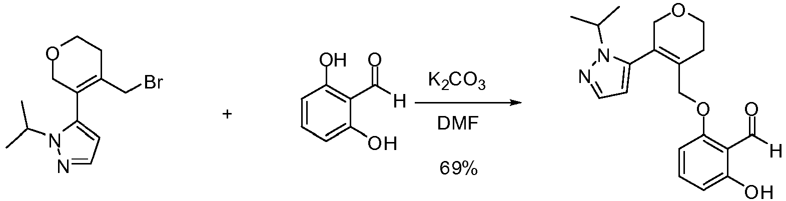
**Step 5:** To a solution of 5-(4-(bromomethyl)-5,6-dihydro-2H-pyran-3-yl)-1-isopropyl-1H-pyrazole (110 mg, 0.38 mmol) and 2,6-dihydroxybenzaldehyde (100 mg, 0.76 mmol) in DMF (6 mL) was added K₂CO₃ (110 mg, 0.76 mmol). After stirred at room temperature for 1 h, it was diluted with water and EtOAc, organic layer was separated, and the aqueous layer was extracted with EtOAc. Organic layer was combined, washed with brine, dried and concentrated to give crude product, which was purified by column (Hexanes/EtOAc=1:1) to give 2-hydroxy-6-((5-(1-isopropyl-1H-pyrazol-5-yl)-3,6-dihydro-2H-pyran-4-yl)methoxy)benzaldehyde (90 mg). 1H NMR (400 MHz, CDCl₃) δ (ppm) 11.89 (s, 1H), 10.33 (s, 1H), 7.53 (d, J=1.6 Hz, 1H), 7.33(t, J=8.8 Hz, 1H), 6.51 (d, J=8.8 Hz, 1H), 6.16 (d, J=8.0 Hz, 1H), 6.08 (d, J=2.0 Hz, 1H), 4.40 (dd, J = 12.8, 6.4 Hz, 1H), 4.35 (s, 2H), 4.18 (s, 2H), 3.97 (t, J=5.2 Hz, 2H), 2.44 (s, 2H), 1.40 (d, J=6.4 Hz, 6H); MS (ESI) m/z 343.3 [M+H]⁺.

### Preparation of 2-[[1-acetyl-5-(2-propan-2-ylpyrazol-3-yl)-3,6-dihydro-2H-pyridin-4-yl]methoxy]-6-hydroxybenzaldehyde

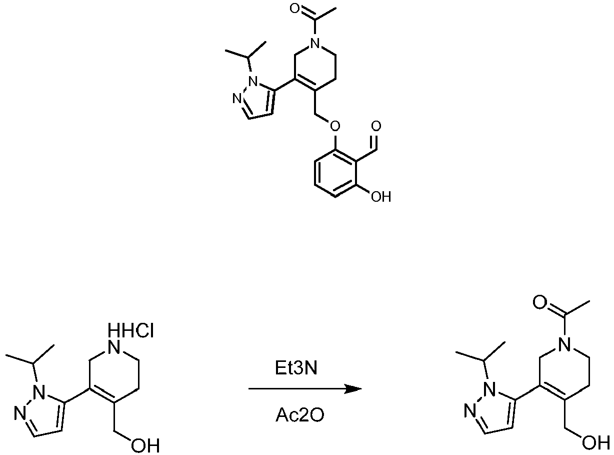

**Step 1:** To a solution of (5-(1-isopropyl-1H-pyrazol-5-yl)-1,2,3,6-tetrahydropyridin-4-yl)methanol hydrochloride (110 mg, 0.41 mmol) in DCM (2 mL) at 0 °C was added Et₃N (0.12 mL, 0.82 mmol) and a solution of Ac₂O (0.04 mL, 0.41 mmol) in DCM (0.4 mL), after stirred for 15 min, it was diluted with Sat. NH₄Cl and EtOAc, organic layer was separated and the aqueous layer was further extracted with EtOAc, organic layers were combined, washed with Sat. NaHCO₃, brine, dried over Na₂SO₄, and was concentrated to give 1-(4-(hydroxymethyl)-5-(1-isopropyl-1H-pyrazol-5-yl)-3,6-dihydropyridin-1(2H)-yl)ethan-1-one as crude product. 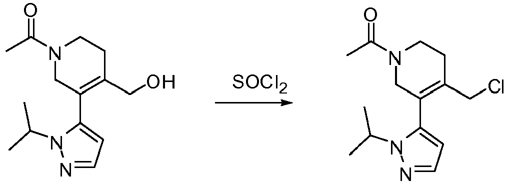
**Step 2:** To a solution of 1-(4-(hydroxymethyl)-3-phenyl-5,6-dihydropyridin-1(2H)-yl)ethanone (88 mg, 0.41 mmol) in DCM (2 mL) was added SOCl₂ (0.58 mL, 8.25 mmol). After stirred at RT for 15 min, the mixture was concentrated and dried under high vacuum to give 1-(4-(chloromethyl)-5-(1-isopropyl-1H-pyrazol-5-yl)-3,6-dihydropyridin-1(2H)-yl)ethan-1-one as crude product (80 mg). 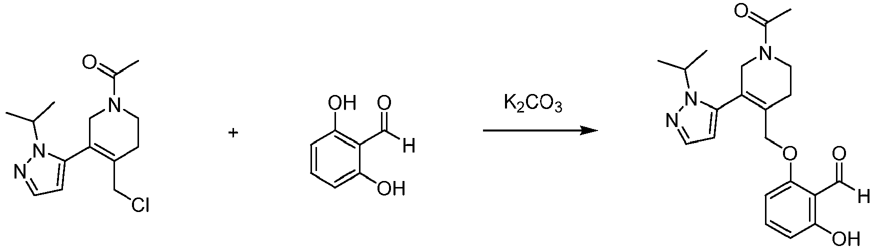
**Step 3:** To a suspension of K₂CO₃ (80 mg, 0.56 mmol) and 2,6-dihydroxybenzaldehyde (80 mg, 0.56 mmol) in DMF (2 ml) was added a solution of 1-(4-(chloromethyl)-5-(1-isopropyl-1H-pyrazol-5-yl)-3,6-dihydropyridin-1(2H)-yl)ethan-1-one (80 mg, 0.28 mmol) in DMF (2 mL), the mixture was heated at 50 °C for 3 h, cooled to room temperature, and was diluted with EtOAc, organic layer was separated and aqueous layer was extracted with EtOAc. EtOAc layers were combined, washed with Sat. NaHCO₃, brine, dried over Na₂SO₄, and was concentrated to give crude oil, which was purified by preparative HPLC (eluted with ACN/H₂O) to give 2-((1-acetyl-5-(1-isopropyl-1H-pyrazol-5-yl)-1,2,3,6-tetrahydropyridin-4-yl)methoxy)-6-hydroxybenzaldehyde (9 mg). 1H NMR (400 MHz, CDCl₃, NMR shows rotamer exist, only one set of signal was reported) δ (ppm) 11.87 (s, 1H), 10.34 (s, 1H), 7.54 (d, J = 1.6 Hz, 1H), 7.34 (t, J = 8.0 Hz, 1H), 6.53 (d, J = 8.4 Hz, 1H), 6.15 (d, J = 8.4 Hz, 1H), 6.11(d, J = 1.6 Hz, 1H), 4.36 (s, 2H), 4.34 (m, 1H), 4.21 (s, 2H), 3.71 (t, J = 6.0 Hz, 2H), 2.51 (m, 2H), 2.19 (s, 3H), 1.42 (d, J = 6.8 Hz, 6H); MS (ESI) m/z 384.3 [M+H]⁺

### Preparation of 2-hydroxy-6-[[1-methyl-5-(2-propan-2-ylpyrazol-3-yl)-3,6-dihydro-2H-pyridin-4-yl]methoxy]benzaldehyde

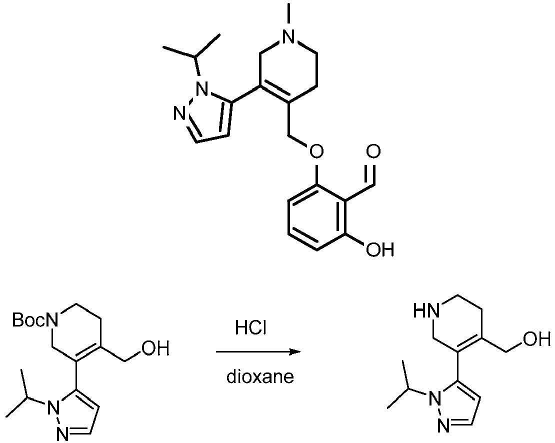

**Step 1:** To a solid of tert-butyl 4-(hydroxymethyl)-5-(1-isopropyl-1H-pyrazol-5-yl)-3,6-dihydropyridine-1(2H)-carboxylate (150 mg, 0.47 mmol) in round bottom flask was added 4N HCI in dioxane (3 mL) at room temeprature, and was stirred for 1 h, then the mixture was concentrated and dried under high vacuum to give (5-(1-isopropyl-1H-pyrazol-5-yl)-1,2,3,6-tetrahydropyridin-4-yl)methanol as HCI salt (120 mg). 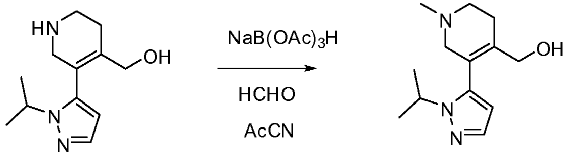
**Step 2:** To a solution of (5-(1-isopropyl-1H-pyrazol-5-yl)-1,2,3,6-tetrahydropyridin-4-yl)methanol hydrochloride in ACN (3 mL) was added Et₃N followed by formalin. After stirred at room temperature for 10 min, it was added Na(OAc)₃BH and after another 30 min, the mixture was concentrated and pass through a short silica gel column, the column was washed with 10% MeOH in CHCl₃, and then the filtrated was collected and concentrated to give crude product, which was further diluted with EtOAc, filtered to get rid of triethylamine HCI salt, the filtrate was concentrated again to give (5-(1-isopropyl-1H-pyrazol-5-yl)-1-methyl-1,2,3,6-tetrahydropyridin-4-yl)methanol (100 mg). 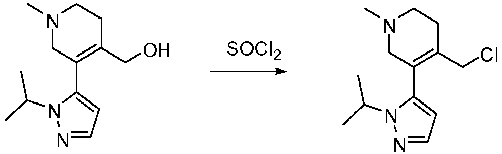
**Step 3:** To a solution of (5-(1-isopropyl-1H-pyrazol-5-yl)-1-methyl-1,2,3,6-tetrahydropyridin-4-yl)methanol (100 mg, 0.42 mmol) in DCM (2.5 mL) was added SOCl₂ (0.76 mL, 10.5 mmol) at room temperature and then was stirred at room temperature for 30 min, the mixture was concentrated and diluted with toluene and concentrated, dried under high vacuum to give 4-(chloromethyl)-5-(1-isopropyl-1H-pyrazol-5-yl)-1-methyl-1,2,3,6-tetrahydropyridine as crude product. 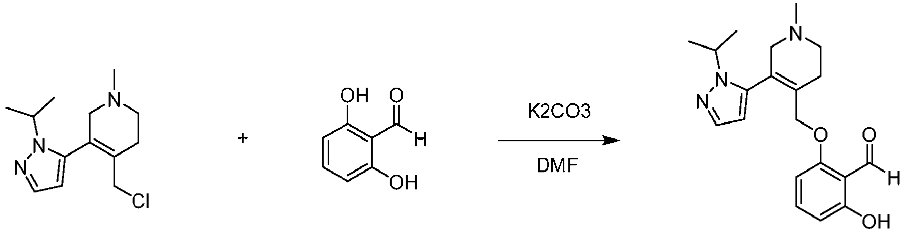
**Step 4:** To a suspension of K₂CO₃ (230 mg, 1.68 mmol) and 2,6-dihydroxybenzaldehyde (120 mg, 0.84 mmol) in DMF (2 ml) was added a solution of 4-(chloromethyl)-5-(1-isopropyl-1H-pyrazol-5-yl)-1-methyl-1,2,3,6-tetrahydropyridine (110 mg, 0.42 mmol) in DMF (3 mL), the mixture was heated at 50 °C for 4 h, cooled to room temperature, and was diluted with EtOAc, organic layer was separated and aqueous layer was extracted with EtOAc. EtOAc layer was combined, washed with Sat. NaHCO₃, brine, dried over Na₂SO₄, and was concentrated to give crude oil, which was purified by column (Hexane/EtOAc= 65:35 followed by DCM/MeOH= 95:5) to give 2-hydroxy-6-((5-(1-isopropyl-1H-pyrazol-5-yl)-1-methyl-1,2,3,6-tetrahydropyridin-4-yl)methoxy)benzaldehyde (44 mg). 1H NMR (400 MHz, CDCl₃) δ (ppm) 11.89 (s, 1H), 10.34 (s, 1H), 7.52 (d, J = 1.6 Hz, 1H), 7.31 (dd, J = 8.4, 7.2 Hz, 1H), 6.51 (d, J = 8.4 Hz, 1H), 6.16 (d, J = 7.2 Hz, 1H), 6.07 (d, J =1.6 Hz, 1H), 4.36 (m, 1H), 4.34 (s, 2H), 3.07 (s, 2H), 2.71 (s, 2H), 2.52 (s, 2H), 2.43 (s, 3H), 1.41 (d, J = 6.4 Hz, 6H); MS (ESI) m/z 356.3 [M+H]⁺.

The following exemplary A-ring and B-ring intermediates may also be incorporated into the compounds of the invention.

### Preparation of:

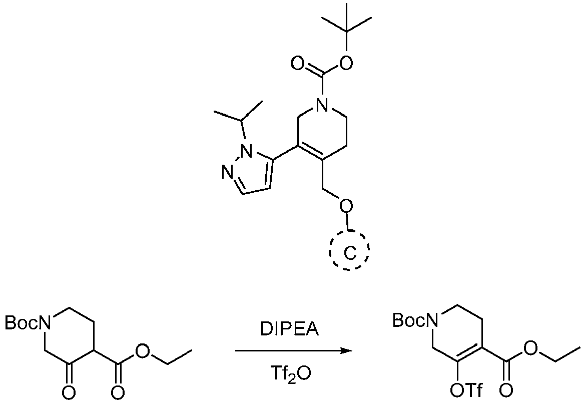

**Step 1:** To a solution of 1-tert-butyl 4-ethyl 3-oxopiperidine-1,4-dicarboxylate (2.0g, 7.37 mmol) in DCM (45 mL) was added DIPEA (1.54 ml, 8.84 mmol) and Tf₂O (1.36 mL, 8.11 mmol) at -78 °C, then the temperature was warmed up to room temperature and the solution was stirred at RT for 1.5 h, the mixture was diluted with DCM (100 mL), organic layer was washed with Sat. NaHCO₃, brine, dried and concentrated to give 1-(tert-butyl) 4-ethyl 5-(((trifluoromethyl)sulfonyl)oxy)-3,6-dihydropyridine-1,4(2H)-dicarboxylate, which was used for next step without purification. 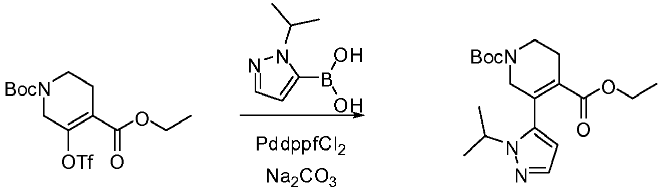
**Step 2:** To a solution of 1-tert-butyl 4-ethyl 3-(((trifluoromethyl)sulfonyl)oxy)-5,6-dihydropyridine-1,4(2H)-dicarboxylate (1.49 g, 3.7 mmol) and (1-isopropyl-1H-pyrazol-5-yl)boronic acid (0.57 g, 3.7 mmol) in dioxane (10 mL) was added Pd(dppf)Cl₂ (0.27 g, 0.37 mmol) and a solution of sodium carbonate (1.18 g, 11.10) in water (3 ml), the mixture was degased with N₂ for 5 min, and was heated at 100 °C for 15 h, after cooling to room temperature the mixture was diluted with EtOAc and washed with Sat. NaHCO₃ and brine, organic layer was combined, dried and concentrated to give crude product, which was purified by column chromatography (Hexanes/EtOAc=3:1) to give desired product 830 mg (62%). 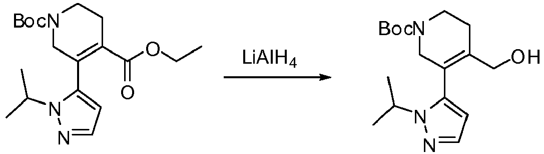
**Step 3:** To a solution of 1-(tert-butyl) 4-ethyl 5-(1-isopropyl-1H-pyrazol-5-yl)-3,6-dihydropyridine-1,4(2H)-dicarboxylate (450 mg, 1.24 mmol) in THF (6 mL) was added LiAlH₄ (1M in THF, 1.49 mL, 1.49 mmol) at -20 °C, the reaction was stirred at -20 °C for 30 min, and was quenched with Sat. NH₄Cl, the aqueous layer was extracted with EtOAc, the combined organics were washed with brine, dried and concentrated to give crude oil, which was purified by column (Hexanes/EtOAc= 100:0 to 40:60) to give tert-butyl 4-(hydroxymethyl)-5-(1-isopropyl-1H-pyrazol-5-yl)-3,6-dihydropyridine-1(2H)-carboxylate (370 mg, 91%). 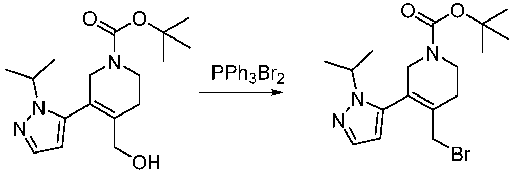
**Step 4:** To a solution of give tert-butyl 4-(hydroxymethyl)-5-(1-isopropyl-1H-pyrazol-5-yl)-3,6-dihydropyridine-1(2H)-carboxylate (25 mg, 0.08 mmol) in DCM (1 mL) was added triphenylphosphine bromine adduct (40 mg, 0.09 mmol) at room temperature, after stirring for 30 min, it was diluted with DCM, washed with Sat. NaHCO₃, brine, dried and concentrated to give crude product, which was purified by column to give tert-butyl 4-(bromomethyl)-5-(1-isopropyl-1H-pyrazol-5-yl)-3,6-dihydropyridine-1(2H)-carboxylate (18 mg).

### Preparation of 2-hydroxy-6-[[cis-3-(2-propan-2-ylpyrazol-3-yl)oxan-4-yl]methoxy]benzaldehyde

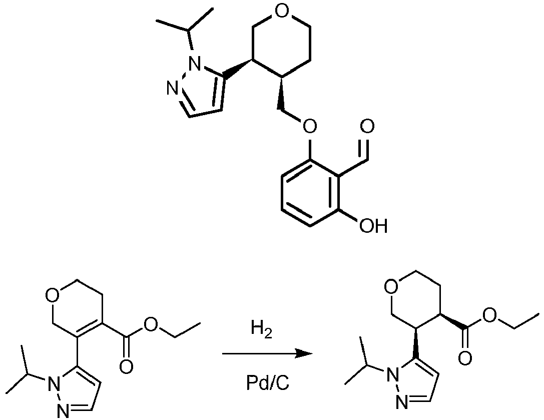

**Step 1:** To a solution of ethyl 5-(1-isopropyl-1H-pyrazol-5-yl)-3,6-dihydro-2H-pyran-4-carboxylate (100 mg, 0.38 mmol) in EtOH (2 mL) was added Pd/C (50 mg), then it was charged with H₂ (1atm) and stirred at room temperature for 3 days, Mass spec shows about 50% conversion. The mixture was then added a solution of NH₄CO₂H (200 mg) in water (2 ml) and additional Pd/C, and the mixture was further heated at 75 °C for 1.5 h, after cooled to room temperature, the reaction was diluted with EtOH, pd/C was filtered off, and the filtrate was concentrated to give crude oil, which was diluted with CHCl3, organic layer was washed with Sat. NaHCO₃, dried and concentrated to give crude product, which was purified by column (Hexanes/EtOAc=65:35) to give (±) ethyl (3S,4R)-3-(1-isopropyl-1H-pyrazol-5-yl)tetrahydro-2H-pyran-4-carboxylate (70 mg). 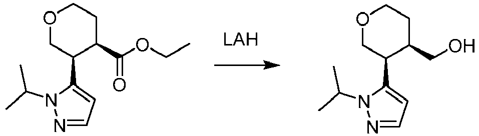
**Step 2:** To a solution of (±) (3S,4R)-ethyl 3-(1-isopropyl-1H-pyrazol-5-yl)tetrahydro-2H-pyran-4-carboxylate (70 mg, 0.26 mmol) in THF (1.5 mL) at -15 °C was added 1M LiAH₄ solution in THF (0.34 mL, 0.34 mmol) slowly. After stirred for 30 min, it was quenched with Sat. NH₄Cl; the mixture was extracted with EtOAc. Organic layers were combined, dried and concentrated to give (±) (3S,4R)-3-(1-isopropyl-1H-pyrazol-5-yl)tetrahydro-2H-pyran-4-yl)methanol as crude product (60 mg). 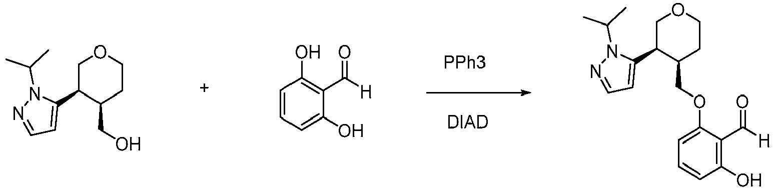
**Step 3:** To a solution of (±) ((3S, 4R)-3-(1-isopropyl-1H-pyrazol-5-yl)tetrahydro-2H-pyran-4-yl)methanol (50 mg, 0.22 mmol) and 2,6-dihydroxybenzaldehyde (60 mg, 0.44 mmol)in THF (1 mL) was added PPh₃ (120 mg, 0.44 mmol) and DIAD (0.09 mL, 0.44 mmol) at 0 °C. After stirred for 30 min, the solution was concentrated and the residue was purified by column (Hexanes/EtOAc=60:40) to give impure product, which was further purified by prep HPLC (eluted with ACN/H₂O) to give (±) 2-hydroxy-6-(((3S,4R)-3-(1-isopropyl-1H-pyrazol-5-yl)tetrahydro-2H-pyran-4-yl)methoxy)benzaldehyde (6 mg). 1H NMR (400 MHz, CDCl₃) δ (ppm) 11.90 (s, 1H), 10.36 (s, 1H), 7.79 (s, 1H), 7.50 (d, J=2.0 Hz, 1H), 7.32 (t, J = 8.8 Hz, 1H), 6.52 (d, J = 8.4 Hz, 1H), 6.43 (d, J = 1.6 Hz, 1H), 6.16 (d, J = 8.0 Hz, 1H), 4.46 (m, 1H), 4.13 (dt, J = 11.2, 4.0 Hz, 1H), 3.95 (dd, J= 11.2, 3.2 Hz, 1H), 3.81 (dd, J = 11.6, 3.2 Hz, 1H), 3.73 (dd, J = 9.2, 5.6 Hz, 1H), 3.65 (dt, J = 11.6, 3.2 Hz, 1H), 3.57 (t, J = 8.8 Hz, 1H), 3.28 (d, J = 4.0 Hz, 1H), 2.56 (m, 1H), 1.87 (m, 1H), 1.58 (m, 1H), 1.31 (d, J = 6.8 Hz, 3H), 1.29 (d, J = 7.6 Hz, 3H); MS (ESI) m/z 334.3 [M+H]⁺.
**Step 5:** To a solution of tert-butyl 4-(bromomethyl)-5-(1-isopropyl-1H-pyrazol-5-yl)-3,6-dihydropyridine-1(2H)-carboxylate (18 mg, 0.05 mmol) and 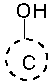 10 mg, 0.06 mmol) in DMF (1 mL) is added K₂CO₃ (14 mg, 0.1 mmol). After stirring at room temperature for 1 h, it is diluted with water and EtOAc, organic layer is separated, and the aqueous layer is extracted with EtOAc, organic layer is combined, washed with brine, dried and concetrated to give crude product, which is purified by column (Hexanes/EtOAc=2:1. 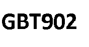 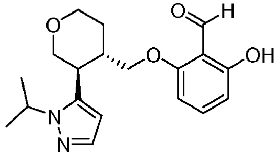
2-hydroxy-6-(((3S,4S)-3-(1-isopropyl-1*H*-pyrazol-5-yl)tetrahydro-2*H*-pyran-4-yl)methoxy)benzaldehyde

**GBT902- (±) 2-hydroxy-6-(((3S, 4S)-3-(1-isopropyl-1H-pyrazol-5-yl)tetrahydro-2H-pyran-4-yl)methoxy)benzaldehyde.** The compound was synthesized in three steps starting from (±) (3S,4R)-ethyl 3-(1-isopropyl-1H-pyrazol-5-yl)tetrahydro-2H-pyran-4-carboxylate. 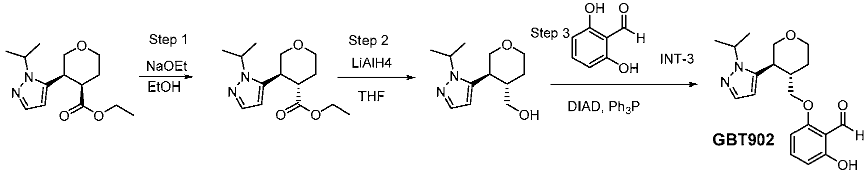
Step 1: To EtOH (2 mL) in round bottom flask was added NaH (65% dispersion in mineral oil, 60 mg, 1.36 mmol), after stirring for 5 min, the mixture was added a solution of (±) (3S,4R)-ethyl 3-(1-isopropyl-1H-pyrazol-5-yl)tetrahydro-2H-pyran-4-carboxylate (297 mg, 1.13 mmol) in EtOH. The mixture was heated at 80 °C for 3 h, cooled and diluted with EtOAc and Sat. NH4CI, organic layer was separated and the aqueous layer was extracted with EtOAc, organic layer was combined, dried and concentrated to give crude product, which was purified by column (Hexanes/EtOAc=2:1) to give ethyl (3*S*,4*S*)-3-(1-isopropyl-1H-pyrazol-5-yl)tetrahydro-2H-pyran-4-carboxylate 190 mg.
Step 2: To a solution of (3*S*,4*S*)-ethyl 3-(1-isopropyl-1H-pyrazol-5-yl)tetrahydro-2H-pyran-4-carboxylate (190 mg, 0.71 mmol) in THF (3 mL) at -20 °C was added LiAlH₄ (1M in THF, 0.89 mL, 0.89 mmol). After stirring at -20 °C for 15 min, the reaction was quenched with Sat. NH₄Cl, extracted with EtOAc, organic layer was combined, washed with brine, dried and concentrated to give ((3*S*,4*S*)-3-(1-isopropyl-1H-pyrazol-5-yl)tetrahydro-2H-pyran-4-yl)methanol as crude product (160 mg).
Step 3: To a solution of ((3*S*,4*S*)-3-(1-isopropyl-1H-pyrazol-5-yl)tetrahydro-2H-pyran-4-yl)methanol (160 mg, 0.71 mmol) in THF (2 mL) was added 2,6-dihydroxybenbzaldehyde (0.15 g, 1.06 mmol) at room temperature, then it was cooled to 0 °C and added DIAD (0.20 mL, 1.06 mmol). After stirring for 1 h, the mixture was concentrated and subjected to column chromatography to (±) 2-hydroxy-6-(((3S, 4S)-3-(1-isopropyl-1H-pyrazol-5-yl)tetrahydro-2H-pyran-4-yl)methoxy)benzaldehyde 104 mg (Hexanes/EtOAc=100:0 to 65:35 to 55:45) to give ¹H NMR (400 MHz, Chloroform-d) δ 11.90 (d, J = 0.4 Hz, 1H), 10.35 (d, J = 0.6 Hz, 1H), 7.51 (dt, *J* = 2.0, 0.6 Hz, 1H), 7.30 (t, J = 8.5 Hz, 1H), 6.51 (dt, *J* = 8.5, 0.7 Hz, 1H), 6.16 (dd, *J* = 8.3, 0.8 Hz, 1H), 6.06 (dd, *J* = 1.9, 0.4 Hz, 1H), 4.47 (p, J = 6.6 Hz, 1H), 4.21 - 4.07 (m, 1H), 3.99 - 3.84 (m, 2H), 3.80 (dd, *J* = 9.2, 5.2 Hz, 1H), 3.65 - 3.53 (m, 1H), 3.36 (t, J = 11.3 Hz, 1H), 3.09 (td, *J* = 11.0, 4.4 Hz, 1H), 2.29 - 2.21 (m, 1H), 1.95 - 1.85 (m, 2H), 1.44 (d, J = 6.6 Hz, 3H), 1.33 - 1.21 (m, 3H). MS (M+H)+ found for C₁₉H₂₄N₂O₄: 345.3. 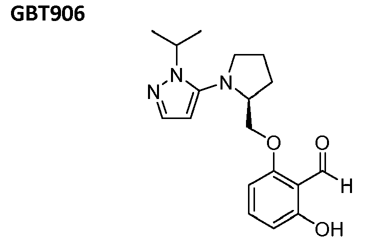
(*S*)-2-hydroxy-6-((1-(1-isopropyl-1*H*-pyrazol-5-yl)pyrrolidin-2-yl)methoxy)benzaldehyde

**GBT906** - **Preparation of (*S*)-2-hydroxy-6-((1-(1-isopropyl-1H-pyrazol-5-yl)pyrrolidin-2-yl)methoxy)benzaldehyde.** The compound was prepared from (*S*)-pyrrolidin-2-ylmethanol and 5-iodo-1-isopropyl-1H-pyrazole according to scheme 1, reaction steps 3 and 4. ¹H NMR (400 MHz, Chloroform-d) δ 11.92 (s, 1H), 10.07 (d, *J* = 0.6 Hz, 1H), 7.45 (dd, J = 2.0, 0.5 Hz, 1H), 7.34 (t, *J* = 8.4 Hz, 1H), 6.49 (dt, *J* = 8.5, 0.7 Hz, 1H), 6.24 (dd, *J* = 8.3, 0.8 Hz, 1H), 5.91 - 5.81 (m, 1H), 4.70 - 4.55 (m, 1H), 3.97 (dd, *J* = 9.3, 4.8 Hz, 1H), 3.88 (dd, *J* = 9.3, 5.6 Hz, 1H), 3.75 (dddd, *J* = 7.6, 6.6, 5.5, 4.8 Hz, 1H), 3.48 - 3.37 (m, 1H), 2.94 (dt, *J* = 9.2, 7.2 Hz, 1H), 2.31 - 2.15 (m, 1H), 2.09 - 1.81 (m, 3H), 1.45 (d, *J* = 6.7 Hz, 3H), 1.29 (d, *J* = 6.6 Hz, 3H). MS (M+H)+ found for C₁₈H₂₃N₃O₃: 330.3. 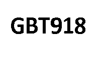 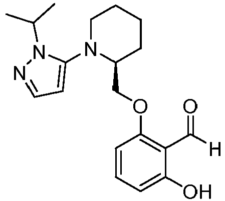 (*S*)-2-hydroxy-6-((1-(1-isopropyl-1H-pyrazol-5-yl)piperidin-2-yl)methoxy)benzaldehyde

**GBT918** - **Preparation of (*S*)-2-hydroxy-6-((1-(1-isopropyl-1H-pyrazol-5-yl)piperidin-2-yl)methoxy)benzaldehyde.** The compound was prepared from (*S*)-piperidin-2-ylmethanol hydrochloride and 5-iodo-1-isopropyl-1H-pyrazole according to scheme 1, reaction steps 3 and 4. ¹H NMR (400 MHz, Chloroform-*d*) δ 11.88 (d, *J* = 0.4 Hz, 1H), 10.31 (d, *J* = 0.6 Hz, 1H), 7.53 (d, *J* = 2.0 Hz, 1H), 7.36 - 7.28 (m, 1H), 6.49 (dt, *J* = 8.5, 0.7 Hz, 1H), 6.09 (dd, *J* = 8.3, 0.8 Hz, 1H), 6.06 (d, *J* = 2.1 Hz, 1H), 4.84 (hept, *J* = 6.7 Hz, 1H), 3.87 - 3.75 (m, 2H), 3.17 (dq, *J* = 8.4, 3.8 Hz, 1H), 3.02 (dt, *J* = 11.8, 3.8 Hz, 1H), 2.75 (td, *J* = 11.2, 3.4 Hz, 1H), 2.03 - 1.86 (m, 2H), 1.71 (dddd, *J* = 16.8, 15.4, 11.8, 7.2 Hz, 3H), 1.59 - 1.45 (m, 1H), 1.42 (d, *J* = 6.7 Hz, 3H), 1.32 (d, *J* = 6.7 Hz, 3H). MS (M+H)+ found for C₁₉H₂₅N₃O₃: 344.4.

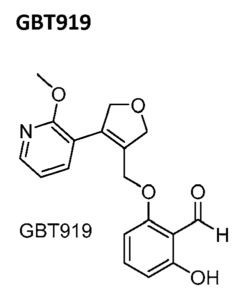

**GBT919- 2-hydroxy-6-((4-(2-methoxypyridin-3-yl)-2,5-dihydrofuran-3-yl)methoxy)benzaldehyde.** The compound was synthesized according to scheme 2 in five steps starting from ethyl 4-oxotetrahydrofuran-3-carboxylate using reaction steps 1, 2, 3, 4 and method B. 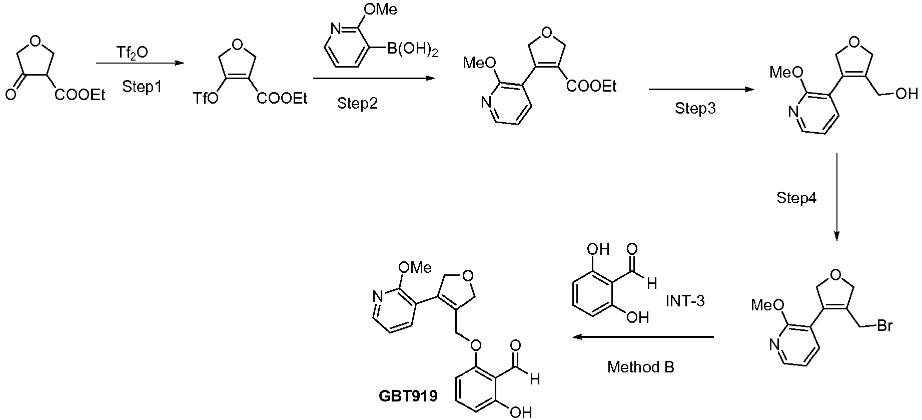
Step 1: To a solution of ethyl 4-oxotetrahydrofuran-3-carboxylate (1.13 g, 7.15 mmol) in DCM (20 ml) was added DIPEA (1.38 mL, 7.87 mmol) and Tf₂O (1.20 mL, 7.15 mmol) at -78 °C, then it was warmed up to room temperature and was further stirred for 15 h, the mixture was diluted with DCM, washed with Sat. NaHCO₃, brine, dried and concentrated to give ethyl 4-(((trifluoromethyl)sulfonyl)oxy)-2,5-dihydrofuran-3-carboxylate as crude product, which was used for next step without purification (2.3 g).
Step 2: To a solution of ethyl 4-(((trifluoromethyl)sulfonyl)oxy)-2,5-dihydrofuran-3-carboxylate (2.3 g) and (2-methoxypyridin-3-yl)boronic acid (1.09 g, 7.15 mmol) in Dioxane (20 mL) was added Pd(dppf)Cl₂ (530 mg, 0.72 mmol) and a solution of Na₂CO₃ (2.27 g, 21.45 mmol) in water (10 mL), the mixture was degassed and heated at 100 °C for 15 h, the solution was diluted with EtOAc, organic layer was washed with water, brine, dried over MgSO₄ and was concentrated to give crude product, which was purified by column chromatography to give ethyl 4-(2-methoxypyridin-3-yl)-2,5-dihydrofuran-3-carboxylate (1.1 g).
Step 3: To a solution of ethyl 4-(2-methoxypyridin-3-yl)-2,5-dihydrofuran-3-carboxylate (146 mg, 0.60 mmol) in THF (2 ml) at -20 °C was added 1M LiAlH₄ in THF (0.72 mL, 0.72 mmol). After stirred for 20 min, it was quenched with Sat. NH₄Cl, the mixture was extracted with EtOAc (3X), organic layers were combined, dried over MgSO₄ and was concentrated to give (4-(2-methoxypyridin-3-yl)-2,5-dihydrofuran-3-yl)methanol as crude product (120 mg), which was used without purification in next step.
Step 4: To a solution of (4-(2-methoxypyridin-3-yl)-2,5-dihydrofuran-3-yl)methanol (120 mg, 0.58 mmol) in DCM (2 mL) was added PPh₃Br₂ (300 mg, 0.72 mmol). After stirred at room temperature for 30 min, it was diluted with DCM and washed with Sat. NaHCO₃, brine, dried over MgSO₄ and concentrated to give crude product, which was purified by column chromatography to give 3-(4-(bromomethyl)-2,5-dihydrofuran-3-yl)-2-methoxypyridine (62 mg).

Method B: To a solution of 3-(4-(bromomethyl)-2,5-dihydrofuran-3-yl)-2-methoxypyridine (62 mg, 0.22 mmol) and 2,6-dihydroxybenzaldehyde (60 mg, 0.44 mmol) in DMF (1 mL) was added K₂CO₃ (90 mg, 0.66 mmol). After stirred at room temperature for 30 min, the mixture was added water, extracted with EtOAc (3X), organic layers were combined, washed with brine, dried and concentrated to give crude product, which was purified by column chromatography to give 2-hydroxy-6-((4-(2-methoxypyridin-3-yl)-2,5-dihydrofuran-3-yl)methoxy)benzaldehyde (47 mg). ¹H NMR (400 MHz, Chloroform-d) δ 11.93 (s, 1H), 10.20 (d, *J* = 0.6 Hz, 1H), 8.16 (dd, *J* = 5.0, 1.9 Hz, 1H), 7.42 (dd, *J* = 7.3, 1.9 Hz, 1H), 7.34 (t, *J* = 8.4 Hz, 1H), 6.96 - 6.87 (m, 1H), 6.52 (dt, *J* = 8.5, 0.7 Hz, 1H), 6.24 (dd, *J* = 8.3, 0.8 Hz, 1H), 5.00 (ddq, *J* = 4.9, 2.2, 1.2 Hz, 2H), 4.97 - 4.90 (m, 2H), 4.73 - 4.67 (m, 2H), 3.94 (s, 3H). MS(M-H) found for C18H17NO5: 326.2.

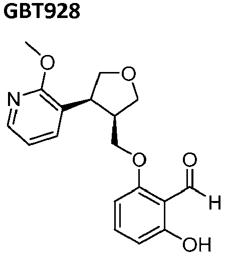

**GBT928-2-hydroxy-6-(((3*S*,4*S*)-4-(2-methoxypyridin-3-yl)tetrahydrofuran-3-yl)methoxy)benzaldehyde.** The compound was synthesized in three steps according to scheme 2 starting from ethyl 4-(2-methoxypyridin-3-yl)-2,5-dihydrofuran-3-carboxylate using reaction steps 5, 6 and method A. 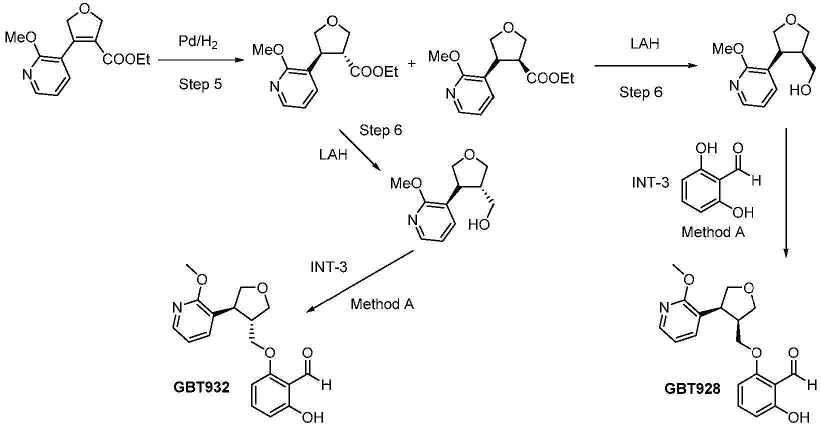
Step 5: To a solution of ethyl 4-(2-methoxypyridin-3-yl)-2,5-dihydrofuran-3-carboxylate (500 mg, 1 mmol) in EtOH (3 mL) was added Pd/C (50 mg), charged with H₂ (1 atm). After stirred for 24 h, Pd/C was filtered off and the filtrate was concentrated to give crude product, which was purified by column chromatography to give (±) ethyl (3*S*,4*S*)-4-(2-methoxypyridin-3-yl)tetrahydrofuran-3-carboxylate (140 mg) and (±) ethyl (3*R*,4*S*)-4-(2-methoxypyridin-3-yl)tetrahydrofuran-3-carboxylate (100 mg).
Step 6: To a solution of (3S,4S)-ethyl 4-(2-methoxypyridin-3-yl)tetrahydrofuran-3-carboxylate (140 mg) in THF (2 mL) was added LiAlH₄ in THF at -20 °C, then after 30 min, it was quenched with Sat. NH₄Cl, extracted with EtOAc, organic layers were combined, washed with brine, dried and concentrated to give ((3*R*,4*S*)-4-(2-methoxypyridin-3-yl)tetrahydrofuran-3-yl)methanol as crude oil 120 mg.

Method A: To a solution of ((3*R*,4*S*)-4-(2-methoxypyridin-3-yl)tetrahydrofuran-3-yl)methanol (120 mg, 0.57 mmol) and 2,6-dihydroxybenzaldehyde (0.10 g, 0.71 mmol) in THF (1mL) was added PPh₃ (0.22 g, 0.85 mmol) and DIAD (0.17 mL, 0.85 mmol) at room temperature, after stirred for 1 h, it was concentrated to give drude oil, which was purified by column chromatography followed by preparative HPLC to give 2-hydroxy-6-(((3S,4S)-4-(2-methoxypyridin-3-yl)tetrahydrofuran-3-yl)methoxy)benzaldehyde 6 mg. ¹H NMR (400 MHz, Chloroform-d) δ 11.90 (s, 1H), 10.12 (t, *J* = 0.5 Hz, 1H), 8.04 (dd, *J* = 5.0, 1.9 Hz, 1H), 7.52 (ddd, *J* = 7.3, 1.8, 0.6 Hz, 1H), 7.29 (t, *J* = 8.4 Hz, 1H), 6.85 (ddd, *J* = 7.4, 5.0, 0.5 Hz, 1H), 6.46 (dq, *J* = 8.5, 0.6 Hz, 1H), 6.06 (dd, *J* = 8.3, 0.8 Hz, 1H), 4.23 - 4.12 (m, 3H), 3.92 (d, *J* = 0.4 Hz, 3H), 3.90 - 3.77 (m, 3H), 3.65 (dd, *J* = 9.3, 7.7 Hz, 1H), 3.20 (qt, *J* = 7.6, 6.2 Hz, 1H). MS found for C18H19NO5: 330.3. 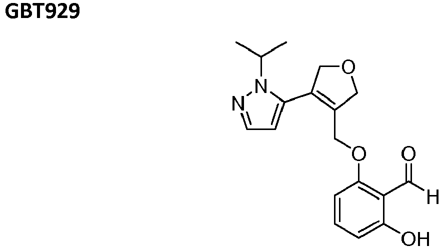 2-hydroxy-6-((4-(1-isopropyl-1*H*-pyrazol-5-yl)-2,5-dihydrofuran-3-yl)methoxy)benzaldehyde

**GBT929-2-hydroxy-6-((4-(1-isopropyl-1H-pyrazol-5-yl)-2,5-dihydrofuran-3-yl)methoxy)benzaldehyde.** The compound was synthesized according to scheme 2 in four steps starting from ethyl 4-(((trifluoromethyl)sulfonyl)oxy)-2,5-dihydrofuran-3-carboxylate using reaction steps 2, 3, 4 and method B 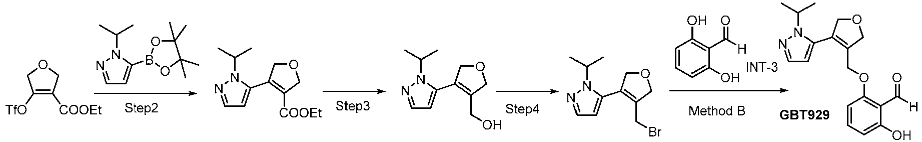
Step 2: To a solution of ethyl 4-(((trifluoromethyl)sulfonyl)oxy)-2,5-dihydrofuran-3-carboxylate (2.76 g, 9.5 mmol) and 1-isopropyl-5-(4,4,5,5-tetramethyl-1,3,2-dioxaborolan-2-yl)-1H-pyrazole (2.24 g, 9.50 mmol) in Dioxane (20 mL) was added Pd(dppf)Cl₂ (700 mg, 0.95 mmol) and a solution of Na₂CO₃ (3.02 g, 28.50 mmol) in water (10 mL), the mixture was degassed and heated at 100 °C for 15 h, the solution was diluted with EtOAc, organic layer was washed with water, brine, dried over MgSO4 and was concentrated to give crude product, which was purified by column chromatography (hexanes/EtOAc=3:1) to give ethyl 4-(2-methoxypyridin-3-yl)-2,5-dihydrofuran-3-carboxylate (900 mg).
Step 3: To a solution of ethyl 4-(1-isopropyl-1H-pyrazol-5-yl)-2,5-dihydrofuran-3-carboxylate (250 mg, 1 mmol) in THF (3 mL) at -20 °C was added LiAlH₄ (1M in THF, 1.2 mL, 1.2 mmol). After stirred for 20 min, it was quenched with Sat. NH₄Cl and was extracted with EtOAc, organic layers were combined, washed with brine, dried over MgSO₄ and was concentrated to give (4-(1-isopropyl-1H-pyrazol-5-yl)-2,5-dihydrofuran-3-yl)methanol as crude product (210 mg).
Step 4: To a solution of (4-(1-isopropyl-1H-pyrazol-5-yl)-2,5-dihydrofuran-3-yl)methanol (210 mg, 1 mmol) in DCM (3 mL) was added PPh₃Br₂ (420 mg, 1mmol) at room temperature, after stirred for 20 min, it was diluted with DCM, organic layer was washed with Sat. NaHCO₃, brine, dried over MgSO₄ and was concentrated to give crude product, which was purified by column chromatography (Hexanes/EtOAc=3:1) to give 5-(4-(bromomethyl)-2,5-dihydrofuran-3-yl)-1-isopropyl-1H-pyrazole (110 mg).

Method B: To a solution of 5-(4-(bromomethyl)-2,5-dihydrofuran-3-yl)-1-isopropyl-1H-pyrazole (110 mg, 0.41 mmol) in DMF (2 mL) was added K₂CO₃ (170 mg, 1.23 mmol) and 2,6-dihydroxybenzaldehyde (0.11 g, 0.82 mmol) at room temperature, after stirred for 30 min, it was diluted with water and EtOAc, EtOAc layer was separated and the aqueous layer was extracted with EtOAc, organic layers were combined, washed with brine, dried and concentrated to give crude product, which was purified by column (Hexanes/EtOAc=2:1) to give 2-hydroxy-6-((4-(1-isopropyl-1H-pyrazol-5-yl)-2,5-dihydrofuran-3-yl)methoxy)benzaldehyde (101 mg). ¹H NMR (400 MHz, Chloroform-d) δ 11.93 (d, *J* = 0.4 Hz, 1H), 10.23 (d, *J* = 0.6 Hz, 1H), 7.57 (dd, *J* = 1.8, 0.6 Hz, 1H), 7.35 (t, *J* = 8.5, 1H), 6.55 (dt, *J* = 8.5, 0.7 Hz, 1H), 6.23 (dd, *J* = 8.3,0.8 Hz, 1H), 6.14 (d, *J* = 1.8 Hz, 1H), 4.97 (dt, *J* = 4.9, 3.3 Hz, 2H), 4.93 - 4.86 (m, 2H), 4.70 - 4.65 (m, 2H), 4.44 - 4.32 (m, 1H), 1.67 - 1.41 (m, 6H). MS found for C₁₈H₂₀N₂O₄: 329.3.

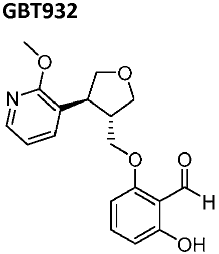

GBT932- **2-hydroxy-6-(((3*S*,4*R*)-4-(2-methoxypyridin-3-yl)tetrahydrofuran-3-yl)methoxy)benzaldehyde.** The compound was synthesized in two steps starting from (3*S*,4*S*)-ethyl 4-(2-methoxypyridin-3-yl)tetrahydrofuran-3-carboxylate using reaction steps 6 and method A.

Step 6: To a solution of (3*S*,4*S*)-ethyl 4-(2-methoxypyridin-3-yl)tetrahydrofuran-3-carboxylate (100 mg, 0.40 mmol) in THF (2 mL) was added LiAIH4 (1M in THF, 0.48 mL, 0.48 mmol) at -20 °C, after stirred for 30 min, it was quenched with Sat. NH₄Cl, extracted with EtOAc, organic layers were combined, washed with brine, dried over MgSO₄, and was concentrated to give ((3S,4S)-4-(2-methoxypyridin-3-yl)tetrahydrofuran-3-yl)methanol as crude oil 80 mg.

Method A: To a solution of ((3R,4S)-4-(2-methoxypyridin-3-yl)tetrahydrofuran-3-yl)methanol (80 mg, 0.40 mmol) and 2,6-dihydroxybenzaldehyde (0.07 g, 0.52 mmol) in THF (1mL) was added PPh₃ (0.16 g, 0.60 mmol) and DIAD (0.12 mL, 0.60 mmol) at room temperature, after stirred for 1 h, it was concentrated to give crude oil, which was purified by column chromatography to give 2-hydroxy-6-(((3*S*,4*R*)-4-(2-methoxypyridin-3-yl)tetrahydrofuran-3-yl)methoxy)benzaldehyde 20 mg. ¹H NMR (400 MHz, Chloroform-*d*) δ 11.93 (d, *J* = 0.3 Hz, 1H), 10.13 (d, *J* = 0.6 Hz, 1H), 8.08 (dd, *J* = 5.0, 1.8 Hz, 1H), 7.53 (ddd, *J* = 7.3, 1.8, 0.5 Hz, 1H), 7.43 - 7.32 (m, 1H), 6.93 - 6.83 (m, 1H), 6.52 (dt, *J* = 8.5, 0.7 Hz, 1H), 6.33 (dd, *J* = 8.3, 0.8 Hz, 1H), 4.20 (ddd, *J* = 8.7, 7.6, 5.2 Hz, 2H), 4.14 - 4.03 (m, 2H), 3.94 (s, 3H), 3.92 - 3.80 (m, 2H), 3.52 (q, *J* = 7.1 Hz, 1H), 2.93 (dq, *J* = 7.4, 6.6 Hz, 1H). MS found for C₁₈H₁₉NO₅: 330.3. 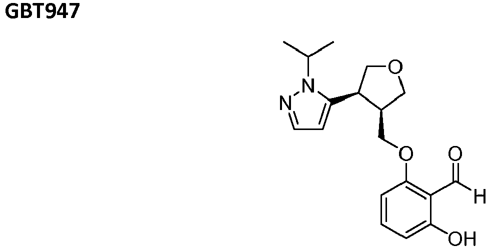 2-hydroxy-6-(((3*S*,4*S*)-4-(1-isopropyl-1*H*-pyrazol-5-yl)tetrahydrofuran-3-yl)methoxy)benzaldehyde

**GBT947-2-hydroxy-6-(((3*S*,4*S*)-4-(1-isopropyl-1H-pyrazol-5-yl)tetrahydrofuran-3-yl)methoxy)benzaldehyde.** The compound was synthesized according scheme 2 in three steps starting from ethyl 4-(1-isopropyl-1H-pyrazol-5-yl)-2,5-dihydrofuran-3-carboxylate using reaction steps 5, 6 and method A. 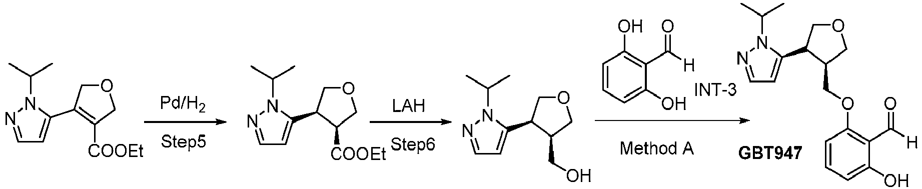
Step 5: To a solution of ethyl 4-(l-isopropyl-1H-pyrazol-5-yl)-2,5-dihydrofuran-3-carboxylate (325 mg, 1.32 mmol) in EtOH (4 mL) was added Pd/C (150 mg), then it was charged with H₂ (1atm) and then stirred at room temperature for 3 h, H₂ balloon was removed and the mixture was added NH₄CO₂H in water (1 mL) and was heated at 75 °C for 3 h, the mixture was cooled and diluted with EtOAc and water, aqueous layer was separated and extracted with EtOAc, organic layers were combined, washed with brine, dried over MgSO₄ and concentrated to give crude oil, which was purified by column (Hexanes/EtOAc=60:40) to give ethyl (3S,4S)-4-(1-isopropyl-1H-pyrazol-5-yl)tetrahydrofuran-3-carboxylate (216 mg).
Step 6: To a solution of (4-(1-isopropyl-1H-pyrazol-5-yl)-2,5-dihydrofuran-3-yl)methanol (216 mg, 0.87 mmol) in THF (4 mL) at -20 °C was added LiAlH₄ (1M in THF, 1.04 mL, 1.04 mmol). After stirred for 20 min, it was quenched with Sat. NH₄Cl, aqueous layer was extracted with EtOAc, organic layers were combined, washed with brine, dried and concentrated to give ((3*R*,4*S*)-4-(1-isopropyl-1H-pyrazol-5-yl)tetrahydrofuran-3-yl)methanol as crude oil (180 mg).

Method A; To a solution of ((3*R*,4*S*)-4-(1-isopropyl-1H-pyrazol-5-yl)tetrahydrofuran-3-yl)methanol (180 mg, 0.86 mmol) and 2,6-dihydroxybenzaldehyde (150 mg, 1.12 mmol) in THF (1.6 mL) was added PPh₃ (340 mg, 1.29 mmol) and DIAD (0.25 mL, 1.29 mmol) at 0 °C, then it was stirred at room temperature for 1 h, and was concentrated and purified by column (Hexanes/EtOAc = 60:40) to give 2-hydroxy-6-(((3S,4S)-4-(1-isopropyl-1H-pyrazol-5-yl)tetrahydrofuran-3-yl)methoxy)benzaldehyde 82 mg. ¹H NMR (400 MHz, Chloroform-*d*) δ 11.88 (d, *J* = 0.4 Hz, 1H), 10.27 (t, *J* = 0.5 Hz, 1H), 7.50 (dd, *J* = 1.8, 0.6 Hz, 1H), 7.35 (t, *J* = 8.5, 1H), 6.51 (dq, *J* = 8.5, 0.6 Hz, 1H), 6.10 (ddt, *J* = 5.4, 3.5, 0.6 Hz, 1H), 4.53 (h, *J* = 6.5 Hz, 1H), 4.29 - 4.22 (m, 1H), 4.19 (dd, *J* = 8.9, 7.1 Hz, 1H), 4.04 (dd, *J* = 8.6, 6.5 Hz, 1H), 3.94 (dd, *J* = 8.9, 5.9 Hz, 1H), 3.79 - 3.69 (m, 2H), 3.60 (dd, *J* = 9.3,6.2 Hz, 1H), 3.02 (dtd, *J* = 13.6, 7.7, 6.1 Hz, 2H), 1.46 (dd, *J* = 14.1, 6.6 Hz, 6H). MS found for C₁₈H₂₂N₂O₄: 331.3.

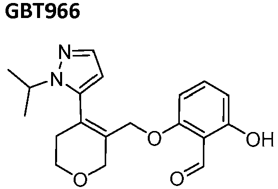

GBT966- **2-hydroxy-6-((4-(1-isopropyl-1H-pyrazol-5-yl)-5,6-dihydro-2H-pyran-3-yl)methoxy)benzaldehyde.** The compound was synthesized in five steps starting from oxan-4-one. 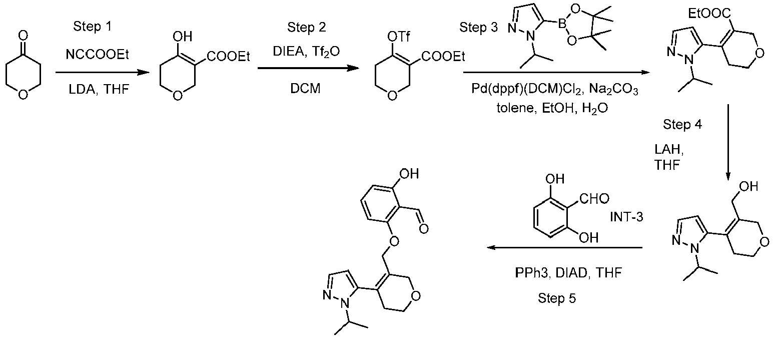
**Step 1:** Into a 250-mL round-bottom flask, was placed a solution of oxan-4-one (5.0 g, 49.94 mmol, 1.00 equiv) in tetrahydrofuran (60 mL). This was followed by the addition of LDA (newly prepared from diisopropylamine and BuLi) (1.20 equiv) dropwise with stirring at -78 °C. The mixture was stirred for 1 h at 0 °C. HMPA (9.8 g, 54.69 mmol, 1.10 equiv) was then added to the reaction dropwise at -78 °C. The mixture was stirred for another 15 min at the same temperature. 2-Ethoxy-2-oxoacetonitrile (5 g, 50.46 mmol, 1.01 equiv) was then added to the reaction dropwise at -78 °C. The resulting solution was stirred for 2 h at 0 °C, and then it was quenched with 50 mL of water. The resulting mixture was concentrated under vacuum, and then it was extracted with EA (50 mL x 3). The combined organic layers were washed with 2x80 mL of water and 1x80 mL of brine, dried over anhydrous sodium sulfate and concentrated under vacuum. The residue was applied onto a silica gel column with ethyl acetate/petroleum ether (1:30) as eluent to yield 1.82 g (21%) of ethyl 4-oxooxane-3-carboxylate as a colorless oil.
**Step 2:** Into a 50-mL round-bottom flask, was placed a solution of ethyl 4-hydroxy-5,6-dihydro-2H-pyran-3-carboxylate (570 mg, 3.31 mmol, 1.00 equiv) and DIEA (2.5 mL, 5.00 equiv) in dichloromethane (20 mL). Tf₂O (1.0 mL, 2.00 equiv) was added to the reaction dropwise at 0 °C. The resulting solution was stirred for 1 h at 0 °C and for another 2 h at room temperature. The reaction was then quenched by the addition of 20 mL of water. The resulting solution was extracted with 2x40 mL of ethyl acetate, and the combined organic layers were washed with 3x20 mL of water and 1x20 mL of brine, dried over anhydrous sodium sulfate and concentrated under vacuum. The residue was applied onto a silica gel column with ethyl acetate/petroleum ether (1:25) as eluent to yield 0.67 g (67%) of ethyl 4-[(trifluoromethane)sulfonyloxy]-5,6-dihydro-2H-pyran-3-carboxylate as a light yellow oil.
**Step 3:** Into a 50-mL round-bottom flask, was placed a solution of ethyl 4-[(trifluoromethane)sulfonyloxy]-5,6-dihydro-2H-pyran-3-carboxylate (540 mg, 1.77 mmol, 1.00 equiv), 1-(propan-2-yl)-5-(tetramethyl-1,3,2-dioxaborolan-2-yl)-1H-pyrazole (543 mg, 2.30 mmol, 1.30 equiv) in a solvent mixture of toluene (15.0 mL), aqueous solution of sodium carbonate (2M) (5.0 mL) and ethanol (5.0 mL). This was followed by the addition of Pd(dppf)Cl₂CH₂Cl₂ (115 mg, 0.08 equiv). The resulting solution was stirred for 4 h at 100 °C under N₂. The reaction was then quenched with 15 mL of water. The resulting solution was extracted with 3x30 mL of ethyl acetate. The combined organic layers were washed with 2x50 mL of water and 1x50 mL of brine. The mixture was dried over anhydrous sodium sulfate and concentrated under vacuum. The residue was applied onto a silica gel column with ethyl acetate/petroleum ether (1:15-1:6) as eluent to yield 372 mg (79%) of ethyl 4-[1-(propan-2-yl)-1H-pyrazol-5-yl]-5,6-dihydro-2H-pyran-3-carboxylate as a light yellow oil.
**Step 4:** Into a 50-mL round-bottom flask, was placed a solution of ethyl 4-[1-(propan-2-yl)-1H-pyrazol-5-yl]-5,6-dihydro-2H-pyran-3-carboxylate (234 mg, 0.89 mmol, 1.00 equiv) in tetrahydrofuran (15 mL). This was followed by the addition of LAH (51 mg, 1.34 mmol, 1.52 equiv) at 0 °C. The resulting solution was stirred for 1 h at 0 °C. The reaction was then quenched by the addition of 10 mL of 2.5 M sodium hydroxide aq. The resulting solution was extracted with 3x30 mL of ethyl acetate and the organic layers combined. The resulting mixture was washed with 1x20 mL of water and 1x20 mL of brine. The mixture was dried over anhydrous sodium sulfate and concentrated under vacuum. The residue was applied onto a silica gel column with ethyl acetate/petroleum ether (2:3) as eluent to furnish 124 mg (63%) of [4-[1-(propan-2-yl)-1H-pyrazol-5-yl]-5,6-dihydro-2H-pyran-3-yl]methanol as a colorless oil.
**Step 5:** Into a 25-mL round-bottom flask, was placed a solution of [4-[1-(propan-2-yl)-1H-pyrazol-5-yl]-5,6-dihydro-2H-pyran-3-yl]methanol (124 mg, 0.56 mmol, 1.00 equiv), 2,6-dihydroxybenzaldehyde (116 mg, 0.84 mmol, 1.50 equiv), and PPh₃ (220 mg, 0.84 mmol, 1.50 equiv), in tetrahydrofuran (10 mL). This was followed by the addition of DIAD (170 mg, 0.84 mmol, 1.50 equiv) dropwise with stirring at 0 °C. The resulting solution was stirred for 30 min at 0 °C and for an additional 1 h at room temperature. The reaction was then quenched by the addition of 10 mL of water. The resulting solution was extracted with 3x25 mL of ethyl acetate and the organic layers combined. The resulting mixture was washed with 2x60 mL of water and 1x40 mL of brine. The mixture was dried over anhydrous sodium sulfate and concentrated under vacuum. The residue was applied onto a silica gel column with ethyl acetate/petroleum ether (1:10-1:7) as eluent. The crude product was further purified by Prep-HPLC with the following conditions (Prep-HPLC-010): Column, SunFire Prep C18 OBD Column,5um, 19*150mm,; mobile phase, water with 0.05%TFA and MeCN (42.0% MeCN up to 55.0% in 8 min, up to 95.0% in 2 min,down to 42.0% in 2 min); Detector, Waters2545 UvDector 254&220nm. This provided 68 mg (36%) of 2-hydroxy-6-([4-[1-(propan-2-yl)-1H-pyrazol-5-yl]-5,6-dihydro-2H-pyran-3-yl]methoxy)benzaldehyde as a light yellow solid. ¹HNMR (400MHz, CDCl₃, *ppm*): 11.90 (s, 1H), 10.32 (s, 1H), 7.56 (s, 1H), 7.33 (t, *J* = 8.4 Hz, 1H), 6.53 (d, *J* = 8.4 Hz, 1H), 6.16 (d, *J* = 8.4 Hz, 1H), 6.07 (s, 1H), 4.44-4.40 (m, 5H), 3.96 (t, *J* = 5.6 Hz, 2H), 2.19 (s, 2H), 1.43 (d, *J* = 6.4 Hz, 6H); MS (ES, *m*/*z*:) 343.2 [M+1]⁺ 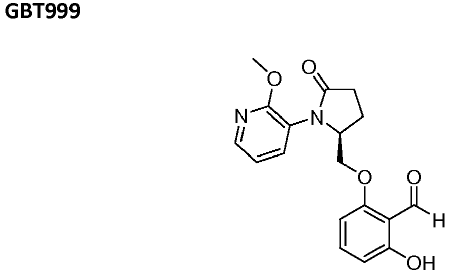 (*S*)-2-hydroxy-6-((1-(2-methoxypyridin-3-yl)-5-oxopyrrolidin-2-yl)methoxy)benzaldehyde

### GBT999- Preparation of (5)-2-hydroxy-6-((1-(2-methoxypyridin-3-yl)-5-oxopyrrolidin-2-yl)methoxy)benzaldehyde

The compound was prepared from (*S*)-5-(hydroxymethyl)pyrrolidin-2-one and 3-iodo-2-methoxypyridine according to scheme 1, reaction steps 3 and 4. ¹H NMR (400 MHz, Chloroform-*d*) δ 11.91 (d, *J* = 0.4 Hz, 1H), 9.85 (d, *J* = 0.6 Hz, 1H), 8.16 - 8.09 (m, 1H), 7.56 - 7.49 (m, 1H), 7.35 (ddd, *J* = 8.8, 8.1, 0.4 Hz, 1H), 6.99 - 6.90 (m, 1H), 6.53 (dt, *J* = 8.5, 0.7 Hz, 1H), 6.21 (dd, *J* = 8.3, 0.8 Hz, 1H), 4.67 (dtd, *J* = 8.5, 4.9, 3.7 Hz, 1H), 4.07 - 3.95 (m, 2H), 3.93 (d, *J* = 0.5 Hz, 3H), 2.76 - 2.56 (m, 2H), 2.50 (dddd, *J* = 13.0, 9.5, 8.4, 7.4 Hz, 1H), 2.20 - 2.04 (m, 1H). MS found for C₁₈H₁₈N₂O₅: 343.3. 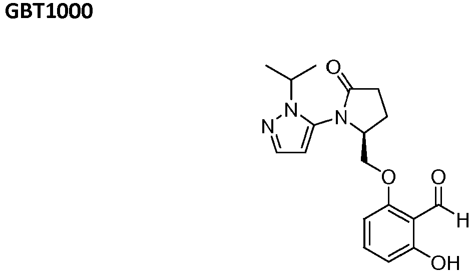 (*S*)-2-hydroxy-6-((1-(1-isopropyl-1*H*-pyrazol-5-yl)-5-oxopyrrolidin-2-yl)methoxy)benzaldehyde

**GBT1000** - **Preparation of (5)-2-hydroxy-6-((1-(1-isopropyl-1H-pyrazol-5-yl)-5-oxopyrrolidin-2-yl)methoxy)benzaldehyde.** The compound was prepared from (S)-5-(hydroxymethyl)pyrrolidin-2-one and 5-iodo-1-isopropyl-1H-pyrazole according to scheme 1, reaction steps 3 and 4. ¹H NMR (400 MHz, Chloroform-d) δ 11.92 (s, 1H), 10.13 (d, *J* = 0.6 Hz, 1H), 7.56 (dd, *J* = 1.9, 0.6 Hz, 1H), 7.38 (t, *J* = 8.4 Hz, 1H), 6.57 (dt, *J* = 8.5, 0.7 Hz, 1H), 6.25 (dd, *J* = 8.3, 0.8 Hz, 1H), 6.02 (d, *J* = 1.9 Hz, 1H), 4.34 - 4.19 (m, 2H), 4.08 (dd, *J* = 10.0, 3.4 Hz, 1H), 4.02 (dd, *J* = 10.0, 3.6 Hz, 1H), 2.79 - 2.60 (m, 2H), 2.52 (dddd, *J* = 13.3, 9.7, 8.4, 7.0 Hz, 1H), 2.28 (dddd, *J* = 13.3, 9.9, 6.8, 5.4 Hz, 1H), 1.48 (d, *J* = 6.6 Hz, 3H), 1.24 (d, *J* = 6.6 Hz, 3H). MS found for C₁₈H₂₁N₃O₄: 344.3. 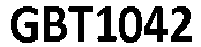 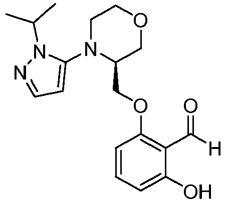 (*S*)-2-hydroxy-6-((4-(1-isopropyl-1*H*-pyrazol-5-yl)morpholin-3-yl)methoxy)benzaldehyde

**GBT1042** - **Preparation of (*S*)-2-hydroxy-6-((4-(1-isopropyl-1H-pyrazol-5-yl)morpholin-3-yl)methoxy)benzaldehyde.** The compound was prepared from (*R*)-tert-butyl 3-(hydroxymethyl)morpholine-4-carboxylate and 3-iodo-2-methoxypyridine according to scheme 1, reaction steps1, 3 and 4. ¹H NMR (400 MHz, Chloroform-*d*) δ 11.88 (s, 1H), 10.26 (d, *J* = 0.6 Hz, 1H), 7.53 (d, *J* = 2.0 Hz, 1H), 7.40 - 7.28 (m, 1H), 6.51 (dt, *J* = 8.5, 0.7 Hz, 1H), 6.11 (dd, *J* = 8.4, 0.8 Hz, 1H), 6.07 (d, *J* = 2.0 Hz, 1H), 4.79 (hept, *J* = 6.6 Hz, 1H), 4.10 (ddd, *J* = 11.4, 3.4, 0.7 Hz, 1H), 3.98 - 3.86 (m, 3H), 3.86 - 3.73 (m, 2H), 3.42 - 3.32 (m, 1H), 3.04 - 2.91 (m, 2H), 1.52 - 1.37 (m, 3H), 1.33 (d, *J* = 6.7 Hz, 3H). MS found for C₁₈H₂₃N₃O₄: 346.3. 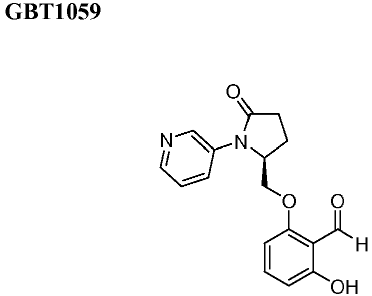 (S)-2-hydroxy-6-((5-oxo-1-(pyridin-3-yl)pyrrolidin-2-yl)methoxy)benzaldehyde

**GBT1059- Preparation of (*S*)-2-hydroxy-6-((5-oxo-1-(pyridin-3-yl)pyrrolidin-2-yl)methoxy)benzaldehyde.** The compound was prepared from (S)-5-(hydroxymethyl)pyrrolidin-2-one and 3-iodopyridine according to scheme 1, reaction steps 3 and 4. ¹H NMR (400 MHz, Chloroform-*d*) δ 11.88 (s, 1H), 10.05 (s, 1H), 8.64 (dd, *J* = 2.7, 0.7 Hz, 1H), 8.48 (dd, *J* = 4.8, 1.5 Hz, 1H), 7.90 (ddd, *J* = 8.3, 2.6, 1.5 Hz, 1H), 7.39 - 7.28 (m, 2H), 6.55 (dd, *J* = 8.5, 0.8 Hz, 1H), 6.22 (dt, *J* = 8.3, 1.0 Hz, 1H), 4.72 (dq, *J* = 8.3, 4.1 Hz, 1H), 4.19 - 4.05 (m, 2H), 2.84 - 2.61 (m, 2H), 2.54 (ddt, *J* = 13.2, 10.0, 8.3 Hz, 1H), 2.22 (dddd, *J* = 13.5, 9.9, 4.9, 3.7 Hz, 1H). MS found for C₁₇H₁₆N₂O₄: 313.3. 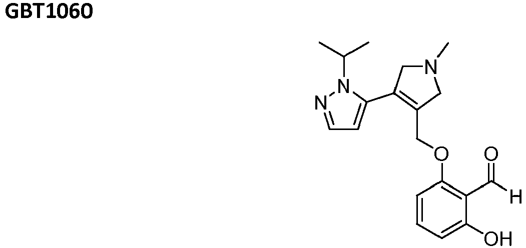 2-hydroxy-6-((4-(1-isopropyl-1*H*-pyrazol-5-yl)-1-methyl-2,5-dihydro-1*H*-pyrrol-3-yl)methoxy)benzaldehyde

**GBT1060-2-hydroxy-6-((4-(1-isopropy)-1H-pyrazo)-5-y))-1-methyl-2,5-dihydro-1H-pyrrol-3-yl)methoxy)benzaldehyde.** The compound was synthesized in 7 steps according to a modified scheme 2 starting from 1-tert-butyl 3-ethyl 4-oxopyrrolidine-1,3-dicarboxylate. 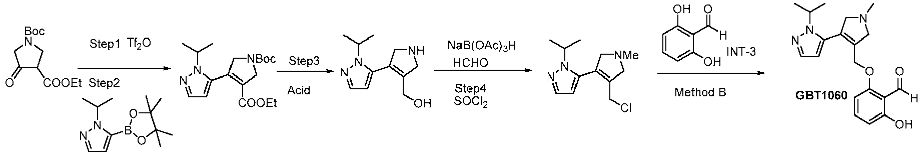
Steps 1&2: To a solution of 1-tert-butyl 3-ethyl 4-oxopyrrolidine-1,3-dicarboxylate (1.49 g, 5.81 mmol) in DCM (15 mL) at -78 °C was added DIPEA (1.22 mL) and Tf₂O (1.08 mL), then it was warmed to room temperature and was further stirred for 2 h, mixture was diluted with more DCM, DCM layer was washed with Sat. NaHCO₃, brine, dried over MgSO₄ and concentrated to give crude triflate. To a solution of this crude triflate in Dioxane (15 mL) was added 1-isopropyl-5-(4,4,5,5-tetramethyl-1,3,2-dioxaborolan-2-yl)-1H-pyrazole (1.37 g, 5.81 mmol) and Pd(dppf)Cl₂ (0.42 g, 0.58 mmol) and a solution of Na₂CO₃ (1.23 g, 11.62 mmol) in water (5 mL). After heated at 100 °C for 15 h the solution was diluted with EtOAc, organic layer was washed with water, brine, dried over MgSO₄ and was concentrated to give crude product, which was purified by column chromatography to give ethyl 1-tert-butyl 3-ethyl 4-(1-isopropyl-1H-pyrazol-5-yl)-1H-pyrrole-1,3(2H,5H)-dicarboxylate (0.59 g).
Step 3: To a solution of 1-tert-butyl 3-ethyl 4-(1-isopropyl-1H-pyrazol-5-yl)-1H-pyrrole-1,3(2H,5H)-dicarboxylate (590 mg, 1.69 mmol) in THF (6 ml) at -20 °C was added 1M LiAlH₄ in THF (2.03 mL, 2.03 mmol). After stirred for 20 min, it was quenched with Sat. NH₄Cl, the mixture was extracted with EtOAc (3X), organic layers were combined, dried over MgSO₄ and was concentrated to give tert-butyl 3-(hydroxymethyl)-4-(1-isopropyl-1H-pyrazol-5-yl)-2,5-dihydro-1H-pyrrole-1-carboxylate as crude product, which was used without purification in next step.
Steps 4a&4: To a suspension of (4-(1-isopropyl-1H-pyrazol-5-yl)-2,5-dihydro-1H-pyrrol-3-yl)methanol (200 mg, 0.96 mmol) in AcCN (2 mL) was added TEA (0.14 mL, 0.96 mmol) and HCHO aqueous solution (0.24 g). After stirred for 30 min, it was added NaB(OAc)₃H (0.41 g, 1.92 mmol). Another 15 min later, it was filtered and the filtrate was concentrated to give crude product, which was purified by column (DCM/MeOH=100:0 to 80:20) to give (4-(1-isopropyl-1H-pyrazol-5-yl)-1-methyl-2,5-dihydro-1H-pyrrol-3-yl)methanol (170 mg). To a solution of 4-(1-isopropyl-1H-pyrazol-5-yl)-1-methyl-2,5-dihydro-1H-pyrrol-3-yl)methanol in DCM (3mL) was added SOCl2 (0.2 mL) at 0 °C, after stirred for 30 min, it was then concentrated to give 5-(4-(chloromethyl)-1-methyl-2,5-dihydro-1H-pyrrol-3-yl)-1-isopropyl-1H-pyrazole as crude HCI salt (140 mg).

Method B: To a solution of 5-(4-(chloromethyl)-1-methyl-2,5-dihydro-1H-pyrrol-3-yl)-1-isopropyl-1H-pyrazole (140 mg, 0.60 mmol) and 2,6-dihydroxybenzaldehyde (170 mg, 1.20 mmol) in DMF (3 mL) was added K₂CO₃ (330 mg, 2.4 mmol). After stirred at 50 °C for 30 min, the mixture was added water, extracted with EtOAc (3X), organic layers were combined, washed with brine, dried and concentrated to give crude product, which was purified by preparative HPLC to give 2-hydroxy-6-((4-(1-isopropyl-1H-pyrazol-5-yl)-1-methyl-2,5-dihydro-1H-pyrrol-3-yl)methoxy)benzaldehyde (6 mg). ¹H NMR (400 MHz, Chloroform-d) δ 10.26 (d, *J* = 0.6 Hz, 1H), 8.26 (s, 1H), 7.55 (dd, *J* = 1.8, 0.6 Hz, 1H), 7.35 (t, *J* = 8.4 Hz, 1H), 6.54 (dt, *J* = 8.5, 0.7 Hz, 1H), 6.21 (dd, *J* = 8.3, 0.8 Hz, 1H), 6.11 (d, *J* = 1.8 Hz, 1H), 4.62 (d, *J* = 1.4 Hz, 2H), 4.47 - 4.33 (m, 1H), 3.98 - 3.88 (m, 4H), 2.67 (s, 3H), 1.44 (d, *J* = 6.7 Hz, 6H). MS (M+H) found for C₁₉H₂₃N₃O₃: 342.2.

## Claims

1. A compound of Formula (I'): 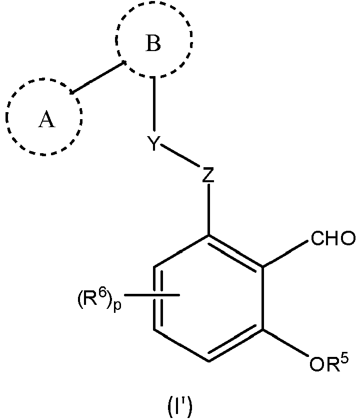 or a tautomer thereof, or a pharmaceutically acceptable salt of each thereof, wherein
ring A is an optionally substituted 5-10 membered heteroaryl containing up to 3 ring N, O, and/or S atoms, and oxidized forms of N and/or S atoms;
wherein ring A is α substituted relative to the Y substituent;
ring B is an optionally substituted C₆-C₁₀ aryl or 4-10 membered heterocycle containing up to 5 ring heteroatoms, wherein the heteroatom is selected from the group consisting of O, N, S, and oxidized forms of N and S;
Y-Z is -CH₂O-, wherein the right hand side of the substituent is joined with the substituted phenyl ring;
R⁵ is hydrogen or C₁-C₆ alkyl, wherein the C₁-C₆ alkyl is optionally substituted with 1-5 halo;
R⁶ is halo, C₁-C₆ alkyl, C₁-C₆ alkoxy, C₁-C₆ alkylthio, C₁-C₆ alkyl-S(O)-, or C₁-C₆ alkyl-S(O)₂-, wherein the C₁-C₆ alkyl is optionally substituted with 1-5 halo; or
R⁶ is 4-10 membered cycloalkyl or heterocycle substituted with an R'R'N- moiety wherein each R' is independently C₁-C₆ alkyl or hydrogen;
p is 0, 1, 2, or 3; and
the term heterocycle refers to a non-aromatic, mono-, bi-, or tricyclic ring containing 2-12 ring carbon atoms and 1-8 ring heteroatoms, provided that the ring contains at least 3 ring atoms.

2. The compound of claim 1, or a tautomer thereof, or a pharmaceutically acceptable salt of each thereof, wherein the compound is of Formula IA, IB or IC: 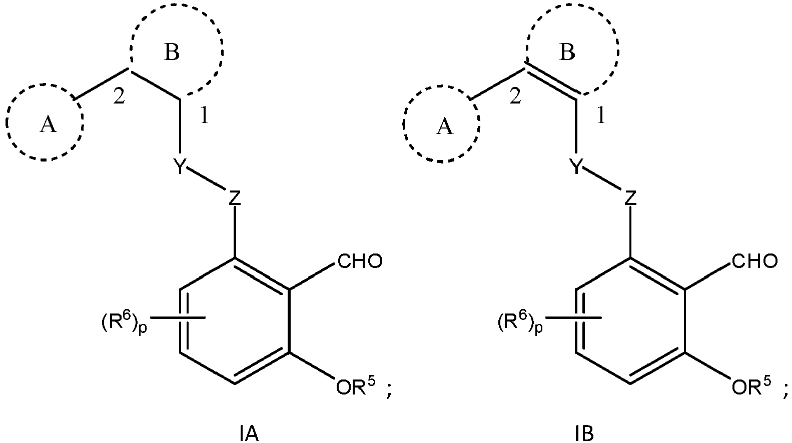 or 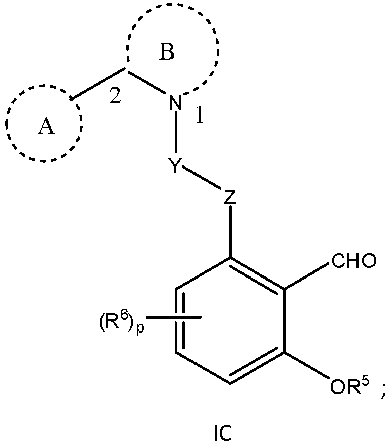 wherein 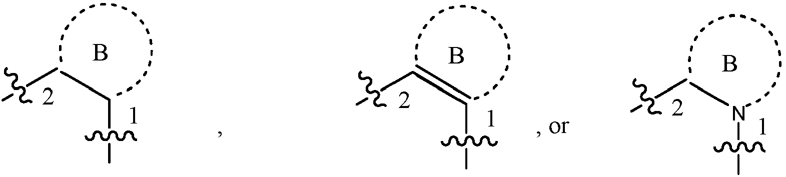 is an optionally substituted 4-10 membered heterocycle containing up to 5 ring heteroatoms, wherein the heteroatom is selected from the group consisting of O, N, S, and oxidized forms of N and S.

3. The compound of claim 1, or a tautomer thereof, or a pharmaceutically acceptable salt of each thereof, wherein ring A is substituted with 1-3 substituents independently selected from: halo, C₁-C₆ alkyl, and C₁-C₆ alkoxy, wherein the C₁-C₆ alkyl is optionally substituted with 1-5 halo.

4. The compound of claim 1, or a tautomer thereof, or a pharmaceutically acceptable salt of each thereof, wherein ring B is substituted with 1-3 substituents independently selected from: halo, C₁-C₆ alkyl, COR¹⁵, and COOR¹⁵; and
R¹⁵ is C₁-C₆ alkyl, C₆-C₁₀ aryl, 5-10 membered heteroaryl, or a 4-10 membered heterocycle containing up to 5 ring heteroatoms, wherein the heteroatom is selected from the group consisting of O, N, S, and oxidized forms of N and S, wherein the alkyl, aryl, heteroaryl or heterocycle is optionally substituted.

5. The compound of claim 1, wherein the compound is selected from the group consisting of 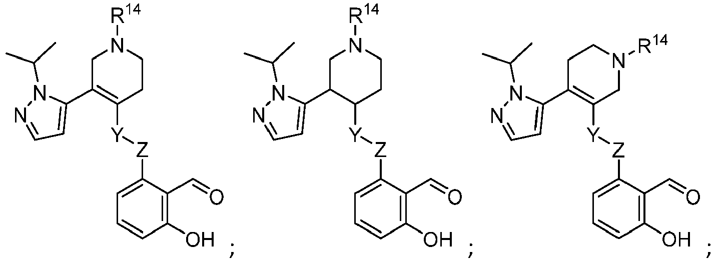 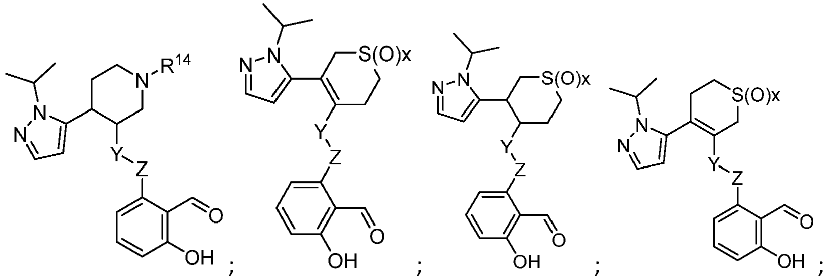 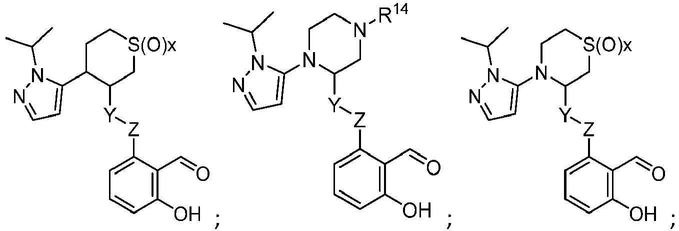 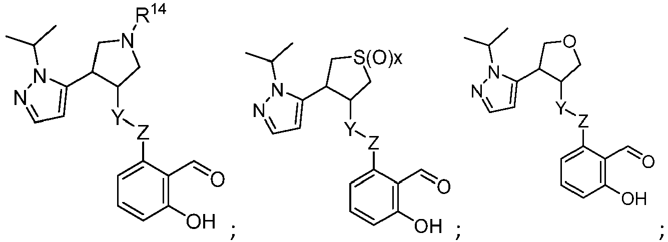 and 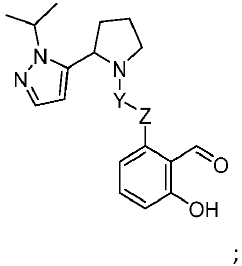 or an N oxide thereof, wherein
x is 0, 1, or 2;
R¹⁴ is C₁-C₆ alkyl, COR¹⁵, or COOR¹⁵;
and R¹⁵ is optionally substituted C₁-C₆ alkyl, optionally substituted C₆-C₁₀ aryl, optionally substituted 5-10 membered heteroaryl or optionally substituted 4-10 membered heterocycle containing up to 5 ring heteroatoms, wherein the heteroatom is selected from the group consisting of O, N, S, and oxidized forms of N and S.

6. The compound of claim 1, wherein the compound is selected from the group consisting of 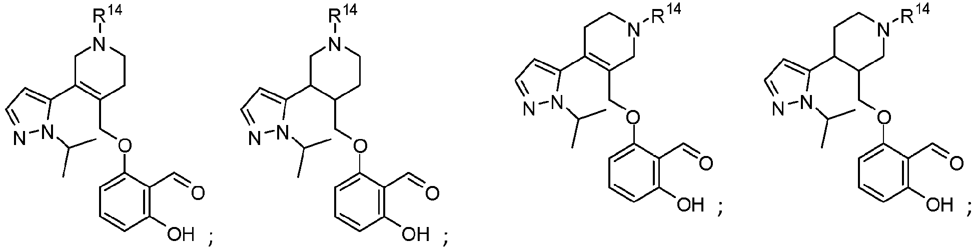 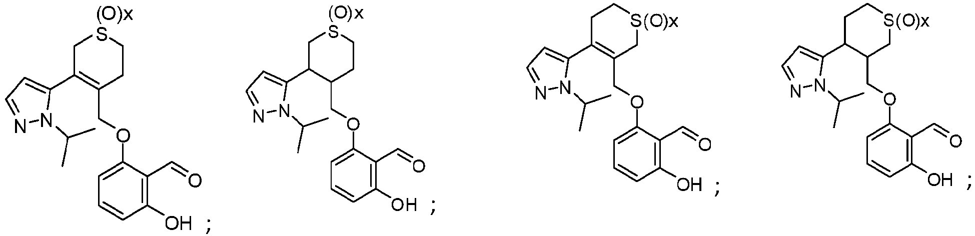 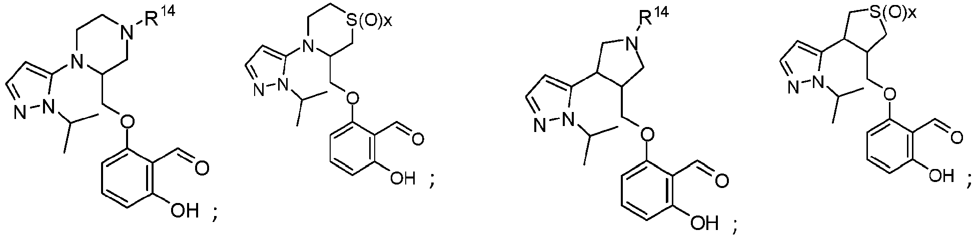 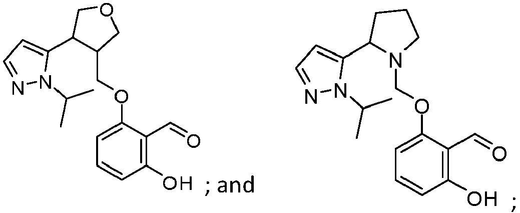 or an N oxide thereof wherein
x is 0, 1, or 2;
R¹⁴ is C₁-C₆ alkyl, COR¹⁵, or COOR¹⁵;
and R¹⁵ is optionally substituted C₁-C₆ alkyl, optionally substituted C₆-C₁₀ aryl, optionally substituted 5-10 membered heteroaryl or optionally substituted 4-10 membered heterocycle containing up to 5 ring heteroatoms, wherein the heteroatom is selected from the group consisting of O, N, S, and oxidized forms of N and S.

7. A compound selected from the group consisting of: 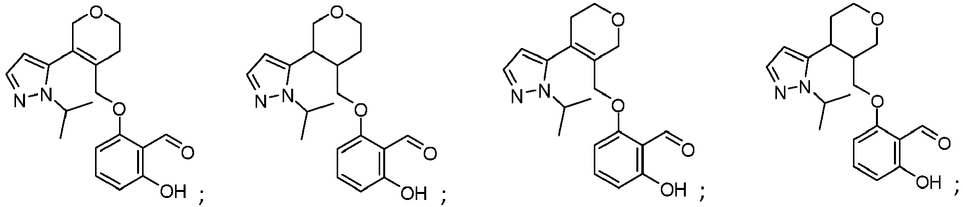 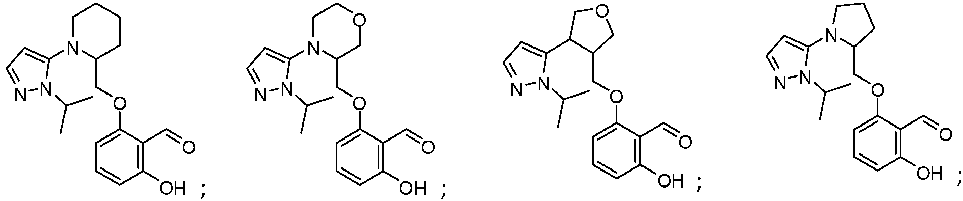 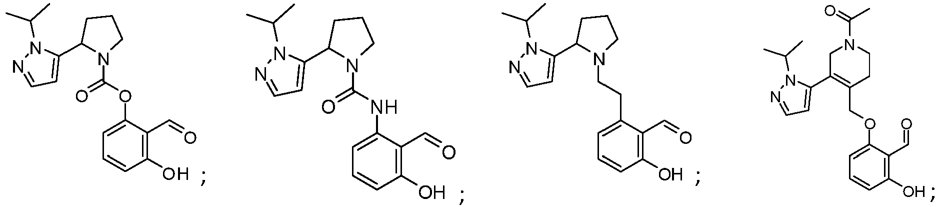 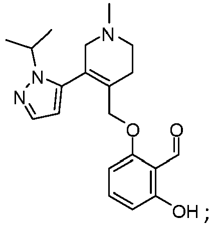 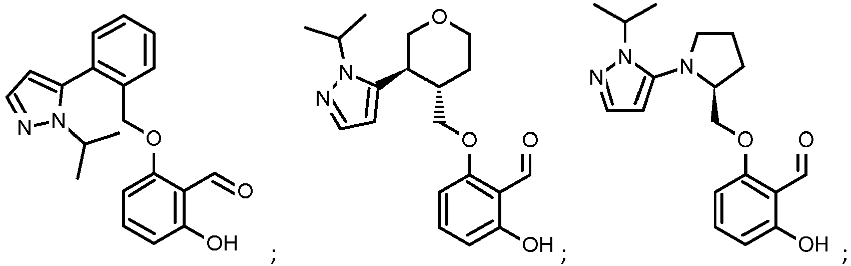 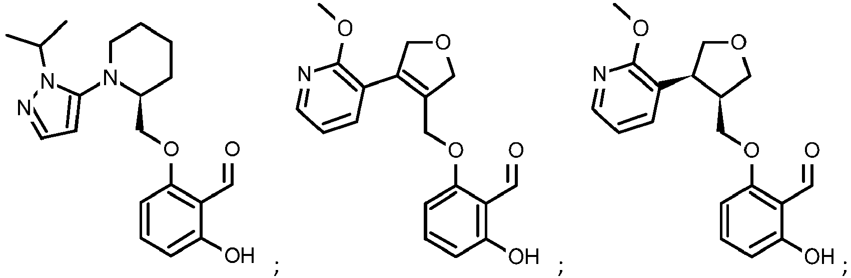 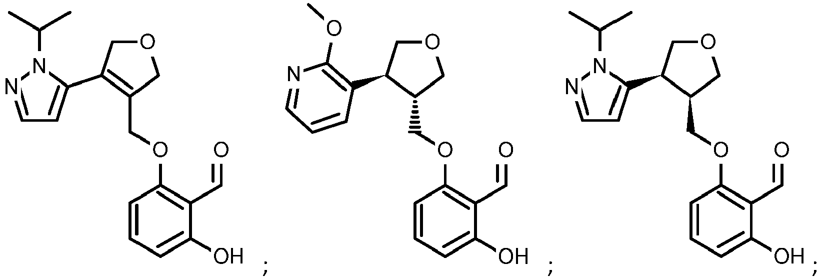 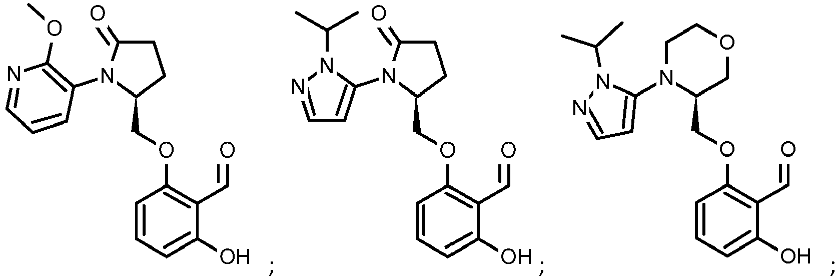 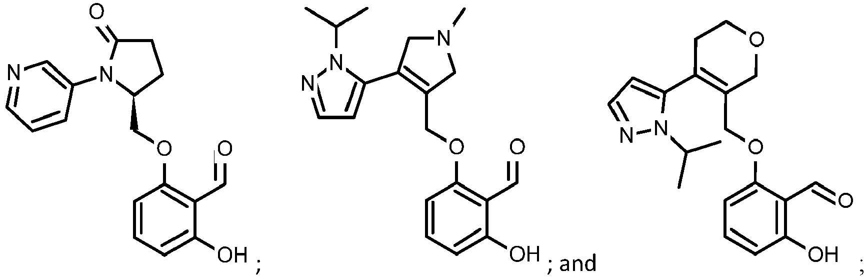 or an N oxide thereof, or a pharmaceutically acceptable salt of each thereof.

8. A composition comprising a compound of any preceding claim and at least one pharmaceutically acceptable excipient.

9. A compound according to any one of claims 1 to 7, or a tautomer thereof, or a pharmaceutically acceptable salt thereof, or the composition of claim 8 for use in a method for increasing oxygen affinity of hemoglobin S in a subject, the method comprising administering to a subject in need thereof the compound of any one of claims 1 to 7, or the tautomer thereof, or the pharmaceutically acceptable salt thereof, or the composition of claim 8.

10. A compound according to any one of claims 1 to 7, or a tautomer thereof, or a pharmaceutically acceptable salt thereof, or the composition of claim 8 for use in α method for treating α condition associated with oxygen deficiency, the method comprising administering to α subject in need thereof the compound of any one of claims 1 to 7, or the tautomer thereof, or the pharmaceutically acceptable salt thereof, or the composition of claim 8.

11. The compound, or the tautomer thereof, or the pharmaceutically acceptable salt thereof, or the composition for use according to claim 10, wherein the condition is selected from cancer, α pulmonary disorder, stroke, high altitude sickness, an ulcer, α pressure sore, Alzheimer's disease, acute respiratory disease syndrome, and α wound.

12. The compound, or the tautomer thereof, or the pharmaceutically acceptable salt thereof, or the composition for use according to claim 10, wherein the condition is sickle cell disease.

## Patentansprüche

1. Verbindung der Formel (I'): 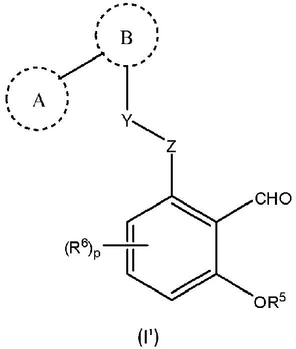 oder ein Tautomer davon, oder ein pharmazeutisch annehmbares Salz von jedem davon,
wobei
Ring A ein gegebenenfalls substituiertes 5-10-gliedriges Heteroaryl ist, das bis zu 3 N-, O- und/oder S-Ringatome und oxidierte Formen von N- und/oder S-Atomen enthält; wobei Ring A in Bezug auf den Y-Substituenten α-substituiert ist;
Ring B ein gegebenenfalls substituiertes C₆-C₁₀-Aryl oder ein 4-10-gliedriger Heterocyclus ist, der bis zu 5 Ringheteroatome enthält, wobei das Heteroatom ausgewählt ist aus der Gruppe bestehend aus O, N, S und oxidierten Formen von N und S;
Y-Z -CH₂O- ist, wobei die rechte Seite des Substituenten mit dem substituierten Phenylring verbunden ist;
R⁵ Wasserstoff oder C₁-C₆-Alkyl ist, wobei das C₁-C₆-Alkyl gegebenenfalls durch 1-5 Halogenatome substituiert ist; R⁶ Halogen, C₁-C₆-Alkyl, C₁-C₆-Alkoxy, C₁-C₆-Alkylthio, C₁-C₆-Alkyl-S(O)- oder C₁-C₆-Alkyl-S(O)₂- ist, wobei das C₁-C₆-Alkyl gegebenenfalls durch 1-5 Halogenatome substituiert ist; oder
R⁶ 4-10-gliedriges Cycloalkyl oder Heterocyclus ist, der durch eine R'R'N-Einheit substituiert ist, wobei R' jeweils unabhängig C₁-C₆-Alkyl oder Wasserstoff ist;
p 0,1, 2 oder 3 ist; und
sich der Begriff Heterocyclus auf einen nichtaromatischen, mono-, bi- oder tricyclischen Ring bezieht, der 2-12 Ringkohlenstoffatome und 1-8 Ringheteroatome enthält, vorausgesetzt, dass der Ring mindestens 3 Ringatome enthält.

2. Verbindung nach Anspruch 1 oder ein Tautomer davon, oder ein pharmazeutisch annehmbares Salz von jedem davon, wobei die Verbindung die Formel IA, IB oder IC aufweist: 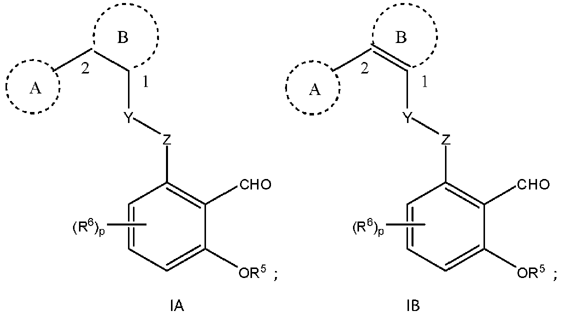 oder 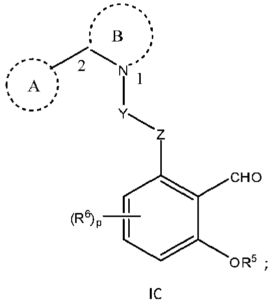 wobei 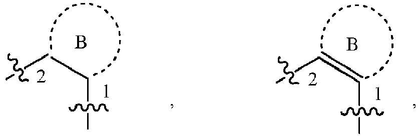 oder 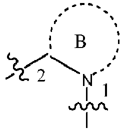 ein gegebenenfalls substituierter 4-10-gliedriger Heterocyclus ist, der bis zu 5 Ringheteroatome enthält, wobei das Heteroatom ausgewählt ist aus der Gruppe bestehend aus O, N, S und oxidierten Formen von N und S.

3. Verbindung nach Anspruch 1 oder ein Tautomer davon, oder ein pharmazeutisch annehmbares Salz von jedem davon, wobei Ring A durch 1-3 Substituenten substituiert ist, die unabhängig ausgewählt sind aus: Halogen, C₁-C₆-Alkyl, und C₁-C₆-Alkoxy, wobei das C₁-C₆-Alkyl gegebenenfalls durch 1-5 Halogenatome substituiert ist.

4. Verbindung nach Anspruch 1 oder ein Tautomer davon, oder ein pharmazeutisch annehmbares Salz von jedem davon, wobei Ring B durch 1-3 Substituenten substituiert ist, die unabhängig ausgewählt sind aus: Halogen, C₁-C₆-Alkyl, COR¹⁵ und COOR¹⁵; und
R¹⁵ C₁-C₆-Alkyl, C₆-C₁₀-Aryl, 5-10-gliedriges Heteroaryl oder ein 4-10-gliedriger Heterocyclus ist, der bis zu 5 Ringheteroatome enthält, wobei das Heteroatom ausgewählt ist aus der Gruppe bestehend aus O, N, S und oxidierten Formen von N und S, wobei das Alkyl, das Aryl, das Heteroaryl oder der Heterocyclus gegebenenfalls substituiert ist.

5. Verbindung nach Anspruch 1, wobei die Verbindung ausgewählt ist aus der Gruppe bestehend aus 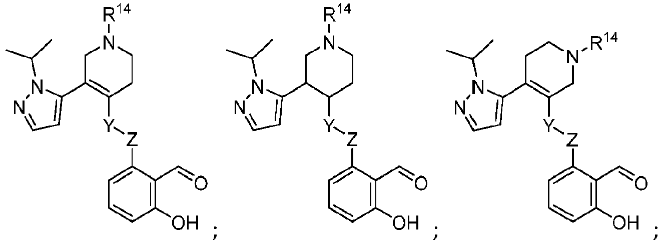 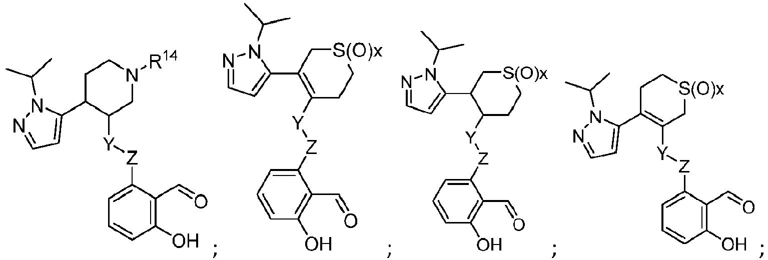 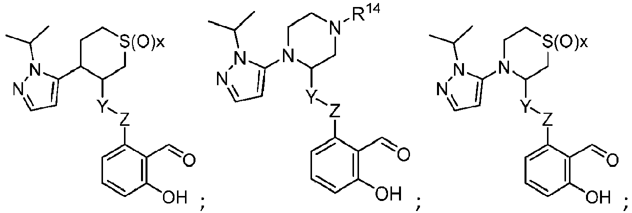 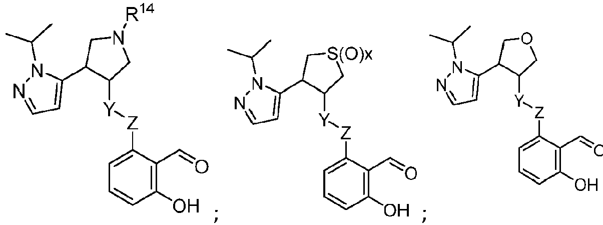 und 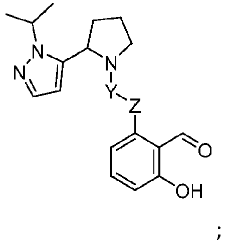 oder einem N-Oxid davon, wobei
x 0,1 oder 2 ist;
R¹⁴ C₁-C₆-Alkyl, COR¹⁵ oder COOR¹⁵ ist;
und R¹⁵ gegebenenfalls substituiertes C₁-C₆-Alkyl, gegebenenfalls substituiertes C₆-C₁₀-Aryl, gegebenenfalls substituiertes 5-10-gliedriges Heteroaryl oder gegebenenfalls substituierter 4-10-gliedriger Heterocyclus ist, der bis zu 5 Ringheteroatome enthält, wobei das Heteroatom ausgewählt ist aus der Gruppe bestehend aus O, N, S und oxidierten Formen von N und S.

6. Verbindung nach Anspruch 1, wobei die Verbindung ausgewählt ist aus der Gruppe bestehend aus 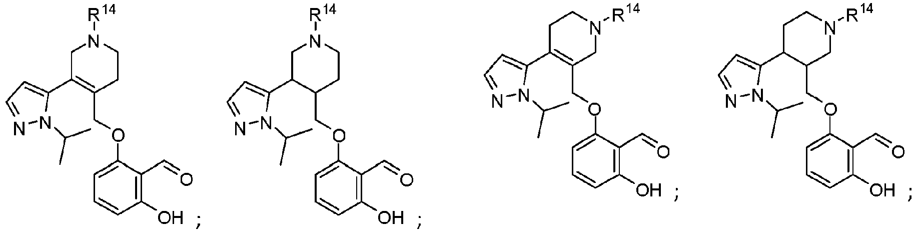 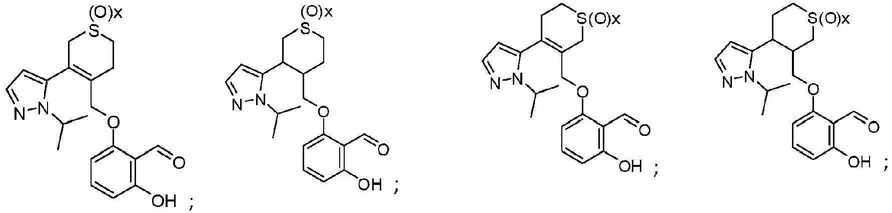 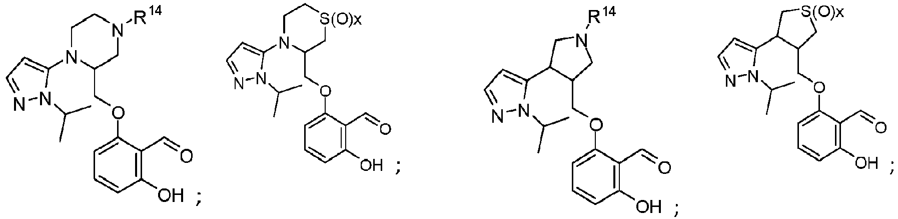 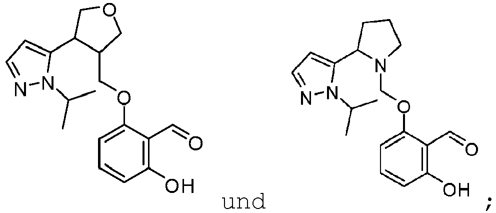 oder einem N-Oxid davon, wobei
x 0, 1 oder 2 ist;
R¹⁴ C₁-C₆-Alkyl, COR¹⁵ oder COOR¹⁵ ist;
und R¹⁵ gegebenenfalls substituiertes C₁-C₆-Alkyl, gegebenenfalls substituiertes C₆-C₁₀-Aryl, gegebenenfalls substituiertes 5-10-gliedriges Heteroaryl oder gegebenenfalls substituierter 4-10-gliedriger Heterocyclus ist, der bis zu 5 Ringheteroatome enthält, wobei das Heteroatom ausgewählt ist aus der Gruppe bestehend aus O, N, S und oxidierten Formen von N und S.

7. Verbindung, die ausgewählt ist aus der Gruppe bestehend aus : 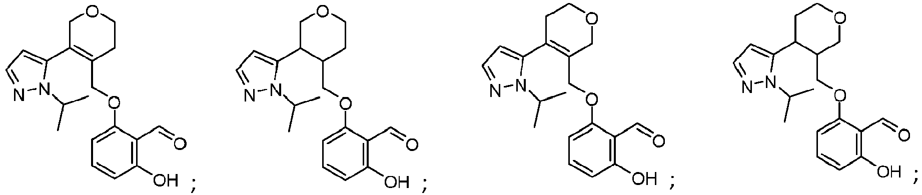 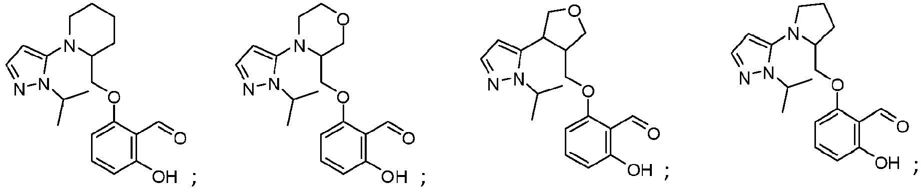 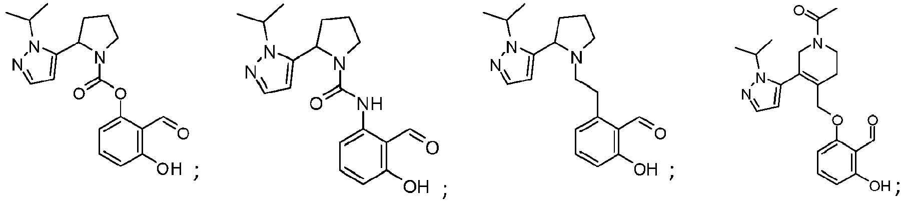 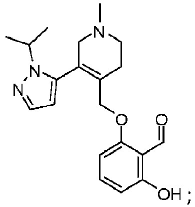 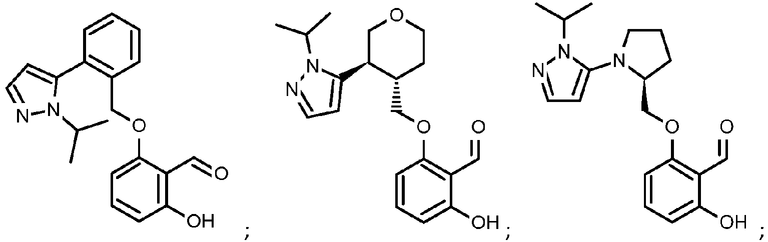 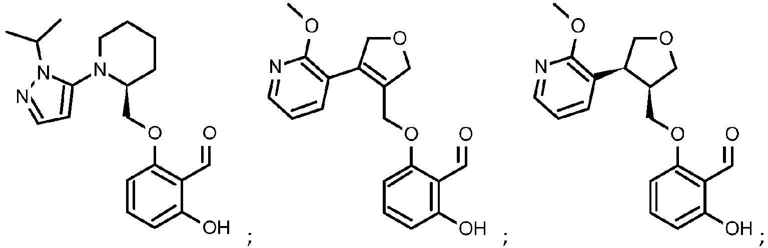 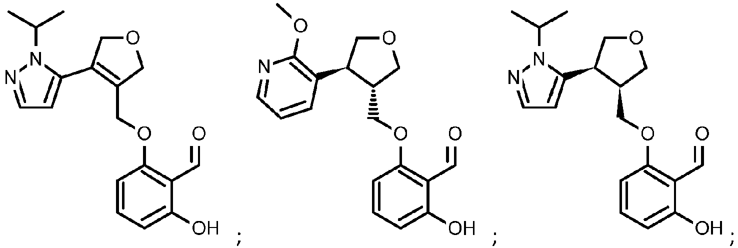 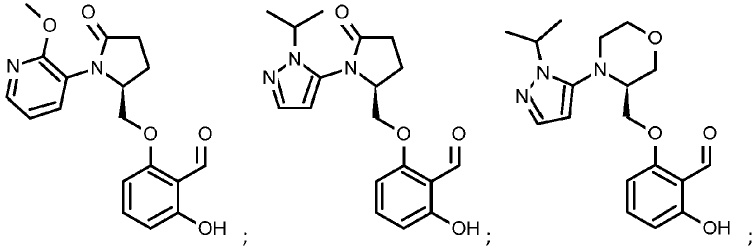 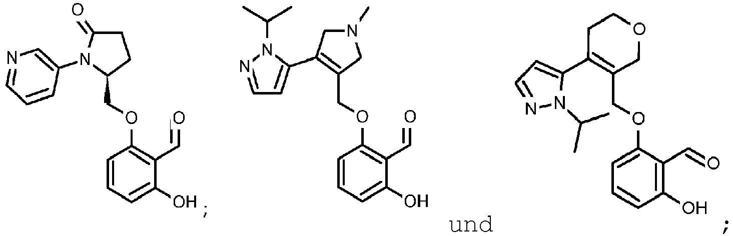 oder einem N-Oxid davon, oder ein pharmazeutisch annehmbares Salz von jedem davon.

8. Zusammensetzung, umfassend eine Verbindung nach einem der vorstehenden Ansprüche und mindestens einen pharmazeutisch annehmbaren Hilfsstoff.

9. Verbindung nach einem der Ansprüche 1 bis 7 oder ein Tautomer davon, oder ein pharmazeutisch annehmbares Salz davon, oder Zusammensetzung nach Anspruch 8 zur Verwendung in einem Verfahren zur Steigerung der Sauerstoffaffinität von Hämoglobin S bei einem Individuum, wobei das Verfahren das Verabreichen der Verbindung nach einem der Ansprüche 1 bis 7 oder des Tautomers davon, oder des pharmazeutisch annehmbaren Salzes davon, oder der Zusammensetzung nach Anspruch 8 an ein Individuum, das dessen bedarf, umfasst.

10. Verbindung nach einem der Ansprüche 1 bis 7 oder ein Tautomer davon, oder ein pharmazeutisch annehmbares Salz davon, oder Zusammensetzung nach Anspruch 8 zur Verwendung in einem Verfahren zur Behandlung einer Krankheit, die mit Sauerstoffmangel assoziiert ist, wobei das Verfahren das Verabreichen der Verbindung nach einem der Ansprüche 1 bis 7 oder des Tautomers davon, oder des pharmazeutisch annehmbaren Salzes davon, oder der Zusammensetzung nach Anspruch 8 an ein Individuum, das dessen bedarf, umfasst.

11. Verbindung oder das Tautomer davon, oder das pharmazeutisch annehmbare Salz davon, oder Zusammensetzung zur Verwendung nach Anspruch 10, wobei die Krankheit ausgewählt ist aus Krebs, einer Lungenerkrankung, Schlaganfall, Höhenkrankheit, einem Geschwür, einem Druckgeschwür, Alzheimer-Krankheit, akutem Atemwegsyndrom und einer Wunde.

12. Verbindung oder das Tautomer davon, oder das pharmazeutisch annehmbare Salz davon, oder Zusammensetzung zur Verwendung nach Anspruch 10, wobei die Krankheit Sichelzellenkrankheit ist.

## Revendications

1. Composé de formule (I') : 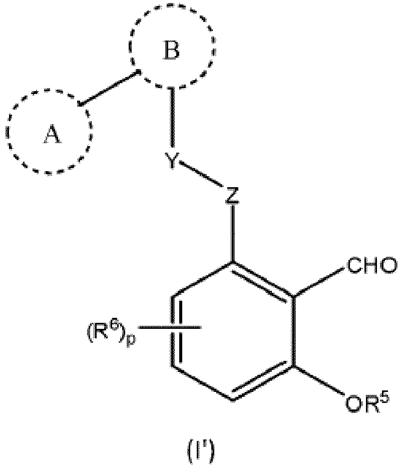 ou forme tautomère correspondante, ou sel pharmaceutiquement acceptable de chacun de ceux-ci,
le cycle A étant un hétéroaryle à 5 à 10 chaînons éventuellement substitué contenant jusqu'à 3 atomes N, O, et/ou S de cycle, et formes oxydées d'atomes de N et/ou de S ;
le cycle A étant substitué en α par rapport au substituant Y ;
le cycle B étant un C₆₋₁₀ aryle ou hétérocycle à 4 à 10 chaînons contenant jusqu'à 5 hétéroatomes de cycle, éventuellement substitué, l'hétéroatome étant choisi dans le groupe constitué par O, N, S, et des formes oxydées de N et S ;
Y-Z étant -CH₂O-, le côté droit du substituant étant joint au cycle phényle substitué ;
R⁵ étant hydrogène ou C₁₋₆ alkyle, le C₁₋₆ alkyle étant éventuellement substitué par 1 à 5 halogéno ;
R⁶ étant halogéno, C₁₋₆ alkyle, C₁₋₆ alcoxy, C₁₋₆ alkylthio, C₁₋₆ alkyl-S(O)-, ou C₁₋₆ alkyl-S(O)₂-, le C₁₋₆ alkyle étant éventuellement substitué par 1 à 5 halogéno ; ou
R⁶ étant cycloalkyle ou hétérocycle à 4 à 10 chaînons substitué par un fragment R'R'N, chaque R' étant indépendamment C₁₋₆ alkyle ou hydrogène ;
p étant 0, 1, 2, ou 3 ; et
le terme hétérocycle faisant référence à un cycle non aromatique, monocyclique, bicyclique ou tricyclique contenant 2 à 12 atomes de carbone de cycle et 1 à 8 hétéroatomes de cycle, étant entendu que le cycle contient au moins 3 atomes de cycle.

2. Composé selon la revendication 1, ou forme tautomère correspondante, ou sel pharmaceutiquement acceptable de chacun de ceux-ci, le composé étant de Formule IA, IB ou IC : 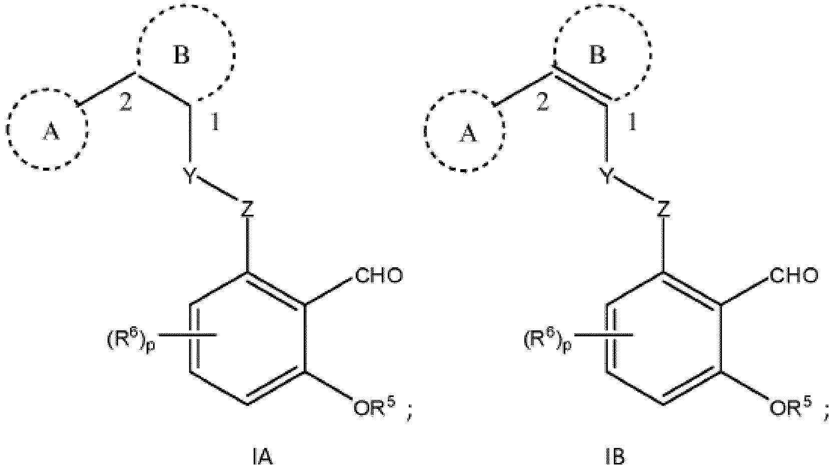 ou 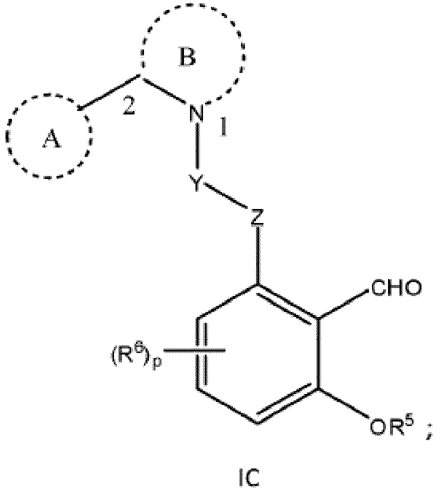 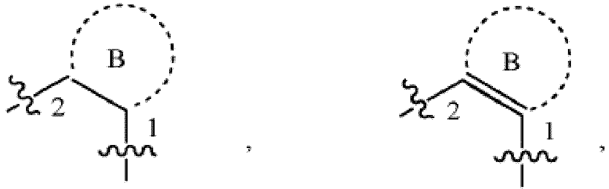 ou 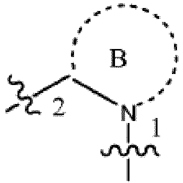 étant un hétérocycle à 4 à 10 chaînons éventuellement substitué contenant jusqu'à 5 hétéroatomes de cycle, l'hétéroatome étant choisi dans le groupe constitué par O, N, S, et des formes oxydées de N et de S.

3. Composé selon la revendication 1, ou forme tautomère correspondante, ou sel pharmaceutiquement acceptable de chacun de ceux-ci, le cycle A étant substitué par 1 à 3 substituants indépendamment choisis parmi : halogéno, C₁₋₆ alkyle, et C₁₋₆ alcoxy, le C₁₋₆ alkyle étant éventuellement substitué par 1 à 5 halogéno.

4. Composé selon la revendication 1, ou forme tautomère correspondante, ou sel pharmaceutiquement acceptable de chacun de ceux-ci, le cycle B étant substitué par 1 à 3 substituants indépendamment choisis parmi : halogéno, C₁₋₆ alkyle, COR¹⁵, et COOR¹⁵ ; et
R¹⁵ étant C₁₋₆ alkyle, C₆₋₁₀ aryle, hétéroaryle à 5 à 10 chaînons, ou un hétérocycle à 4 à 10 chaînons contenant jusqu'à 5 hétéroatomes de cycle, l'hétéroatome étant choisi dans le groupe constitué par O, N, S, et des formes oxydées de N et de S, l'alkyle, l'aryle, l'hétéroaryle ou l'hétérocycle étant éventuellement substitué.

5. Composé selon la revendication 1, le composé étant choisi dans le groupe constitué par 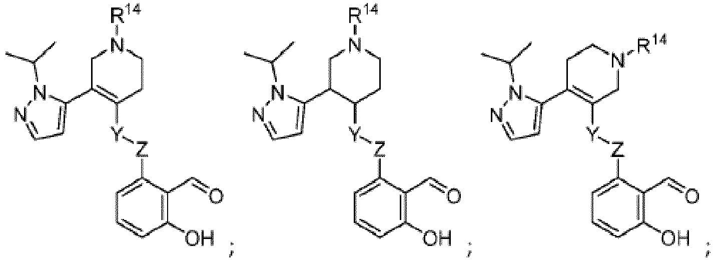 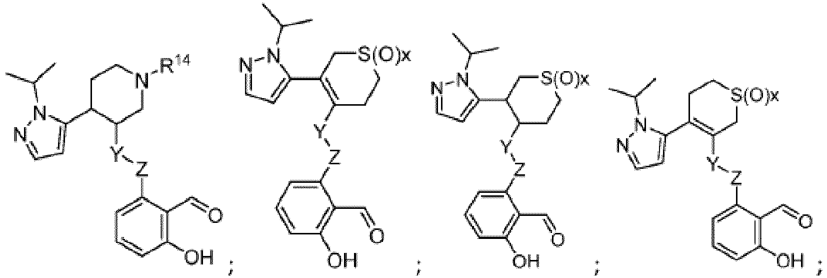 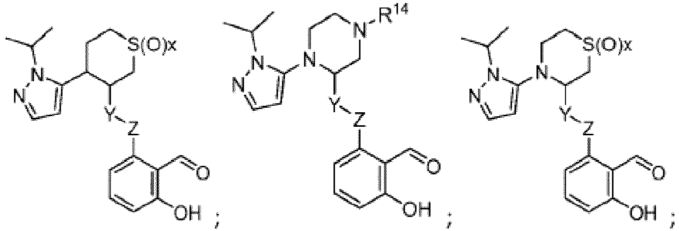 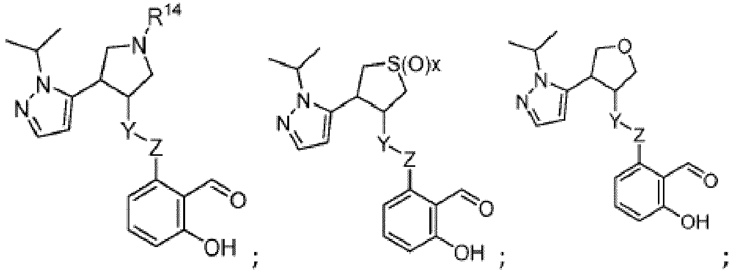 et 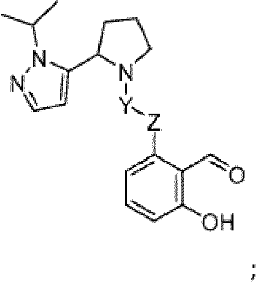 ou N-oxyde correspondant,
x étant 0, 1, ou 2 ;
R¹⁴ étant C₁₋₆ alkyle, COR¹⁵, ou COOR¹⁵ ;
et R¹⁵ étant C₁₋₆ alkyle éventuellement substitué, C₆₋₁₀ aryle éventuellement substitué, hétéroaryle à 5 à 10 chaînons éventuellement substitué, ou hétérocycle à 4 à 10 chaînons éventuellement substitué contenant jusqu'à 5 hétéroatomes de cycle, l'hétéroatome étant choisi dans le groupe constitué par O, N, S et des formes oxydées de N et de S.

6. Composé selon la revendication 1, le composé étant choisi dans le groupe constitué par 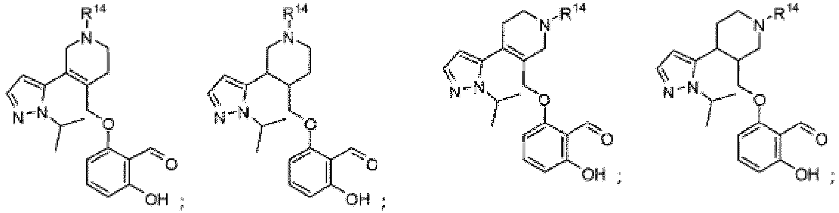 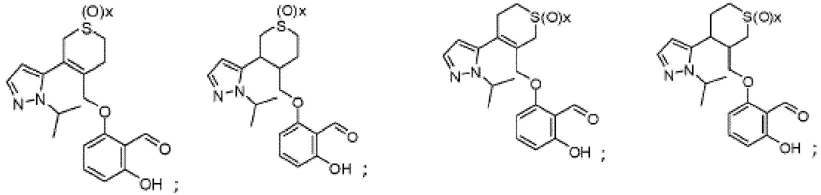 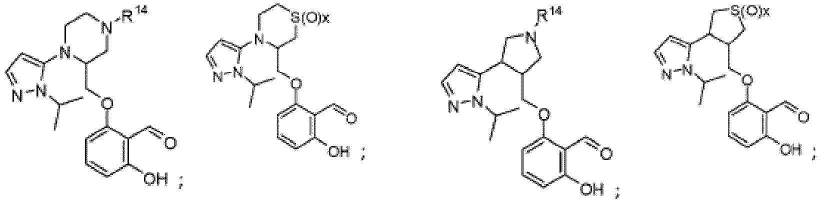 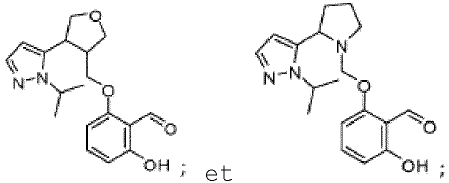 ou N-oxyde correspondant,
x étant 0, 1, ou 2 ;
R¹⁴ étant C₁₋₆ alkyle, COR¹⁵, ou COOR¹⁵ ;
et R¹⁵ étant C₁₋₆ alkyle éventuellement substitué, C₆₋₁₀ aryle éventuellement substitué, hétéroaryle à 5 à 10 chaînons éventuellement substitué, ou hétérocycle à 4 à 10 chaînons éventuellement substitué contenant jusqu'à 5 hétéroatomes de cycle, l'hétéroatome étant choisi dans le groupe constitué par O, N, S et des formes oxydées de N et de S.

7. Composé choisi dans le groupe constitué par : 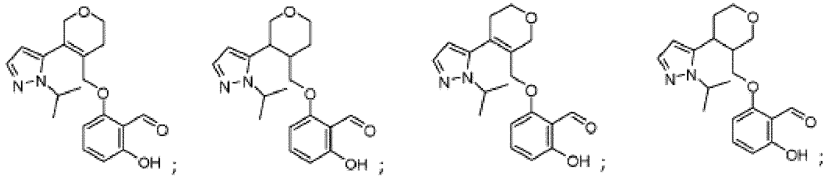 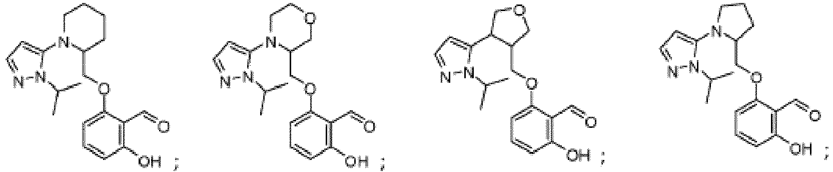 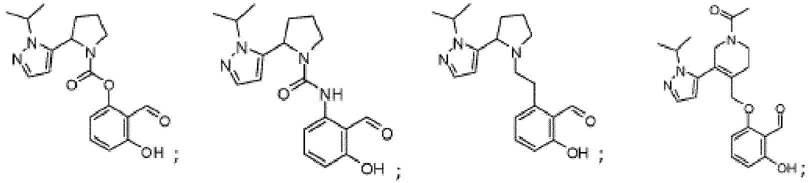 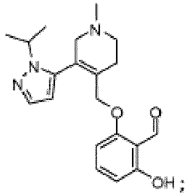 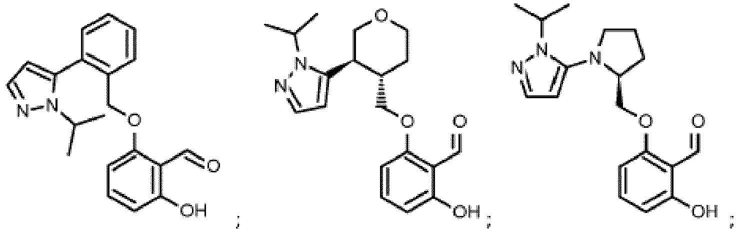 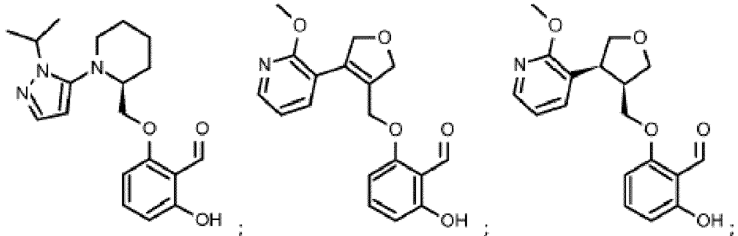 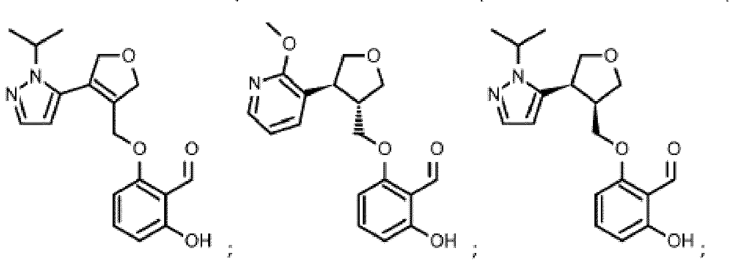 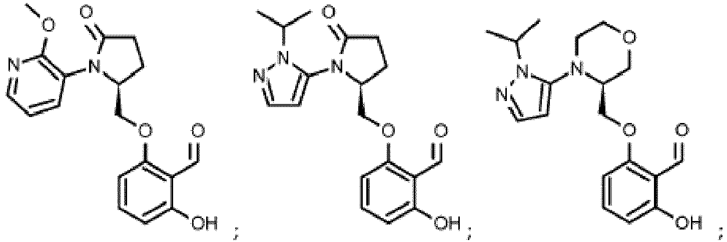 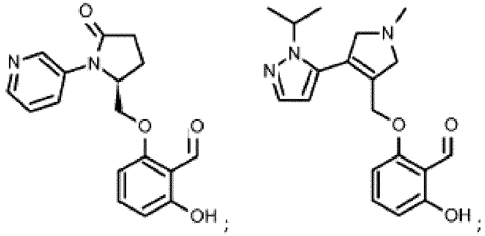 et 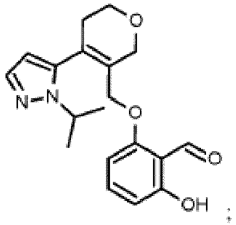 ou N-oxyde correspondant, ou sel pharmaceutiquement acceptable de chacun de ceux-ci.

8. Composition comprenant un composé selon une quelconque revendication précédente et au moins un excipient pharmaceutiquement acceptable.

9. Composé selon l'une quelconque des revendications 1 à 7, ou forme tautomère correspondante, ou sel pharmaceutiquement acceptable correspondant, ou composition selon la revendication 8 pour une utilisation dans un procédé pour l'augmentation de l'affinité pour l'oxygène de l'hémoglobine S chez un sujet, le procédé comprenant l'administration au sujet qui en a besoin du composé selon l'une quelconque des revendications 1 à 7, ou de la forme tautomère correspondante, ou du sel pharmaceutiquement acceptable correspondant, ou de la composition selon la revendication 8.

10. Composé selon l'une quelconque des revendications 1 à 7, ou forme tautomère correspondante, ou sel pharmaceutiquement acceptable correspondant, ou composition selon la revendication 8 pour une utilisation dans un procédé pour le traitement d'une affection associée à une déficience en oxygène, le procédé comprenant l'administration au sujet qui en a besoin du composé selon l'une quelconque des revendications 1 à 7, ou de la forme tautomère correspondante, ou du sel pharmaceutiquement acceptable correspondant, ou de la composition selon la revendication 8.

11. Composé, ou forme tautomère correspondante, ou sel pharmaceutiquement acceptable correspondant, ou composition pour une utilisation selon la revendication 10, l'affection étant choisie parmi un cancer, un trouble pulmonaire, un accident vasculaire cérébral, une maladie de haute altitude, un ulcère, un escarre de décubitus, la maladie d'Alzheimer, un syndrome de détresse respiratoire aiguë, et une plaie.

12. Composé, ou forme tautomère correspondante, ou sel pharmaceutiquement acceptable correspondant, ou composition pour une utilisation selon la revendication 10, l'affection étant une drépanocytose.
